# EUROPÄISCHE PATENTSCHRIFT

(11) **EP 2 430 167 B1**
(45) Veröffentlichungstag und Bekanntmachung des Hinweises auf die Patenterteilung: **07.01.2015**
(21) Anmeldenummer: 10721147.6
(22) Anmeldetag: 17.05.2010
(51) Int. Cl.: C12N 15/867

(54) **ASLV-VEKTORSYSTEM**
ASLV VECTOR SYSTEM
SYSTÈME DE VECTEUR ASLV

(30) Priorität: 15.05.2009 DE 102009021592
(43) Veröffentlichungstag der Anmeldung: 21.03.2012
(73) Patentinhaber: Medizinische Hochschule Hannover, 30625 Hannover (DE)
(72) Erfinder: SCHAMBACH, Axel, 21220 Seevetal (DE); BAUM, Christopher, 22589 Hamburg (DE); SÜRTH, Julia, Debora, 30163 Hannover (DE)
(74) Vertreter: Taruttis, Stefan Georg
(86) Internationale Anmeldenummer: PCT/EP2010/056757
(87) Internationale Veröffentlichungsnummer: WO 2010/130844

(56) Entgegenhaltungen:
- EP-A2- 1 757 703
- HU JINGQIONG ET AL: "Reduced genotoxicity of avian sarcoma leukosis virus vectors in rhesus long-term repopulating cells compared to standard murine retrovirus vectors." MOLECULAR THERAPY : THE JOURNAL OF THE AMERICAN SOCIETY OF GENE THERAPY SEP 2008 LNKD- PUBMED:18578011, Bd. 16, Nr. 9, September 2008 (2008-09), Seiten 1617-1623, XP002595957 ISSN: 1525-0024
- HU JINGQIONG ET AL: "Transduction of rhesus macaque hematopoietic stem and progenitor cells with avian sarcoma and leukosis viral vectors" HUMAN GENE THERAPY, Bd. 18, Nr. 8, August 2007 (2007-08), Seiten 691-700, XP002595958 ISSN: 1043-0342
- SCHAMBACH ET AL: "Equal potency of gammaretroviral and lentiviral SIN vectors for expression of O<6>-methylguanine-DNA methyltransferase in hematopoietic cells" MOLECULAR THERAPY, ACADEMIC PRESS, SAN DIEGO, CA, US LNKD- DOI:10.1016/J.YMTHE.2005.08.012, Bd. 13, Nr. 2, 1. Februar 2006 (2006-02-01), Seiten 391-400, XP005252899 ISSN: 1525-0016 & SCHAMBACH ET AL: "829. Design of a Safe and Efficient Vector for Bone Marrow Chemoprotection by O<6>-Methyl-Guanine-DNA-MethylTransferase" MOLECULAR THERAPY, ACADEMIC PRESS, SAN DIEGO, CA, US, Bd. 11, 15. August 2005 (2005-08-15), Seiten 322-323, XP005016168 ISSN: 1525-0016
- SCHAMBACH ET AL: "Vector design for expression of O<6>-methylguanine-DNA methyltransferase in hematopoietic cells" DNA REPAIR, ELSEVIER, AMSTERDAM, NL LNKD- DOI:10.1016/J.DNAREP.2007.03.017, Bd. 6, Nr. 8, 17. Juli 2007 (2007-07-17), Seiten 1187-1196, XP022155652 ISSN: 1568-7864
- MODLICH U ET AL: "Cell-culture assays reveal the importance of retroviral vector design for insertional genotoxicity" BLOOD, AMERICAN SOCIETY OF HEMATOLOGY, US LNKD- DOI:10.1182/BLOOD-2005-08-024976, Bd. 108, Nr. 8, 6. Juli 2006 (2006-07-06), Seiten 2545-2553, XP002455802 ISSN: 0006-4971
- SCHAMBACH A ET AL: "Overcoming promoter competition in packaging cells improves production of self-inactivating retroviral vectors" GENE THERAPY, MACMILLAN PRESS LTD., BASINGSTOKE, GB LNKD- DOI:10.1038/SJ.GT.3302807, Bd. 13, Nr. 21, 8. Juni 2006 (2006-06-08), Seiten 1524-1533, XP002455803 ISSN: 0969-7128
- BAUM ET AL: "Retrovirus Vectors: Toward the Plentivirus?" MOLECULAR THERAPY, ACADEMIC PRESS, SAN DIEGO, CA, US LNKD- DOI:10.1016/J.YMTHE.2006.03.007, Bd. 13, Nr. 6, 1. Juni 2006 (2006-06-01), Seiten 1050-1063, XP005469379 ISSN: 1525-0016
- BAILEY ET AL: "Novel Gammaretroviral Vectors for Gene Therapy of SCID-X1" MOLECULAR THERAPY, ACADEMIC PRESS, SAN DIEGO, CA, US, Bd. 13, 1. Januar 2006 (2006-01-01), Seiten S256-S257, XP005675772 ISSN: 1525-0016
- GALLA ET AL: "Reverse Transcriptase Deficient Retroviral Vectors for Transient Cell Modification" MOLECULAR THERAPY, ACADEMIC PRESS, SAN DIEGO, CA, US, Bd. 13, 1. Januar 2006 (2006-01-01), Seite S177, XP005675564 ISSN: 1525-0016
- CHEN-WICHMANN ET AL: "SIN gammaretroviral vectors for the gene therapy of x-CGD" BLOOD CELLS, MOLECULES AND DISEASES, LAJOLLA, US LNKD- DOI:10.1016/J.BCMD.2007.10.030, Bd. 40, Nr. 2, 12. Februar 2008 (2008-02-12), Seite 260, XP022477828 ISSN: 1079-9796
- HUGHES STEPHEN H: "The RCAS vector system." FOLIA BIOLOGICA 2004 LNKD- PUBMED:15373344, Bd. 50, Nr. 3-4, 2004, Seiten 107-119, XP008125380 ISSN: 0015-5500
- HILDINGER M ET AL: "Design of 5' untranslated sequences in retroviral vectors developed for medical use", JOURNAL OF VIROLOGY, THE AMERICAN SOCIETY FOR MICROBIOLOGY, US, vol. 73, no. 5, 1 May 1999 (1999-05-01), pages 4083-4089, XP002240461, ISSN: 0022-538X
- "Duden Deutsches Universalwörterburch, 6. überarbeitete und erweiterte Auflage", October 2006 (2006-10), Dudenverlag, Mannheim, Leipzig, Wien, Zürich ISBN: 3411055065 page 1658, * page 1653 *

## Beschreibung

Im folgenden wird ein viraler selbst inaktivierender (SIN-) Vektor auf Basis des Avian Sarkoma Leukosis Virus (ASLV), virale Partikel, die diesen Vektor enthalten, ein Split-Packaging-System, umfassend oder bestehend aus dem SIN-Vektor, einem ersten Helferplasmid, das zur Expression des viralen Fusionsproteins gag-pol dient, und einem zweiten Helferplasmid, das zur Expression des retroviralen Hüllproteins (env) dient beschrieben, sowie ein Verfahren zur Herstellung des viralen Partikels mittels des Split-Packaging-Systems, insbesondere durch Expression in kultivierten humanen Zellen. Weiterhin finden die viralen Partikel Verwendung als Medikament, insbesondere zum Transfer eines Transgens in Zellen, sowie zur die Herstellung des Medikaments. Das erste und zweite Helferplasmid, beispielsweise in einer Verpackungs-Zelllinie enthalten oder transient transfiziert, dienen der Erzeugung von nicht replizierenden (RCR - inkompetenten) viralen Partikeln, die RNA mit einem erfindungsgemäßen SIN-LTR am 3'-Ende enthalten, wobei die RNA einen therapeutisch wirksamen Abschnitt aufweisen kann, der z.B. als Transgen bezeichnet wird. Dieser 3' SIN LTR beinhaltet eine umfangreiche Deletion der U3 Region, welche im Zuge der reversen Transkription in den 5' LTR kopiert wird. Für die Expression eines Transgens, das z.B. zur Verwendung für die Gentherapie ein humanes oder heterologes Gen sein kann, enthält der virale SIN-Vektor vorzugsweise eine Expressionskassette, die in 5' zum 3' SIN LTR angeordnet ist.

Der Vektor erlaubt auch in humanen Verpackungszellen die Erzeugung viraler Partikel mit hohem Titer und zeichnet sich dadurch aus, dass bei der Transduktion von Zielzellen, insbesondere humaner Zellen, durch Kontaktieren der Zellen extrakorporal, d.h. in vitro, oder mittels Verabreichung eines dazu hergerichteten Partikels an einen Patienten in vivo transduzierte Zellen erzeugt werden, die das Transgen über einen langen Zeitraum stabil bzw. mit geringer Abschwächung exprimieren, wobei die Häufigkeit der Transformation von Zielzellen durch Onkogenaktivierung gering ist. Die Expressionskassette weist in 5' vor der Nukleinsäuresequenz, die das Transgen kodiert, einen Promotor auf, und vorzugsweise in 3' zu der das Transgen kodierenden Sequenz ein posttranskriptionell regulatorisches Element (PRE), insbesondere ein WPRE ( PRE des Waldmurmeltier Hepatitis Virus (WHV)).

### Stand der Technik

Hughes (Folia Biologika 50,107-119 (2004)) beschreibt eine Serie von viralen Vektoren auf Basis des Avian Leukosis Virus (ALV), die zur besseren Produktion replikationskompetenter Retroviren (RCR) einen funktionierenden LTR aufweisen. (RCAS: Replication-Competent ASLV LTR with a Splice Acceptor). Bei diesem System liegen alle Komponenten zur Virusproduktion auf einem Plasmid (wie im Volllängenvirus) und ein mögliches Transgen wird in die 3' untranslatierte Region (UTR) eingefügt.

Hu et al. (Molecular Therapy, 1617-1623 (2008)) zeigen, dass ASLV nicht dazu neigt, sich vorzugsweise innerhalb oder in der Nähe von Proto-Onkogenen zu inserieren, und dass die LTRs von ASLV nicht dazu neigen, benachbarte Gene zu aktivieren und kommt daher zu dem Schluß, dass ASLV eine geeignete Basis für virale Vektoren bilden sollte. Es wird angedeutet, dass eine Deletion der Verstärkerregion (enhancer) im LTR oder das Verhindern des Hindurch-Transkribierens über ein Terminationssignal, durch eine verbesserte Polyadenylierung erreicht werden könnte.

Die EP 1 757 703 A2 beschreibt selbstinaktivierende retrovirale Vektoren, insbesondere lentivirale und gammaretrovirale Vektoren, die sich dadurch auszeichnen, dass ein starker Promotor das U3 Element im 5'-LTR ersetzt, um die Transkription der Volllängen-mRNA zur Verpackung zu kontrollieren.

Schambach et al. in Molecular Therapy 391-400 (2006) beschreiben gammaretrovirale und lentivirale selbstinaktivierende Vektoren, die für die Gentherapie eine Expressionskassette von O⁶-Methylguanin-DNA-Methyltransferase enthalten.

Hildinger et al. in J. Virol. 4083-4089 (1999) beschreibt, dass das Vorliegen eines Spleißdonors in Kombination mit dem Verpackungssignal ψ eine wesentliche Voraussetzung für die Erzeugung hoher Titer viraler Partikel in Verpackungszellen ist.

Hu et al. (Human Gene Therapy 691-700 (2007)) und Mitchell et al. (Plos Biology, e234, 2004) zeigen, dass ASLV nicht dazu neigt, sich vorzugsweise innerhalb oder in der Nähe von Proto-Onkogenen zu inserieren, insbesondere ohne deutliche Präferenz für promoternahe Bereiche. Demzufolge ist die Gefahr zur Insertionsmutagenese im Vergleich zu den klinisch verwendeten Vektorsystemen vom murinen Leukämievirus (MLV) und humanem Immundefizienzvirus (HIV) als geringer einzuschätzen. Es wird angedeutet, dass eine Deletion der Verstärkerregion (Enhancer) im LTR oder das Verhindern des Hindurch-Transkribierens über ein Terminationssignal wünschenswert wäre. Aufgrund der Vektorarchitektur (1 Plasmid, replizierend) ist dieses System für klinische Anwendungen i.d.R. nicht geeignet.

Hu et al. zeigen, dass von ASLV abgeleitete virale Vektoren mit intakten LTRs in Säugerzellen nicht replizieren. Die genaue Ursache hierfür ist unklar.

Aufgrund der retroviralen Architektur von 2 LTRs haben Retroviren intrinsisch schwache polyA-Signale. Für lentivirale Vektoren ist bekannt, dass das Hindurch-Transkribieren vom 3' Ende des inserierten viralen Vektors zur unerwünschten Genaktivierung führen kann, so zum Beispiel Zaiss et al. (Journal of Virology 7209-7219 (2002)) und Schambach, Baum et al. (Molecular Therapy 2007, 15(6):1167-73). Dies ist auch deshalb bedeutsam, weil die U3 Region neben Promotor-/Enhancer-Elementen auch Polyadenylierungsverstärker enthält und so etwaige Deletionen für jede Virusfamilie neu in puncto residuale Enhanceraktivität und Effizienz der Polyadenylierung zu validieren sind.

Für gammaretrovirale Vektoren schlagen Kraunus et al. (Gene Therapy 568-1578 (2004)) vor, den vollständigen Bereich des Enhancerelements und Promoters der 3' U3-Region zu deletieren und nur die ersten 22 bp stromaufwärts des Enhancers und die letzten 14 bp stromabwärts zu belassen. Bei ASLV bzw. Rous Sarkoma Virus (RSV) wird die LTR-Region durch eine sehr kurze R-Region verkompliziert, so dass das polyA Signal (AATAAA) in der U3-Region zu liegen kommt. Dies ist für Retroviren sehr ungewöhnlich (sonst in der R-Region lokalisiert) und erschwert die Konstruktion eines SIN Vektors.

Aubert et al. (The J. of Cell Biology 113, 497-506 (1991) und Flamant et al. (Journal of General Virology 74, 39-46 (1993)) beschreiben Deletionen der U3-Region von ALV von -149 bis -15, relativ zur R-Region bzw. von -149 bis -15, und -98 bis -59, ebenfalls relativ zur R-Region. In allen diesen U3-Regionen sind noch restliche Enhancer-Elemente enthalten (Cullen et al., MCB 1985, 438-47), was problematisch für mögliche klinische Anwendungen ist. Denn diese Enhancerelemente können per Insertionsmutagenese möglicherweise benachbarte Gene fehlregulieren (z.B. Onkogene).

### Aufgabe der Erfindung

Der Erfindung stellt sich daher die Aufgabe, ein klinisch einsetzbares Split-packaging-System für die Produktion in Säugetierzellen (z.B. humane Zellen, beispielsweise HEK293T) mit einem viralen Vektor und Helferplasmiden bereitzustellen, mit dem der virale Vektor als viraler Partikel verpackt hergestellt werden kann, bei dem das Risiko der Rekombination zu einem replikationskompetenten Retrovirus (RCR) minimiert ist, und auch das Risiko minimiert ist, dass die Insertion des viralen Vektors zur Aktivierung benachbarter Gene der Zielzelle (Insertionsmutagenese) führt. Vorzugsweise soll der virale Vektor, bzw. das System mit Helferplasmiden überdies die Erzeugung viraler Partikel mit hohem Titer erlauben, wobei der virale Vektor eine Expressionskassette enthalten können soll, von welcher ein Transgen und genregulatorische Sequenzen (z.B. shRNA, miRNA etc.) effizient transkribiert und/oder translatiert werden. Vorzugsweise werden diese Vektoren mit klinisch einsetzbaren Hüllproteinen (z.B. RD114/TR, MLV amphotropes env, Glykoprotein des vesikulären Stomatitisvirus (VSVg)) pseudotypisiert. All diese Kriterien sind in Kombination angestrebt, da sie für mögliche klinische Anwendungen essentiell sind.

### Allgemeine Beschreibung der Erfindung

Die Erfindung löst diese Aufgabe mit den Merkmalen der Ansprüche und insbesondere mit einem Vektor, der die angestrebten Eigenschaften aufweist. Von einem Plasmid kann eine virale RNA gemäß den Ansprüchen transkribiert werden, die einen viralen Vektor auf Basis von ASLV bereitstellt, deren 3'-U3-Region eine selbst inaktivierende 3'-LTR (SIN-LTR) erzeugt bzw. ist, sowie den viralen Partikel, der eine den viralen Vektor kodierende Nukleinsäuresequenz enthält, insbesondere RNA. Weiterhin stellt die Erfindung ein System mit einem ersten Helferplasmid, das in einer Expressionskassette die kodierende Sequenz für das gag-pro-pol-Fusionsprotein von ASLV kodiert, und mit einem zweiten Helferplasmid bereit, das in einer Expressionskassette ein Hüllprotein (env) kodiert, z.B. env von ASLV, RD114/TR, MLV-Amphotrop, VSVg oder MLV-Ecotrop.

Bei Anwesenheit von erstem und zweitem Helferplasmid in einer eukaryotischen Zelle, nachfolgend auch als Verpackungszelle bezeichnet, werden virale Partikel mit hohem Titer erzeugt, deren virale RNA wegen der Deletionen in der während der reversen Transkription zur viralen DNA aus der 3'-U3-Region entstehenden 5'-U3-Region in einer Zielzelle selbst inaktivierend (SIN) sind, jedoch eine hohe Transkriptionsrate und effiziente Translation des Transgens erlauben, das in einer Expressionskassette in 5' vor dem 3'-SIN-LTR angeordnet ist. In dieser Konfiguration ist die Aktivierung benachbarter genomischer Sequenzen durch die Insertion des viralen Vektors signifikant reduziert. Die Verminderung der Aktivierung benachbarter Sequenzen ist für γ-retrovirale Vektoren von Zychlinski et al. (Mol. Ther. 16: 718-725 (2008)) gezeigt worden.

Mutationen, die für virale SIN-Vektoren auf Basis anderer Retroviren, beispielsweise Lentiviren, bekannt sind, welche die Eigenschaften des hohen Titers in Verpackungszellen einerseits, keine Promotoraktivität im LTR, sowie eine effiziente Transkription eines Transgens aus einer Vektor-internen Expressionskassette ohne Hindurch-Transkription in genomische Sequenzen am Insertionsort kombinieren, lassen sich nicht auf die Konstruktion von SIN-LTR-Vektoren auf Basis von ASLV übertragen. Denn die Anordnung funktionaler Elemente in den einzelnen Viren und deren Zusammenwirken mit zellulären Wirtsfaktoren sind nicht miteinander identisch, und die Auswirkungen der Veränderung von Sequenzen auf den Titer, die Verhinderung der Aktivierung benachbarter genomischer Sequenzen am Insertionsort und auf eine Transkription des Transgens aus einer internen Expressionskassette lassen sich nicht vorhersagen.

Der virale Vektor enthält anstelle des Wildtyp-LTRs einen 3'-LTR, dessen U3- Region über einen großen Abschnitt deletiert ist, sodass die transkriptionelle Aktivität der Promotor- und Enhancer-Regionen der U3-Region eliminiert ist. Die Eliminierung der Promotor- und Enhancer-Regionen der U3-Region ist z.B. durch Deletion gemäß Anspruch 1 bestimmt. Insbesondere weist erfindungsgemäß die U3-Region von den 229 Nukleotiden der Wildtyp-U3 Region (Seq. ID Nr. 1) von ASLV nur die Nukleotide 1-27 und die Nukleotide 187-229 auf, vorzugsweise nur die Nukleotide 1-27 und 207-229. Diese veränderte 3'- U3-Region des viralen Vektors, bei der von der Wildtyp-U3 Region die Nukleotide 28-186 deletiert sind, und vorzugsweise zusätzlich die Nukleotide 187-202 deletiert sind, weist nach reverser Transkription und Integration in das Genom einer Zielzelle im Wesentlichen keine Promotor- und/oder Enhanceraktivität auf. Die verpackbare virale RNA wird durch einen starken, in 5' angeordneten Promotor getrieben, welcher im Zuge der reversen Transkription verloren geht, bzw. nicht in der durch Transkription erzeugten viralen RNA enthalten ist. Dies erlaubt die Erzeugung eines Transkripts, das in Verbindung mit einem ersten und zweiten Helferplasmid zur Erzeugung viraler Partikel mit hohem Titer geeignet ist, die die virale RNA enthalten.

Die Deletion von Promotor- und Enhancer-Regionen kann optional durch eine Insertion mit einer Nukleotidsequenz ersetzt sein, die keine Promotor- oder Enhanceraktivität aufweist.

Der 3'-SIN LTR des Vektors kann aus den vorgenannten Abschnitten der deletierten Wildtyp-U3-Region, einer Wildtyp-R-Region und einer Wildtyp-U5-Region bestehen. Die erfindungsgemäß U3-Region mit Deletionen kann optional zwischen den verbleibenden Abschnitten der Wildtyp-U3-Region zusätzliche Nukleotidsequenzen ohne Promotoraktivität enthalten, die beispielsweise aus der Gruppe ausgewählt sind, die miRNA, shRNA, Polyadenylierungsverstärker (USE), Rekombinaseerkennungssequenzen (z.B. LoxP, Rox, FRT) und Isolatorelemente (z.B. cHS4) umfasst.

Zur Insertion von Nukleotidsequenzen ohne Promotoraktivität in die erfindungsgemäße U3-Region weist diese vorzugsweise eine einmalige Schnittstelle für eine Restriktase auf, insbesondere bevorzugt dadurch, dass die Nukleotide 187-190 in der Nummerierung der Wildtyp-U3 Region durch Mutation die Basenfolge CGTA ergeben, wodurch eine sekundäre Schnittstelle für SnaBI mit der Erkennungssequenz TACGTA erzeugt wird.

Als Alternative zur Deletion der Nukleotide 187-202 können auch die Nukleotide 200-206 (TATTTAA) mutiert werden, beispielsweise zur Sequenz TGTCTAA. Es wird vermutet, dass die Nukleotide 200-206 die TATA-Box der Wildtyp-U3 Region bilden, sodass eine Mutation, die die diese TATA-Box verändert, diesen Teil der Promotoraktivität der U3-Region reduziert bzw. abschafft.

Die erfindungsgemäße deletierte U3-Region weist noch eine Integrase-Angriffsstelle (integrase attachment) auf, insbesondere in Nukleotiden 1-25, sowie ein PolyA-Signal, z.B. an Nukleotiden 223-228, jeweils in der Nummerierung der Nukleotide der Wildtyp-U3-Region. Es wird vermutet, dass die Beibehaltung der Integrase-Angriffsstelle und des polyA-Signals wichtig für die virale Infektion sind. Überraschenderweise erlaubt der Vektor einerseits eine Produktion viraler Partikel mit hohem Titer in einer Verpackungszelle bzw. Verpackungzelllinie, andererseits nach reverser Transkription und Integration in das Genom in einer Zielzelle eine effiziente Transkription eines Transgens aus einer Expressionskassette von einem internen starken Promotor, ohne dass eine Hindurch-Transkription über die 3'-SIN-LTR in angrenzende Regionen der Integrationsstelle oder die Aktivierung angrenzender genomischer Sequenzen erfolgt.

In Verpackungszellen erzeugte virale Partikel, die virale RNA mit einer erfindungsgemäßen 3'-SIN LTR, bzw. einer erfindungsgemäßen 3'-U3-Region enthielten, wiesen ohne Aufkonzentration Titer von ca. 1x10⁶ infektiöse Partikel /mL auf. Dabei wurde der Titer von dem das gag-pro-pol-Fusionsprotein bzw. das Hüllprotein kodierenden Gen des ersten bzw. zweiten Helferplasmids, bzw. der Verpackungzelllinie beeinflusst, und nicht signifikant von der Art oder dem Ausmaß der Deletion in der U3-Region.

Überraschenderweise war die Expression eines Transgens von einer Expressionskassette bei erfindungsgemäßen viralen Vektoren mit SIN-Deletion etwa um den Faktor 3 höher als in einem Vergleichsvektor, bei dem der 3'- LTR eine U3-Region mit Wildtyp-Sequenz aufwies. Erfindungsgemäß ist weiterhin bevorzugt, dass die kodierenden Sequenzen des ersten und/oder zweiten Helferplasmids kodonoptimiert sind, sodass die viralen Kodons gegen Kodons der Zielzelle ersetzt sind, insbesondere gegen humane Kodons ausgetauscht sind, z.B. in Verfahren zur Herstellung erfindungsgemäßer viraler Partikel und viraler Vektoren, die z.B. Verwendung als Medikament oder in der Herstellung pharmazeutischer Zusammensetzungen finden. Ein erfindungsgemäß bevorzugtes Helferplasmid enthält eine das gag-pro-pol-Fusionsprotein kodierende Sequenz, in der virale Kodons an den humanen Kodongebrauch angepasst sind.

In der Verwendung als Medikament kann der virale Partikel, der den viralen Vektor enthält, zur Verabreichung z.B. an einen Patienten/Menschen oder ein nicht-menschliches Tier hergerichtet sein. Alternativ kann der virale Partikel bzw. der virale Vektor mit Zellen kontaktiert werden, die einem solchen Patienten entstammen, d.h. zur in vitro Transduktion von Zellen, die anschließend, wahlweise mit dem Schritt der Kultivierung und/oder Selektion, als transduzierte Zellen zur Übertragung in einen Patienten hergerichtet bzw. bereitgestellt werden, wobei vorzugsweise der Patient derselbe ist, dem die Zellen entstammen.

Bei der kodon-optimierten kodierenden Sequenz für das gag-pro-pol-Fusionsprotein ist erfindungsgemäß die ursprüngliche Verschiebung des Leserahmens um -1 und der Übergang von pro zu pol vom Wildtyp- ASLV beibehalten worden.

Es hat sich gezeigt, dass die Anpassung des Kodongebrauchs von ASLV an den Kodongebrauch der Zielzelle, insbesondere an den humanen Kodongebrauch, für die kodierenden Bereiche der Helferplasmide eine signifikante Steigerung des Titers viraler Partikel zur Folge haben, wobei z.B. allein die Anpassung des Kodongebrauchs des Fusionsproteins gag-pro-pol an den humanen Kodongebrauch zu einem um den Faktor 50 höheren Titer viraler Partikel in humanen Verpackungszellen führt.

Die Anpassung des Kodongebrauchs an den humanen Kodongebrauch erfolgt vorzugsweise dadurch, dass vorzugsweise die für Homo sapiens optimalen, nicht-limitierten tRNA Pools ausgeschöpft werden; so wurde z.B. das Glycin-Kodon GGT vorzugsweise zu GGC adaptiert. Desweiteren wurden negative RNA-Instabilitätsmotive sowie kryptische Polyadenylierungsstellen sowie RNA-Sekundärstrukturen entfernt, so dass eine stabilere mRNA mit besserer Translatierbarkeit in humanen Zellen generiert wurde. Überdies wurde in der kodierenden Sequenz die natürliche Spleißdonorstelle, die Spleißakzeptorstelle und weitere kryptische Spleiß-signale unwirksam gemacht. Folglich weist ein bevorzugtes Helferplasmid eine inaktive Spleißdonorstelle im gag-pol-Gen auf, z.B. entsprechend Nukleotiden 1501 bis 1502 in Seq. ID Nr. 14. Bevorzugt wird gag von den Nukleotiden 1475 bis 3436 von Seq. ID Nr. 14 kodiert und pol von Nukleotiden 3738 bis 6291 von Seq. ID Nr. 14, da in diesen Abschnitten kryptische RNA-Instabilitätsmotive und Polyadenylierungsstellen sowie RNA-Sekundärstrukturen im Wesentlichen entfernt sind, so dass ein hoher Titer viraler Partikel erzeugt werden kann, die eine hohe Transduktionseffizienz aufweisen.

Schließlich ist bevorzugt, dass der virale Vektor, bevorzugt zusätzlich das erste Helferplasmid und weiter bevorzugt zusätzlich das zweite Helferplasmid, keine Sequenzhomologien zur Zielzelle aufweisen, und überdies bevorzugt insbesondere keine Sequenzüberlappung haben, z.B. keine zueinander homologen Sequenzabschnitte von mehr als 5 Nukleotiden, vorzugsweise keine zueinander homologen Sequenzabschnitte von mehr als 10 bis 20 Nukleotiden. Auf diese Weise wird die Wahrscheinlichkeit für eine Rekombination in Verpackungszellen und/oder den Zielzellen minimiert, und damit auch die Wahrscheinlichkeit der Generierung von RCR.

Die Erfindung stellt damit eine virale RNA zur Verfügung, die von 5' nach 3' eine 5'-R-Region und 5'-U5-Region, eine Primerbindungsstelle (PBS), ein Verpackungssignal (ψ), das im Gegensatz zum Stand der Technik bei gammaretroviralen oder lentiviralen Vektoren ohne einen retroviralen Spleißdonor auskommt, eine Expressionskassette für ein bzw. mit einem Transgen und einer erfindungsgemäß verkürzten (gekennzeichnet als Δ oder d) 3'-U3-Region, einer 3'-R-Region und einem angehängten PolyA-Abschnitt (PolyA-Schwanz) aufweist oder daraus besteht, sowie einen Vektor zur Transkription der viralen RNA, der zusätzlich zu einer Nukleinsäuresequenz, die die virale RNA kodiert, in 5' vor deren 5'-R-Region einen eukaryotischen bzw. viralen Promotor aufweist, und bei dem die Nukleinsäuresequenz, die die virale RNA kodiert, in Form von DNA vorliegt, die im 3'-SIN-LTR einen PolyA-Abschnitt aufweist.

Weiterhin stellt die Erfindung die Verwendung der viralen RNA und eines die virale RNA enthaltenden viralen Partikel als Medikament und zur Herstellung einer pharmazeutischen Zusammensetzung zur Verwendung für die Transduktion von Zielzellen, z.B. von somatischen Zellen eines Menschen bereit, sowie pharmazeutische Zusammensetzungen, die die virale RNA oder die virale RNA enthaltende virale Partikel umfassen, und die Verwendung solcher pharmazeutischen Zusammensetzungen als Medikament zum Transfer des Transgens in Zellen, insbesondere zur Gentherapie.

Weiterhin betrifft die Erfindung Zellen, die eine integrierte oder nicht-integrierte Nukleinsäuresequenz aufweisen, die durch Kontaktierung mit erfindungsgemäßen viralen Partikeln erzeugt sind und eine erfindungsgemäße U3-Region enthalten, z.B. einen DNA-Abschnitt, der durch reverse Transkription aus einer erfindungsgemäßen viralen RNA entstanden ist. Dies können extrakorporale oder intrakorporale tierische Zellen sein, ausgenommen menschliche Keimzellen.

Die Expressionskassette weist einen Promotor und zumindest eine transkribierbare und/oder translatierbare Sequenz eines Transgens auf, das ein zur Zielzelle homologes oder heterologes Gen sein kann, und vorzugsweise stromabwärts der transkribierbaren Sequenz des Transgens ein PRE oder andere posttranskriptionell regulatorische Nukleinsäureabschnitte oder -module. Die transkribierbare Sequenz kann auch interne Ribosomen-Eintrittsstellen (1RES) in Verbindung mit stromabwärts zu diesen angeordneten, Protein kodierenden Sequenzen aufweisen, sowie andere funktionale Nukleinsäureabschnitte oder enzymkodierende Nukleinsäureabschnitte (z.B. bidirektionale Promotoren, kodierende Sequenzen für 2A-Protease-Schnittstellen oder 2A-Protease-Gene). Weiter optional kann in die deletierte 3'-U3-Region, z.B. in die darin enthaltene Restriktionsschnittstelle, eine Isolatorsequenz eingefügt werden, die eine Wechselwirkung des Transgens mit benachbarten genomischen Sequenzen abschwächt, oder eine Rekombinaseerkennungssequenz eingesetzt sein, z.B. eine Erkennungssequenz aus der Gruppe, die rox, loxP, FRT umfasst. Nach erfolgter reverser Transkription und Kopieren in den 5'-LTR kann durch Zugabe bzw. Expression von Rekombinase in Zielzellen die Rekombinaseerkennungssequenz aktiviert werden, und z.B. ein zwischen Rekombinaseerkennungssequenzen angeordneter Nukleinsäureabschnitt in der integrierten DNA (z.B. ein Teil der Expressionskassette) umgedreht oder entfernt werden. Andere Sequenzen, die in die 3' U3 Region eingefügt werden können, umfassen beispielsweise micro-RNA-Zielsequenzen, small hairpin RNAs oder Sequenzabschnitte, die den Nachweis der Insertion über PCR erleichtern.

Die im erfindungsgemäßen 3'-SIN-LTR enthaltene deletierte U3-Region erlaubt trotz der Deletion der Enhancer- und Promotorelemente aus der Wildtyp-U3-Region die Erzeugung von viralen Partikeln mit Titern, die gegenüber den Titern für Vektoren mit Wildtyp-LTR in Verpackungszellen erreicht werden, sodass die häufig bei Deletionen von U3-Regionen beobachteten drastischen Reduktionen des viralen Titers vermieden werden.

Die Transkription von Transgenen, die in der Expressionskassette angeordnet sind, wird durch den Promotor der Expressionskassette kontrolliert, der auch interner Promotor genannt wird. Derzeit wird angenommen, dass die erfindungsgemäß deletierten U3-Regionen ein PolyA-Signal aufweisen, das ein Hindurch-Transkribieren über die Vektorsequenz hinaus vermeidet, und das korrekte PolyA-Signal nutzt. Diese Annahme wird durch die 3-fach bessere Expression eines Transgens der Expressionskassette eines erfindungsgemäßen Vektors im Vergleich zur Expression des Transgens von einem Vektor mit 3'-LTR vom Wildtyp gestützt.

Besonders bevorzugt weist die Nukleinsäuresequenz des viralen Vektors eine vollständige Deletion der für virale Strukturproteine kodierenden Sequenzen einschließlich der Spleißdonorstelle (SD) auf, z.B. eine vollständige Deletion der Sequenzen gag, env, pol und src. Es hat sich gezeigt, dass bei ASLV-Vektoren mit Deletion der die viralen Strukturproteine kodierenden Sequenzen auch die Spleißdonorstelle deletiert wird. Überraschender Weise hat sich gezeigt, dass ein solcher Vektor auf Basis von ASLV die Verpackung der viralen RNA in Verpackungszellen mittels des Split-Packaging-Systems erlaubt. Dies ist insofern unerwartet, als in bekannten retroviralen Vektoren Anteile der kodierenden viralen Sequenzen für die Verpackung erforderlich sind, da im Bereich der die viralen Strukturproteine kodierenden Sequenzen Signale für den RNA-Export, die Wechselwirkung mit dem viralen Nukleocapsid und die reverse Transkription enthalten sind, wie z.B. in HIV. Daher kann der Vektor das Verpackungssignal (ψ), das in Seq. -ID No. 5 durch die Nukleotide 1042 - 1292 umfasst ist, ohne Abschnitte von Sequenzen enthalten, die virale Strukturproteine kodieren.

Die erfindungsgemäß bevorzugte Ausführungsform, in der die Nukleinsäuresequenz des viralen Vektors keinen viralen Spleißdonor aufweist, ist ein bedeutender Unterschied zu bekannten lentiviralen oder γ-retroviralen Vektoren, die den natürlichen viralen Spleißdonor stromabwärts der Primerbindungsstelle aufweisen. Diese bekannten Vektoren benötigen den Spleißdonor zur Erzeugung hoher Titer viraler Partikel. Überraschender Weise hat sich gezeigt, dass der Vektor auch ohne seinen natürlichen oder einen anderen Spleißdonor hohe Titer in Verpackungszellen ergibt. Da der natürliche Spleißdonor von ASLV im gag-Gen liegt, führt dessen vollständige Deletion auch zur Entfernung des Spleißdonors. Weiter bevorzugt ist erfindungsgemäß auch der virale Spleißakzeptor nicht im Vektor enthalten. Der Spleißakzeptor von ASLV liegt im pol-Gen, so dass dessen vollständige Deletion auch zur Entfernung des Spleißakzeptors führt.

Beim Vektor führt die Deletion von Spleißdonor und Spleißakzeptor zur Optimierung der Produktion viraler Partikel in Verpackungszellen und verleiht dem Vektor zusätzlich eine bessere biologische Sicherheit, da die Rekombination zu replikationskompetenten Retroviren erschwert ist. Auch ist bei intragenischen Insertionen des Vektors eine mögliche Interferenz mit zellulären Spleißsignalen weniger wahrscheinlich.

Der Vektor im 5'-Bereich eine R-Region, eine U5-Region und das Verpackungssignal in einer Nukleinsäuresequenz auf, die keine viralen Strukturproteine kodiert, und an welche sich z.B. unmittelbar, insbesondere nach der Leaderregion, in 3' die Expressionskassette des Transgens anschließt. Es hat sich gezeigt, dass der virale Vektor an seinem 5'-Ende eine R-Region, U5-Region und ein Verpackungssignal aufweisen kann, das von den Nukleotiden Nrn. 1042 bis 1292 der Seq.-ID Nr. 5 umfasst ist oder daraus besteht, wobei vorzugsweise in 3' angrenzend an diese Sequenz die Expressionskassette mit dem Transgen angeordnet ist.

Für die Zwecke der Erfindung werden die Anordnungen von genetischen Elementen in 5' nach 3' angegeben, sofern nicht anders beschrieben; Nukleotidsequenzen sind von 5' nach 3' angegeben.

### Genaue Beschreibung der Erfindung

Die Erfindung wird nun genauer anhand von Beispielen mit Bezug auf die Figuren beschrieben, in denen
- Figur 1 die schematische Struktur des ASLV-Wildtyps zeigt,
- Figur 2 die schematische Struktur eines SIN-Vektors und eines den Vektor enthaltenden Plasmids zeigt,
- Figur 3 die schematische Struktur eines ersten Helferplasmids zeigt,
- Figur 4 die schematische Struktur eines zweiten Helferplasmids zeigt,
- Figur 5 einen Vergleich der Wildtyp-U3-Region (U3) mit der erfindungsgemäß deletierten U3-Region (dU3), der erfindungsgemäß deletierten U3-Region ohne TATA- Box(dU3 noTATA) und mit der erfindungsgemäß deletierten U3-Region mit mutierter TATA-Box (dU3 TATAmut) zeigt,
- Figur 6 eine Plasmidkarte eines erfindungsgemäßen Vektors zeigt, in dem die Expressionskassette EGFP als Beispiel für ein Transgen kodiert,
- Figur 7 eine Plasmidkarte mit erfindungsgemäßem Vektor zeigt, in dem die TATA-Box der U3-Region mutiert ist,
- Figur 8 eine Plasmidkarte mit erfindungsgemäßem Vektor zeigt, in dessen U3-Region zusätzlich die TATA-Box deletiert ist,
- Figur 9 eine Plasmidkarte für ein erstes Helferplasmid zur Translation des gag-pol-Fusionsproteins zeigt,
- Figur 10 eine Plasmidkarte für ein erstes Helferplasmid zeigt, bei dem die das gag-pol-Fusionsprotein kodierende Sequenz für eine humane Verpackungs- und/oder Zielzelle kodonoptimiert ist,
- Figur 11 grafisch die Höhe der Expression eines Transgens (EGFP) von erfindungsgemäßem Vektor im Vergleich zur Expression von einem viralen Vektor mit Wildtyp-3'-LTR zeigt,
- Figur 12 grafisch die Höhe der Expression eines Transgens (EGFP) von erfindungsgemäßen SIN-Vektoren zeigt,
- Figur 13 eine Darstellung der integrierten Struktur eines erfindungsgemäßen Vektors im Vergleich zur Struktur eines lentiviralen bzw. γ-retroviralen SIN-Vektors zeigt,
- Figur 14 schematisch DNA-Nukleinsäurekonstrukte für die Transkription viraler RNA mit Expressionskassetten zeigt, die funktional in 5' zu einem Reportergen die auf Promotor- und Enhanceraktivität zu testende Nukleinsäuresequenz enthält und
- Figur 15 die Expressionshöhe des Reportergens in Zellen zeigt, die mit viraler RNA von Figur 14 transfiziert sind und die Promotor-/Enhanceraktivität misst.

Bei der Beschreibung der erfindungsgemäßen SIN-U3 wird die Nummerierung der Nukleotide in Bezug auf die Nukleotidsequenz der Wildtyp-U3 von ASLV vorgenommen, und entsprechend sind Deletionen bzw. Abschnitte der SIN-U3-Region entsprechend der Nummerierung der Nukleotide der Wildtyp-U3 bezeichnet.

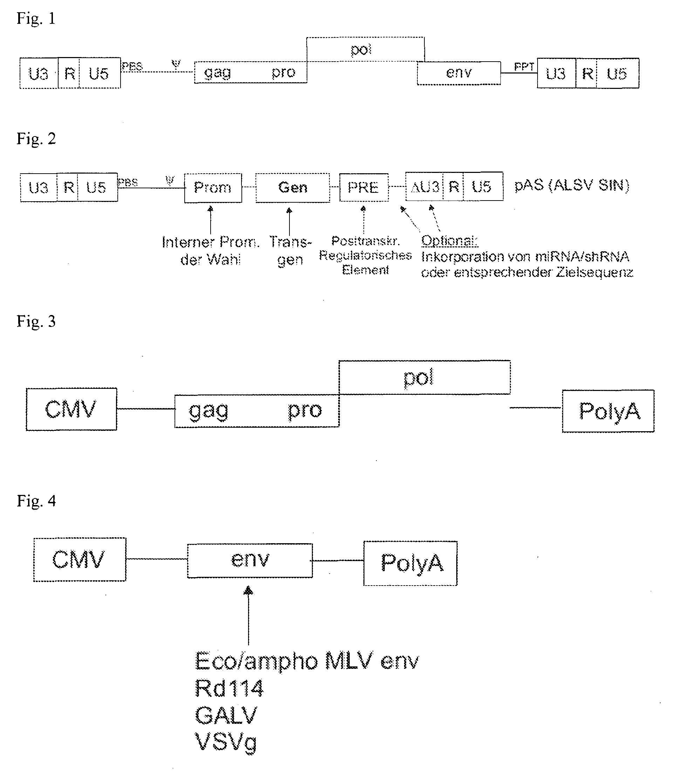

Figur 1 zeigt den schematischen Aufbau des ASLV vom Wildtyp, bei dem die LTRs eine U3-Region, eine R-Region und eine U5-Region aneinander angrenzend enthalten. Die LTRs sind jeweils identisch, da bei der viralen Replikation die U3 Region des 3'-LTR an das 5'-Ende kopiert wird sowie die R und U5 Regionen vom 5'LTR in den 3'LTR kopiert werden. Der Transkriptionstart +1 definiert das 5'-Ende der 5'R-Region.

Figur 2 zeigt den Vektor auf Plasmidebene, an dessen 5'-Ende ein Promotor vor der R-Region angeordnet ist, um die Transkription ab der 5'-R-Region zu ermöglichen. Dieser 5'-Promotor des 5'-LTR ist wegen des Transkriptionstarts bei +1 als erstem Nukleotid der 5'-R-Region nicht in der viralen RNA enthalten; die virale RNA, die erzeugt wird, besteht aus den Bereichen zwischen dem Nukleotid +1 der 5' R-Region und dem 3'-Ende der 3'-R-Region plus polyA-Schwanz. Entsprechend enthalten virale Partikel eine virale RNA, die aus dem Abschnitt des Vektors entsteht, der die 5'-R-Region (einschließlich Nukleotid +1) bis zum 3'-Ende der 3'-R-Region umfasst, mit anhängendem PolyA-Schwanz. Der in Figur 2 stromaufwärts der 5'-R-Region gezeigte Promotor des viralen Vektors, der vorzugsweise aus einem SV40-Enhancer und einer U3-Region vom Wildtyp des RSV (Rous Sarcoma Virus) besteht, wird nicht Bestandteil der viralen RNA, sondern steuert die effiziente Transkription der viralen RNA von einer Figur 2 entsprechenden DNA-Sequenz. Vorliegend ist der Vektor aus Plasmidebene gezeigt, als eine die virale RNA kodierende DNA-Sequenz, die Bestandteil eines Plasmids sein kann, von dem die virale RNA transkribiert wird.

Wie in Figur 2 angedeutet, enthält der virale Vektor zwischen den LTRs, und entsprechend nach der Leaderregion, eine Expressionskassette, die zumindest einen internen Promotor aufweist, beispielsweise den Promotor von SFFV und EFS (Elongationsfaktor 1α), mit einer Nukleotidsequenz, die ein Transgen (Gen) kodiert, vorzugsweise mit einem in 3' angeordneten PRE.

Das PRE, das vorzugsweise WPRE ist, der Expressionskassette weist vorzugsweise eine X-ORF Deletion auf und/oder ATG-Mutationen:

Der Vektor und die erfindungsgemäße virale RNA weisen zumindest im 3'-LTR eine U3-Region mit Deletionen auf, die jegliche Promotoraktivität des LTRs ausschalten. In 5' vor dem 3'-SIN-LTR und/oder innerhalb der 3'-U3-Region kann der virale Vektor homologe und/oder heterologe genetische Elemente enthalten, beispielsweise Sequenzen, die miRNA, shRNA, Polyadenylierungsverstärker, Rekombinaseerkennungssequenzen, USE und/oder Isolatoren umfassen.

Zwischen den Elementen des 5'-LTR und des 3'-LTR, vorzugsweise zwischen den Regionen des 5'-LTR und der Expressionskassette, weist der Vektor vorzugsweise eine Primerbindungsstelle und ein Verpackungsignal (ψ) auf.

Im Unterschied zu replikationskompetenten Retroviren weist der virale Vektor keine Sequenzen auf, die für gag-pol und/oder Hüllproteine kodieren.

Die viralen Proteine, die zur Erzeugung eines viralen Partikels der viralen RNA erforderlich sind, werden von separaten Helferplasmiden kodiert, vorzugsweise einem ersten Helferplasmid, das eine für das gag-pro-pol-Fusionsprotein kodierende Sequenz in einer Expressionskassette zwischen einem Promotor und einer Polyadenylierungssequenz aufweist, wie in Figur 3 gezeigt, und einem zweiten Helferplasmid, das eine das Hüllprotein (env) kodierende Sequenz in einer Expressionskassette enthält, beispielsweise wie in Figur 4 gezeigt zwischen einem Promotor aus CMV (Cytomegalie-Virus) und einer Polyadenylierungsequenz. Zur Pseudotypisierung kann die das Hüllprotein kodierende Sequenz aus einer Sequenz bestehen, die das Hüllprotein von MLV Ecotrop/Amphotrop, VSVg, RD114, GALV oder Chimären dieser kodieren, z.B. RD114/TR.

Das erste Helferplasmid weist in seiner kodierenden Sequenz vorzugsweise die Leserahmenverschiebung um -1 zwischen dem 3'-Ende der gag-pro kodierenden Sequenz und dem 5'-Ende der pol kodierenden Sequenz auf, was in Figur 3 durch die zueinander versetzten Kästen für gag-pro bzw. pol gezeigt ist.

Besonders bevorzugt sind die kodierenden Sequenzen der Helferplasmide an den Kodongebrauch der Zielzelle angepasst, d.h. kodonoptimiert, da sich für die Erfindung gezeigt hat, dass der Titer sich durch diese Kodonoptimierung für das gag-pro-pol-Fusionsprotein, vorzugsweise auch für das Hüllprotein env eine Erhöhung des Titers in Helferzellen um ca. den Faktor 50 ergibt. Daher ermöglicht die Erfindung ein Produktionsverfahren in Titern bzw. Mengen viraler Partikel, wie sie für klinische Anwendungen erforderlich sind, durch Herstellung viraler Partikel, die die erfindungsgemäße virale RNA enthalten, mit erstem und zweitem Helferplasmid, z.B. durch Kultivieren einer Verpackungszelle.

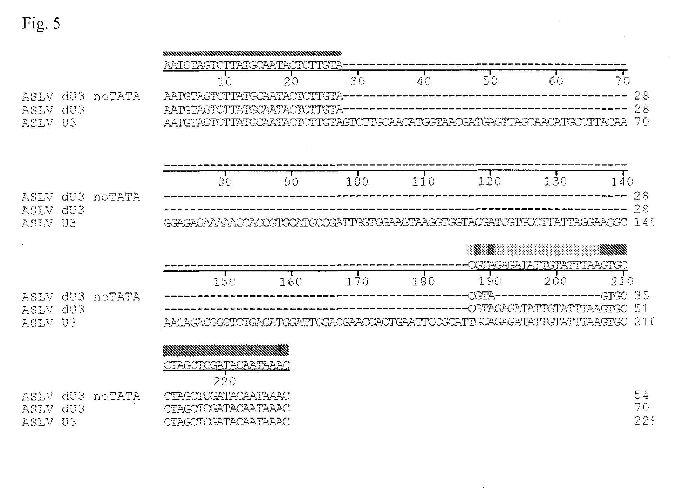

Figur 5 zeigt eine Übersicht über die bevorzugten Sequenzen der erfindungsgemäßen 3'-U3-Region des viralen Vektors im Vergleich zur Sequenz der Wildtyp-U3 (Seq.-ID Nr. 1) von ASLV. Die erfindungsgemäßen U3-Regionen weisen vorzugsweise eine Deletion der Nukleotide 28-186 der Wildtyp-U3 auf. Die Ausführungsform Seq.-ID Nr. 2, als Delta (d) U3 bezeichnet, besteht vorzugsweise aus den Nukleotiden 1-27 und den Nukleotiden 187-229 der Wildtyp-U3, wobei noch die Nukleotide 187 und 189 zu C bzw. T mutiert sind, sodass sich dort eine Restriktionsschnittstelle für SnaBI (TACGTA) ergibt. Diese deletierte U3-Region, auch als SIN-U3 bezeichnet, weist noch die ursprüngliche TATA-Box auf, die als Nukleotide 200-206 der Wildtyp-U3 identifiziert wurde.

In einer bevorzugten Ausführungsform ist auch die TATA-Box der SIN-U3 mutiert, sodass die Funktion einer TATA-Box aufgehoben ist. Eine bevorzugte Ausführung einer mutierten TATA-Box ist in Seq.-ID Nr. 3 (dU3 TATAmut) gezeigt, bei der die Nukleotide 201-203 mutiert sind, sodass die Funktion der TATA-Box zerstört ist.

In einer bevorzugteren Ausführungsform umfasst die Deletion in Bezug auf die Wildtyp-U3-Region die Nukleotide 28-205 (dU3 noTATA; Seq.-ID Nr. 4), wobei die Nukleotide 203-204 mutiert sind, um die Erzeugung einer TATA-Box durch die aneinander angrenzenden Abschnitte zu vermeiden, und/oder um eine Schnittstelle an den aneinander angrenzenden Nukleotiden 26-27 und 203-206 herzustellen, beispielsweise durch Mutation der Nukleotide 203-205 zu CGT, um eine singuläre Schnittstelle für das Restriktionsenzym SnaBI (TACGTA) herzustellen.

Virale Vektoren umfassen daher auch Varianten, bei denen im Bereich der Deletion der Nukleotide 28-222, vorzugsweise im Bereich der Nukleotide 28-190 bzw. 28-206 in Seq. ID Nr. 1 heterologe oder homologe, z.B. in Bezug auf die Zielzelle heterologe oder homologe genetische Elemente inseriert sein können, vorzugsweise mittels einer Restriktionsschnittstelle in den vorgenannten Abschnitten.

### Beispiel 1: Produktion viraler Partikel mit ASLV-SIN Vektor

Als Beispiele für erfindungsgemäße Vektoren, die für eine erfindungsgemäße virale RNA kodieren, wurden Plasmide konstruiert, die unter der Kontrolle eines Promotors, der aus einem Verstärkungselement aus SV40 (SV40 Enhancer) und dem RSV-Promotor (RSV) und unmittelbar angrenzend an den Promotor die R-Region, die U5-Region, eine Expressionskassette für ein Transgen und einen 3'-SIN-LTR aus einer erfindungsgemäßen SIN-U3-Region, einer R-Region und einer U5-Region aufwies. Die Expressionskassette umfasste den U3-Promotor des Spleen-Focus-Forming Virus (SFFV), eine für EGFP kodierende Sequenz als Beispiel für ein Transgen, und ein WPRE (PRE).

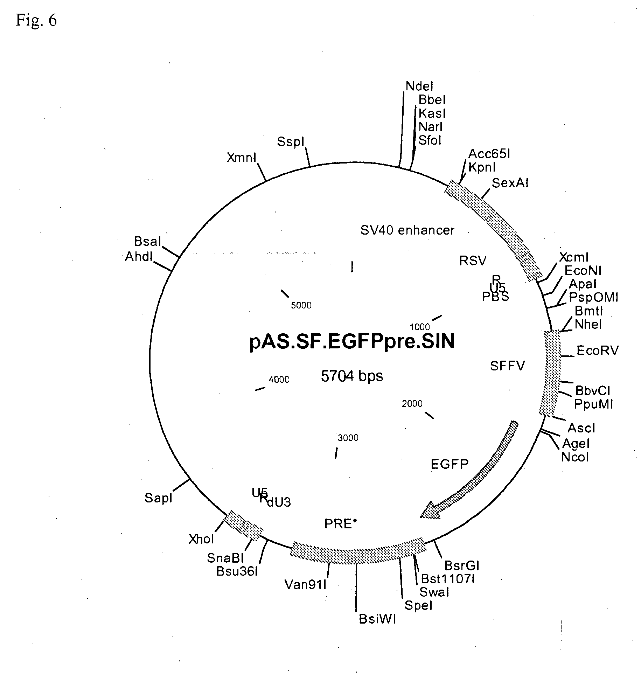

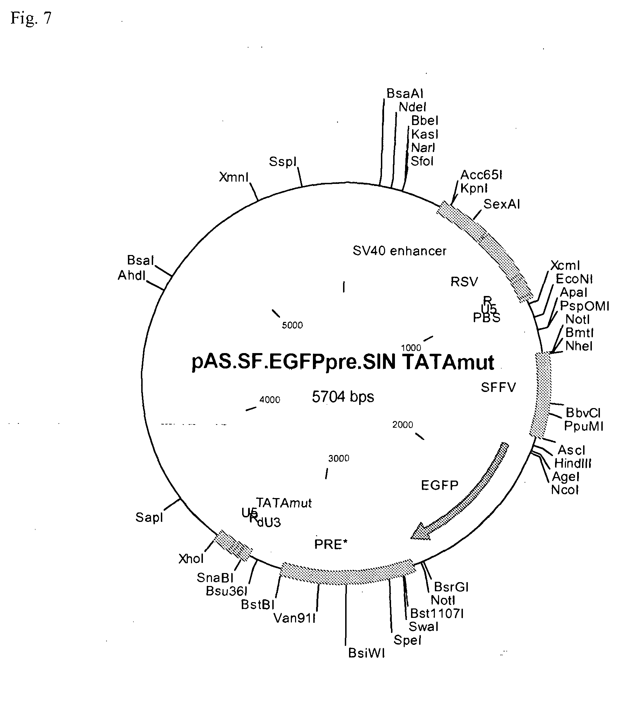

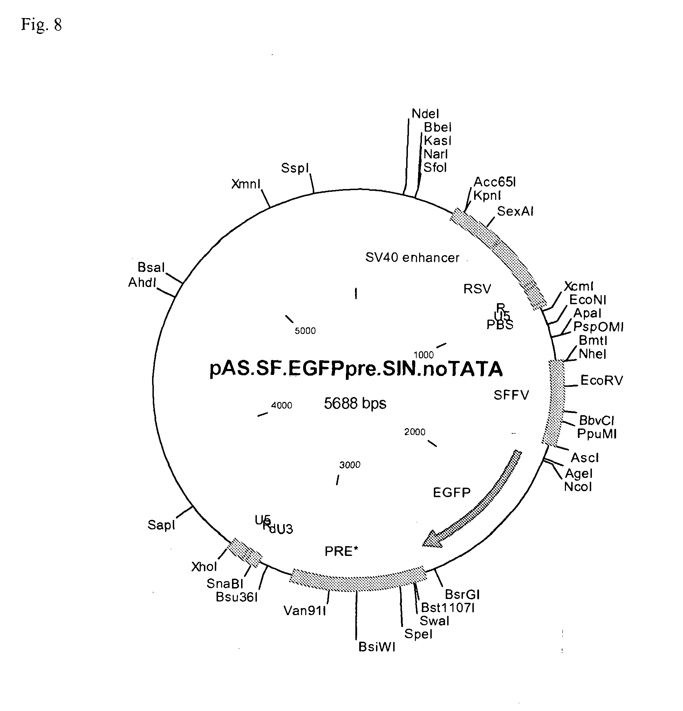

Die SIN-U3-Region entsprach der Seq.-ID Nr. 2 für den in Figur 6 gezeigten Vektor, alternativ der Seq.-ID Nr. 3 für den in Figur 7 gezeigten Vektor, bzw. der Seq.-ID Nr. 4 für den in Figur 8 gezeigten Vektor.

Die Sequenz des Vektors von Figur 6 ist Seq.-ID Nr. 5 enthalten. Entsprechend enthält ein Vektor, bzw. ein Plasmid, das eine erfindungsgemäße RNA kodiert, einen 3'-SIN-LTR mit einer Sequenz entsprechend Nukleotiden 3282-3452 von Seq.-ID Nr. 5. Die davon transkribierte virale RNA enthält die darin enthaltene erfindungsgemäße 3'-U3-Region.

Die Sequenz des Vektors von Figur 7 ist Seq.-ID Nr. 9 enthalten. Entsprechend enthält ein Vektor, von dem eine erfindungsgemäße virale RNA transkribiert wird, einen 3'-SIN-LTR mit einer Sequenz entsprechend den Nukleotiden 3282 - 3452 von Seq.-ID Nr. 9. Die virale RNA enthält am 3'-SIN-LTR die erfindungsgemäße 3'-U3-Region und R-Region, nicht jedoch die U5-Region.

Die Sequenz des Vektors von Figur 8 ist Seq.-ID Nr. 7 enthalten. Entsprechend enthält ein Vektor, von dem eine erfindungsgemäße virale RNA transkribiert wird, einen 3'-SIN-LTR mit einer Sequenz entsprechend den Nukleotiden 3282 - 3436 von Seq.-ID Nr. 7.

Weiter enthält ein viraler Vektor, bzw. eine virale RNA eine Wildtyp-R-Region und eine Wildtyp-U5-Region als 5'-LTR, und unmittelbar in 5' daran angrenzend, in der Form, in der die Nukleotidsequenz für die virale RNA auf einem Plasmid enthalten ist, einen eukaryotischen oder viralen Promotor, vorzugsweise die Verstärkungssequenz aus SV40 in Verbindung mit dem Promotor aus RSV, entsprechend Nukleotiden 452-921 aus Seq.-ID Nr. 9.

Die für eGFP kodierende Sequenz der Seq.-ID Nr. 5 ist als Aminosäuresequenz Seq.-ID Nr. 6 angegeben, eGFP aus Seq.-ID Nr. 7 als Seq.-ID Nr. 8, und eGFP aus Seq.-ID Nr. 9 als Seq.-ID Nr. 10.

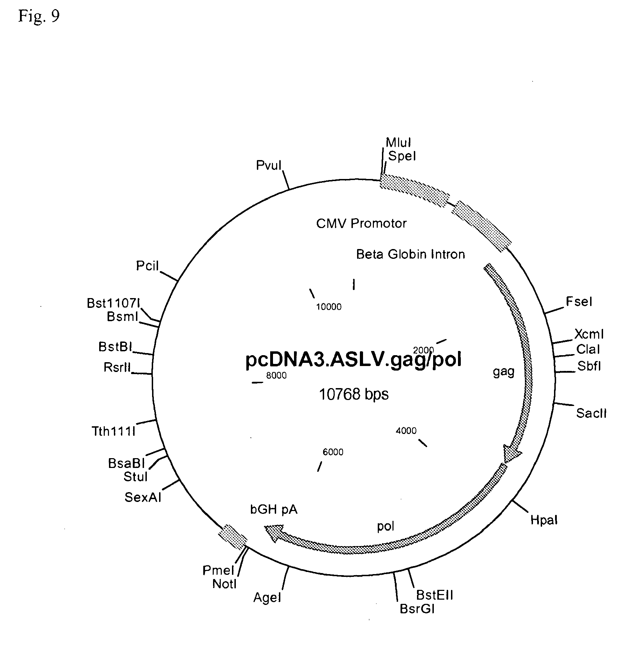

Zur Erzeugung viraler Partikel wurden die Plasmide nach Figuren 6 bis 8 in einer HEK293T-Zellen eingebracht, die mittels transienter Transfektion oder stabiler Integration auch ein erstes Helferplasmid zur Expression des gag-pol-Fusionsproteins von ASLV enthielten. Eine schematische Plasmidkarte ist in Figur 9 gezeigt, die Sequenz ist als Seq.-ID Nr. 11 beigefügt und enthält als kodierende Sequenz das gag-pol-Fusionsprotein bei Nukleotiden 1475-6291. Die von Seq.-ID Nr. 11 kodierte Aminosäuresequenz von gag entspricht Seq.-ID Nr. 12, die von Seq.-ID Nr. 11 kodierte Aminosäuresequenz von pol entspricht Seq.-ID Nr. 13.

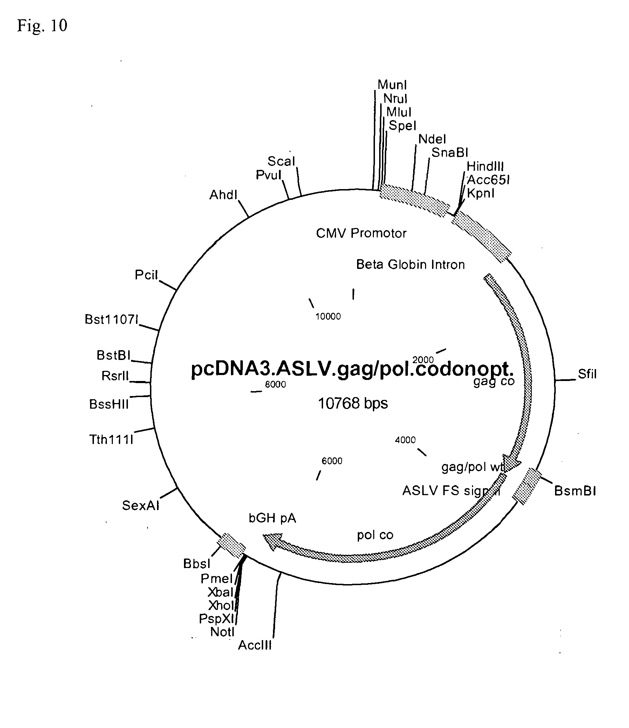

Besonders bevorzugt war das erste Helferplasmid mit einer kodierenden Sequenz für das gag-pol-Fusionsprotein versehen, dessen Kodons an den Kodongebrauch der Verpackungszelle, insbesondere von Säugerzellen, wie z.B. menschliche Zellen angepasst waren. Ein solches Plasmid ist schematisch in Figur 10 gezeigt; die Nukleinsäuresequenz ist als Seq.-ID Nr. 14 enthalten. Entsprechend enthält ein bevorzugtes erstes Helferplasmid, bzw. eine menschliche Verpackungszelle in integrierter Form die kodierende Sequenz für das kodonoptimierte gag-Protein entsprechend Nukleotiden 1475-3436 von Seq.-ID Nr. 14, dessen Aminosäuresequenz Seq.-ID Nr. 15 entspricht, und/oder die kodierende Sequenz für das kodonoptimierte pol-Protein entsprechend Nukleotiden 3738-6291 von Seq.-ID Nr. 14, dessen Aminosäuresequenz Seq.-ID Nr. 16 entspricht, bevorzugt die kodierende Sequenz für das kodonoptimierte gag-pol-Fusionsprotein entsprechend Nukleotiden 1475-6291 der Seq.-ID Nr. 14.

Unter üblichen Kultivierungsbedingungen der Verpackungszelle_wurden für die erfindungsgemäßen viralen Partikel mit einer der gezeigten SIN-U3-Regionen von den Vektoren der Seq.-ID Nr. 7, 9 oder 11 jeweils gleiche Titer erhalten, wie für ein Vergleichkonstrukt (nicht gezeigt), bei dem die 3'- U3-Region dem Wildtyp von ASLV entsprach, nämlich ca. 1 x 10⁶ /mL.

Es ist daher für das erfindungsgemäße Verfahren zur Herstellung viraler Partikel bevorzugt, dass die Verpackungszellen kultivierte humane Zellen sind, die eine Nukleinsäuresequenz aufweisen, die die viralen Strukturgene, z.B. gag-pol, mit humanem Kodongebrauch kodiert, wobei diese Strukturgene bevorzugt keine aktive Spleißdonor und/oder Spleißakzeptorstelle aufweisen, um ein Spleißen innerhalb der kodierenden Sequenzen zu verhindern und damit eine hohe Expression in den Verpackungszellen zu erreichen.

Zur Kontrolle der Transfektion von Zielzellen mit viraler RNA wurden menschliche HT1080 Fibroblasten infiziert. Im Anschluss an die Kultivierung nach der Transfektion wurde die Expression des Transgens (EGFP) per FACS bestimmt. Beim Vergleich mit viralen Partikeln, deren 3'-LTR vom Wildtyp-ASLV war, konnte für die beschriebenen viralen Vektoren mit 3'SIN-LTR eine um den Faktor 3 höhere Expression des Transgens gezeigt werden.

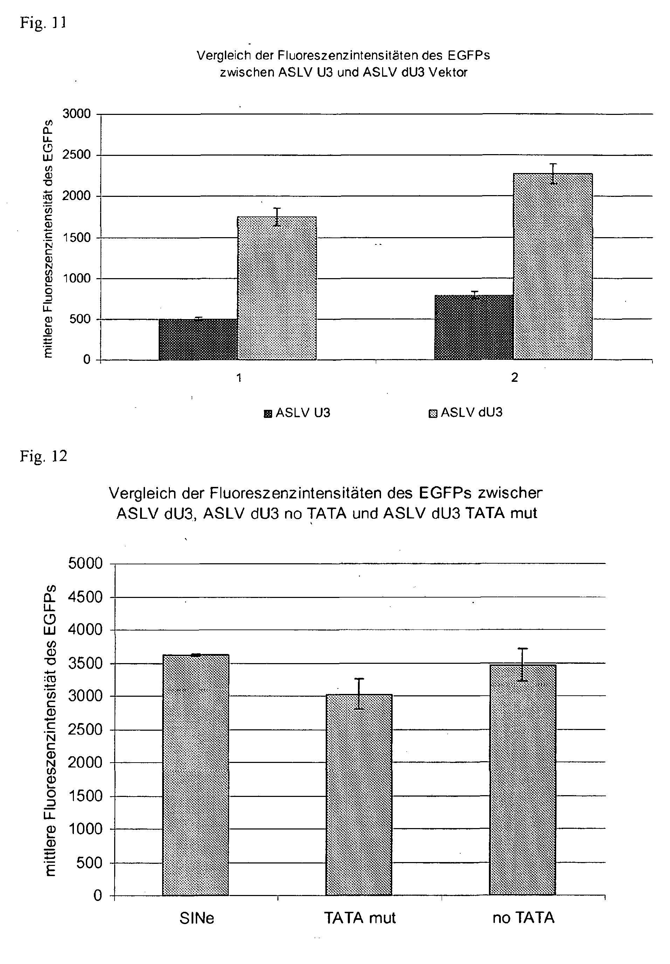

Figur 11 zeigt die Expression des beispielhaften Transgens EGFP von einer Expressionskassette entsprechend Figur 7 in Zielzellen, die mit viralem Vektor transduziert waren. Virale Partikel wurden aus dem Überstand von Verpackungszellen (pseudotypisiert mit VSVg) gewonnen, die ein Plasmid enthielten, das erfindungsgemäße virale RNA gemäß Seq.-ID Nr. 5 kodierte (ASLV dU3, helle rechte Balken), bzw. eine virale RNA mit 3'-U3 vom Wildtyp (ASLV U3, dunkle linke Balken; Wildtyp U3 s. Seq.-ID Nr. 1). Zur Transduktion wurden virale Partikel auf HT1080E-Zellen titriert, 6 Tage kultiviert und anschließend durchflußzytometrisch analysiert. In Experiment 1 war das gag-pol-Fusionsprotein vom Wildtyp (Nukleotide Nr. 1475 bis 6291 Seq.-ID Nr. 11), in Experiment 2 kodonoptimiert (Nukleotide Nr. 1475 bis 6291 in Seq.-ID Nr. 14). Das Ergebnis, dass das erfindungsgemäße virale Partikel mit einer erfindungsgemäßen 3'-U3-Region gegenüber dem viralen Partikel mit U3-Region vom Wildtyp eine ca. 3-fach höhere Expression des Transgens in Zielzellen verursachte. Dies gilt sowohl für die Verwendung des Wildtyp gag/pol (Experiment 1) als auch des kodonoptimierten gag-pol Helferplasmids (Experiment 2). In beiden Fällen kommt es zu einer etwa 3-fachen Steigerung der Expression des Transgens in Zielzellen für erfindungsgemäße virale Partikel (dU3) im Vergleich mit den Wildtyp Vergleichspartikeln (U3).

| | mittlere Fluoreszenzintensität | |
|---|---|---|
| Experiment | ASLV U3 | ASLV dU3 |
| 1 (Wildtyp gag-pol) | 503,8 +/- 15,1 | 1748,2 +/- 112,6 |
| 2 (kodonoptimiertes gag-pol) | 791,3 +/- 48,7 | 22275,6 +/- 120,6 |

Figur 12 zeigt die Werte der Expression des beispielhaften Transgens EGFP von einer Expressionskassette in Zielzellen, die mit viralem Vektor transduziert waren, die von einem Plasmid nach Seq. ID Nr. 5 (SIN, erfindungsgemäß), Seq. ID Nr. 9 (TATAmut) bzw. Seq. ID Nr. 7 (noTATA) transkribierte virale RNA enthielten. Wie voranstehend beischrieben, wurden virale Partikel in Verpackungszellen erzeugt, die kodonoptimiertes gag-pol-Fusionsprotein und VSVg-Hüllprotein translatierten. HT1080-Zellen wurden mit viralen Partikeln transduziert, 6 Tage kultiviert und dann per FACS analysiert. Die gezeigten Werte machen deutlich, dass die erfindungsgemäße 3'-U3-Region in allen erfindungsgemäßen viralen RNAs bzw. viralen Partikeln eine hohe Expressionseffizienz des Transgens in Zielzellen ergibt. Die Ergebnisse sind auch in der nachfolgenden Tabelle dargestellt:

| | mittlere Fluoreszenzintensität |
|---|---|
| ASLV dU3 | 3616,9 +/- 15,8 |
| ASLV dU3 TATAmut | 3020,4 +/- 227,8 |
| ASLV dU3 noTATA | 3463,2 +/- 252,8 |

Eine bevorzugte Ausführung des Vektors, z.B. dessen virale RNA, wahlweise in viralen Partikeln enthalten, weist keine kodierenden viralen Sequenzen auf, z.B. keine Sequenzen, die eines der Strukturgene gag, env, src oder pol vollständig oder teilweise umfassen. Daher ist bevorzugt, dass die virale RNA des Vektors, bzw. die transkribierte Nukleinsäuresequenz eines Plasmids, das die virale RNA des Vektors codiert, die 5'-R-Region, die 5'-US-Region und der Bereich, der das Verpackungssignal umfasst und sich bis angrenzend an die Expressionskassette des Transgens erstreckt, aus diesen Bereichen besteht, und z.B. auch keine Spleißdonorstelle enthält. Eine bevorzugte Sequenz für die 5'-R-Region, die 5'-U5-Region und den Bereich, der das Verpackungssignal umfasst und sich bis angrenzend an die Expressionskassette des Transgens erstreckt, umfasst die Nukleotide Nrn. 922 bis 1292 der Seq.-ID Nr. 5 oder besteht aus dieser Nukleotidsequenz. Diese Nukleotidsequenz kann optional in 3' verkürzt sein, z.B. um 140 nt, bevorzugt um 10 bis 56 nt.

In dieser Form, in der der Bereich der viralen RNA an ihrem 5'-Ende, und damit in 5' unmittelbar angrenzend an die Expressionskassette des Transgens, aus untranslatierten Sequenzen besteht, insbesondere aus einer R-Region, einer U5-Region und dem Verpackungssignal, sind daher keine Abschnitte von gag, env, src oder pol enthalten.

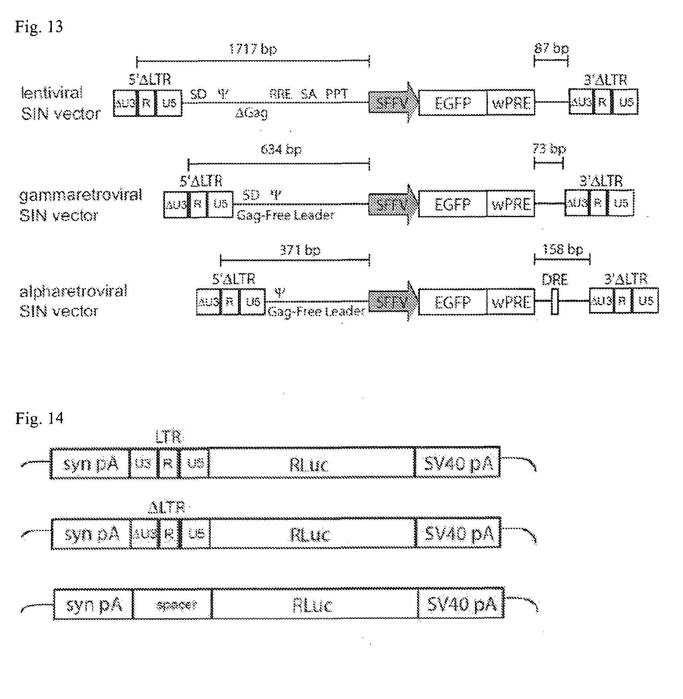

Diese Ausführungsform ist schematisch in Figur 13 gezeigt, die deutlich macht, dass dieser 5'-Abschnitt des Vektors, der auch als Leader bezeichnet wird, deutlich kürzer als der 5'-Bereich von lentiviralen oder γ-retroviralen SIN-Vektoren ist. Während bei lentiviralen und vielen Versionen von γ-retroviralen SIN-Vektoren das Verpackungssignal ψ mit viralen Strukturgenen überlappt, kann überraschender Weise im alpharetroviralen Vektor das Verpackungssignal ohne kodierende virale Sequenzabschnitte enthalten sein. Daher ist der erfindungsgemäße Vektor (alpharetroviral SIN vector) als Gag-Free Leader bezeichnet. Überdies kann im Vektor die virale Spleißdonorstelle entfernt bzw. durch Mutation funktionslos gemacht sein. Dies ist wegen Überlappung mit Verpackungsfunktionen z.B. in lenti- oder γ-retroviralen Vektoren nur unter Minderung des Titers möglich. Weiterhin erlaubt die vollständige Entfernung kodierender viraler Sequenzabschnitte die Kürze des Abschnitts der Vektorsequenz, der in 5' vor der Expressionskassette des Transgens angeordnet ist, z.B. von 371 nt. (Nukleotiden, bp). Damit wird im Vergleich zu lentiviralen und γ-retroviralen Vektoren besonders viel Raum für die Aufnahme von Transgensequenzen geschaffen. Die Expressionskassette besteht in diesen Beispielen jeweils aus dem starken internen Promotor von SFFV, der codierenden Sequenz für grün fluoreszierendes Protein (EGFP) als Reportergen und dem wPRE.

### Beispiel 2: Langzeitexpression des Transgens in eukaryotischen Zielzellen

Als Beispiel für die Verwendung des viralen Vektors als Medikament, z.B. zur Gentherapie, wurden Blutstammzellen mit erfindungsgemäßen viralen Partikeln transduziert. Isolierte Knochenmarkzellen aus Mäusen (Stamm C57BL6) wurden mit erfindungsgemäßen viralen Partikeln transduziert, und zum Vergleich mit lentiviralen Partikeln. Als Transgen war jeweils EGFP in derselben Expressionskassette unter der Kontrolle des SFFV-Promotors enthalten. Unter Bedingungen gleicher Effizienz wurden die Zellen vom lentiviralen Vektor zu 39% transfiziert, und damit um den Faktor 1,6 mehr als durch den beschriebenen Vektor (24%). Dies lässt sich darauf zurückführen, dass lentivirale Partikel auch ruhende, teilungsinaktive Zellen transduzieren können, während die erfindungsgemäßen α-retroviralen Partikel Zellen vorzugsweise während der Teilung transduzieren können.

Die transduzierten Blutstammzellen wurden in je 8 letal bestrahlte Mäuse des gleichen Stamms transplantiert. Transduzierte Zellen wurden per FACS aus peripherem Blut identifiziert. Nach 31 Wochen war der Anteil transduzierter, EGFP-positiver Leukozyten für lentivirale Vektoren mit 32,9 +/- 8,9% etwa auf das doppelte des Werts für die erfindungsgemäßen Vektoren (16,1+/-12%) gestiegen.

Von beiden Gruppen wurde von den Tieren mit den höchsten Titern EGFP-positiver Zellen Knochenmark entnommen und jeweils in 5 letal bestrahlte Tiere einer zweiten Gruppe transplantiert. 6 Wochen nach dieser Transplantation lag der Anteil EGFP-positiver Leukozyten für die lentiviral transduzierten Stammzellen mit 40,6 +/-5,9 noch etwa 30% über dem Anteil bei erfindungsgemäß transduzierten Stammzellen (32,6 +/- 8,1%). Dies zeigt, dass die Geschwindigkeit der epigenetischen Stilllegung der Expression des Transgens für die Vektoren nicht wesentlich höher ist als bei lentiviralen Vektoren.

### Beispiel 3: Nachweis geringen Risikos durch Insertionsmutagenese der Vektoren

In einem von den Erfindern (Mol. Ther. 1919-1928 (2009)) entwickelten in vitro Test wurde das Risiko der Onkogenaktivierung bei der Transduktion bzw. Transfektion mit dem viralen Vektor überprüft. Eine Onkogenaktivierung, z.B. durch Insertion viraler Sequenzen in der genomischen Nachbarschaft, führt dabei zur Transformation primärer hämatopoetischer Zellen. Kurz gesagt wird in diesem Test untersucht, in welcher Frequenz und in welchem Ausprägungsgrad es zur insertionellen Aktivierung transformationsfördernder zellulärer Proto-Onkogene wie *Evil* kommt. In diesem Test werden primäre hämatopoetische Zellen der unbehandelten Maus, z.B. Stamm C57BL6, mit viralen Partikeln kontaktiert. Nach dieser Transduktion wird die Transformationshäufigkeit durch Kultivieren unter Bedingungen bestimmt, die die myeloische Differenzierung verursachen, wobei die neuerliche Aussaat von solchen Zellverdünnungen durchgeführt wird, in denen nicht transformierte Zellen wegen der durch die Verdünnung ausgelösten Unterdrückung der verbleibenden Teilungsfähigkeit nicht proliferieren, sondern nur transformierte Zellen proliferieren. In transformierten Zellen ist z.B. die Expression von Proto-Onkogenen durch Insertionsmutagenese hochreguliert. Dieser Test quantifiziert daher die Transformationshäufigkeit im Verhältnis zu transduzierten Zellen. Die Lebensfähigkeit bzw. Stabilität der Transformanten kann als das Ergebnis der Kultivierung von transformierten Zellen nach neuerlichem Aussäen bzw. Replattieren bestimmt werden.

Dabei hat sich gezeigt, dass γ-retrovirale Vektoren und lentivirale Vektoren zu einer Transformationshäufigkeit von ca. 1x10⁻⁵ bzw. 5x10⁻⁶ führen (Mol. Ther. 1919-1928 (2009)). In Parallelansätzen wurde für die beschriebenen Vektoren eine Transformationshäufigkeit von ca. 3,6x10⁻⁶ festgestellt, die also noch unter dem für lentivirale Vektoren festgestellten Wert lag. Neben der Transformationshäufigkeit wird in diesem Test die Lebensfähigkeit bzw. Fitness der transformierten Zellen analysiert. Der Wert für diese Fitness lag für 3 von 4 bislang isolierter Zellen, die durch den Vektor transformiert waren, etwa 10-fach niedriger als der Durchschnittswert für lentiviral transformierte Zellen. Diese Ergebnisse zeigen anhand der geringen Transformationshäufigkeit und der geringen Fitness transformierter Zellen durch den erfindungsgemäßen α-retroviralen Vektor, dass dieser eine bessere Sicherheit gegen die Onkogenaktivierung bietet, als die bisherigen γ-retroviralen oder lentiviralen Vektoren. Das Risiko der insertionellen Transformation kann durch die Wahl geeigneter interner Promotorsequenzen weiter reduziert werden.

### Beispiel 4: Bestimmung von Deletionen der 3'-U3-Region, die deren Promotor- und Enhanceraktivität eliminieren

Die Deletion bzw. die Ersetzung von Nukleotiden der U3-Region zur Reduktion bzw. Eliminierung der Promotor- und Enhanceraktivität wird vorzugsweise mit einem Aktivitätstest bestimmt, bei dem die Expression eines Reportergens gemessen wird, das funktional in 3' zu der mit Deletionen versehenen U3-Region angeordnet ist. Wenn die Expression eines Reportergens, das in 3' funktional zur deletierten bzw. ersetzten U3-Region angeordnet ist, auf dem Niveau der Hintergrundaktivität des promotorlos exprimierten Reportergens bestimmt wird, ist die Promotoraktivität erfindungsgemäß eliminiert. Vorzugsweise ist in 3' des Reportergens eine Polyadenylierungssequenz angeordnet. Als Hintergrundaktivität des exprimierten Reportergens wird z.B. die Aktivität definiert, die ohne jede U3-Region, bzw. ohne jeden Promotor in 5' des Reportergens gemessen wird.

Figur 14 zeigt schematisch die Nukleinsäurekonstrukte ausschnittsweise, mit denen der Aktivitätstest für die erfindungsgemäß deletierte bzw. ersetzte Promotor- und Enhanceraktivität der U3-Region durchgeführt wurde. Das in Figur 13 oben dargestellte Konstrukt zeigt als Reportergen das Luciferasegen aus Renilla (RLuc), das in 3' von einer Polyadenylierungssignalsequenz aus SV40 (SV40 pA) und in 5' von der auf Promotor- und Enhancersequenz zu testenden Nukleinsäuresequenz flankiert ist, vorliegend dem Wildtyp LTR (U3-R-U5) aus ASLV. Darunter ist ein ansonsten identisches Konstrukt mit Deletion (ΔLTR) gezeigt, dessen U3-Region teilweise deletiert ist (ΔU3). In diesem Konstrukt sind die Nukleotide 28-186 deletiert. Noch darunter ist ein ansonsten identisches Konstrukt gezeigt, in dem der LTR vollständig durch eine zufällige Sequenz (spacer, vom prokaryotischen β-Lactamasegen abgeleiteter Platzhalter) ersetzt ist. In 5' vor dem viralen Sequenzabschnitt ist ein synthetisches Polyadenylierungssignal (syn pA) angeordnet,

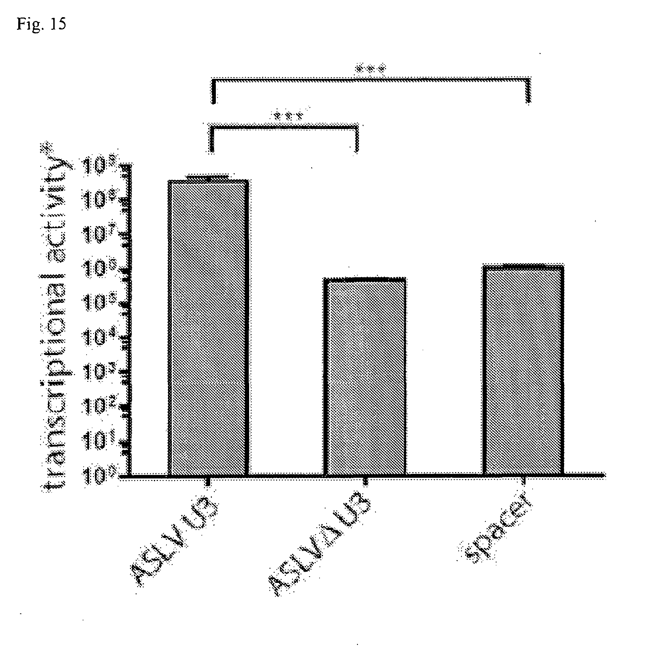

In Figur 15 sind die Luciferase-Aktivitäten von 293T-Zellen gezeigt, die mit den Konstrukten aus Figur 13 transfiziert und unter identischen Bedingungen kultiviert sind. Die Messung der Luciferaseaktivität erfolgte in Zellhomogenaten, die Darstellung zeigt relative Lumineszenzwerte, die auf Gesamtprotein und Transfektionseffizienz normalisiert wurden. Es wird deutlich, dass die erfindungsgemäße Deletion zu ΔU3 (ASLV ΔU3) eine drastische Verringerung (etwa um den Faktor 400) der Promotoraktivität gegenüber dem Wildtyp-LTR (ASLV U3) bewirkt. Die Daten sind nach statistischer Analyse untereinander sehr signifikant (**, P<0,01). Die Ersetzung des LTR durch die willkürliche Sequenz (spacer) führt zu einer ähnlichen Verringerung der Promotoraktivität. Daher kann erfindungsgemäß eine willkürliche Sequenz, die keine bekannte Promotor- und Enhancerfunktion aufweist, als Sequenz mit Hintergrundaktivität verwendet werden. Es wird angenommen, dass die noch gemessene Hintergrundaktivität der Luciferase durch andere Bestandteile des Plasmids verursacht wird, in welchem die Expressionskassette mit dem Reportergen enthalten ist. Vorzugsweise ist das Reportergen auf den Kodongebrauch der transfizierten Zelle angepasst.

## Patentansprüche

1. Retrovirale RNA mit einem 5'-Bereich, der eine R-Region und eine U5-Region aus Avian Sarkoma Leukosis Virus (ASLV) aufweist, und in 3' dazu angeordnet eine Primerbindungsstelle (PBS), ein Verpackungssignal (ψ), eine Expressionskassette, eine 3'-U3-Region und eine R-Region aufweist, **dadurch gekennzeichnet, dass** die 3'-U3-Region eine Nukleotidsequenz entsprechend Seq.-ID Nr. 1 aufweist, aus der zumindest die Nukleotide Nr. 28 bis einschließlich Nr. 186 deletiert sind, wodurch die Promotor- und Enhanceraktivität der 3'-U3-Region eliminiert ist und dass keine Nukleinsäuresequenzen enthalten sind, die zumindest anteilig virale Strukturproteine oder eine Spleißdonorstelle (SD) kodieren.

2. Retrovirale RNA nach Anspruch 1, **dadurch gekennzeichnet, dass** in 3' zur Deletion eine Polyadenylierungssequenz angeordnet ist.

3. Retrovirale RNA nach einem der voranstehenden Ansprüche, **dadurch gekennzeichnet, dass** die 3'-U3-Region eine Nukleotidsequenz entsprechend Seq.-ID Nr. 1 aufweist, und zusätzlich die Nukleotide Nr. 200 bis Nr. 206 zu einer Teilsequenz mutiert sind, die keine TATA-Box ist.

4. Retrovirale RNA nach Anspruch 3, **dadurch gekennzeichnet, dass** die Nukleotide Nr. 200 bis Nr. 206 zu einer Sequenz TGTCTAA mutiert sind

5. Retrovirale RNA nach einem der voranstehenden Ansprüche, **dadurch gekennzeichnet, dass** in der 3'-U3-Region zusätzlich Nukleotid Nr. 187 zu C und Nukleotid Nr. 189 zu T mutiert ist.

6. Retrovirale RNA nach einem der voranstehenden Ansprüche, **dadurch gekennzeichnet, dass** die 3'-U3-Region eine Nukleotidsequenz entsprechend Seq.-ID Nr. 1 aufweist, aus der zusätzlich die Nukleotide Nr. 187 bis Nr. 202 deletiert sind.

7. Retrovirale RNA nach einem der voranstehenden Ansprüche, **dadurch gekennzeichnet, dass** die 3'-U3-Region eine Sequenz entsprechend Seq.-ID Nr. 2, Seq.-ID Nr. 3 oder Seq.-ID Nr. 4 aufweist oder daraus besteht.

8. Retrovirale RNA nach einem der voranstehenden Ansprüche, **dadurch gekennzeichnet, dass** die 3'-U3-Region einschließlich der 3'-R-Region eine Sequenz entsprechend der Nukleotide Nr. 3282 bis Nr. 3372 von Seq.-ID Nr. 5, eine Sequenz entsprechend der Nukleotide Nr. 3282 bis Nr. 3356 von Seq.-ID Nr. 7 oder eine Sequenz entsprechend der Nukleotide Nr. 3282 bis Nr. 3372 von Seq.-ID Nr. 9 aufweist oder daraus besteht.

9. Retrovirale RNA nach einem der voranstehenden Ansprüche, **dadurch gekennzeichnet, dass** die Deletion der 3'-U3-Region durch eine Insertion mit einer Nukleotidsequenz ersetzt ist, die keine Promotor- oder Enhancersequenz aufweist.

10. Retrovirale RNA nach einem der voranstehenden Ansprüche, **dadurch gekennzeichnet, dass** die 3'-U3-Region eine inserierte Nukleinsäuresequenz aufweist, die miRNA, shRNA, einen Polyadenylierungsverstärker, Isolator und/oder eine Erkennungssequenz für eine DNA-Rekombinase kodiert.

11. Retrovirale RNA nach einem der voranstehenden Ansprüche, **dadurch gekennzeichnet, dass** die Expressionskassette von 5' nach 3' einen Promotor, eine ein Transgen kodierende Nukleinsäuresequenz und ein posttranskriptionelles regulatorisches Element (PRE) aufweist.

12. Retrovirale RNA nach einem der voranstehenden Ansprüche , **dadurch gekennzeichnet, dass** die Nukleinsäuresequenz, die in 5' angrenzend an den Promotor angeordnet ist, von 5' nach 3' ein R-Element, daran unmittelbar angrenzend ein U5-Element und ein Verpackungssignal kodiert.

13. Retrovirale RNA nach einem der Ansprüche 1 bis 11, **dadurch gekennzeichnet, dass** die Nukleinsäuresequenz, die in 5' zur Expressionskassette angeordnet ist, die Sequenz der Nukleotide Nrn. 922 bis 1292 von Seq.-ID Nr. 5 aufweist oder daraus besteht.

14. Retrovirale RNA nach einem der voranstehenden Ansprüche, **dadurch gekennzeichnet, dass** die 3'-U3-Region eine Integrase-Angriffsstelle in Nukleotiden 1-25 aufweist, sowie ein PolyA-Signal.

15. DNA mit einem Promotorelement, das funktional in 5' zu einer Nukleotidsequenz angeordnet ist, die eine retrovirale RNA nach einem der voranstehenden Ansprüche kodiert.

16. DNA nach Anspruch 15, **dadurch gekennzeichnet, dass** die die retrovirale RNA kodierende Nukleotidsequenz eine 5'-R-Region und eine 5'-US-Region mit einer Sequenz entsprechend Nukleotiden Nrn. 922 bis 1022 von Seq.-ID Nr. 5 aufweist, und in 3' dazu einen 3'-LTR mit einer Sequenz entsprechend Nukleotiden Nrn. 3282 bis 3452 von Seq.-ID Nr. 5 oder mit einer Sequenz entsprechend Nukleotiden Nrn. 3282 bis 3436 von Seq.-ID Nr. 7 oder mit einer Sequenz entsprechend Nukleotiden Nrn. 3282 bis 3452 von Seq.-ID Nr. 9.

17. Viraler Partikel mit einem Hüllprotein, **dadurch gekennzeichnet, dass** der Partikel eine retrovirale RNA nach einem der Ansprüche 1 bis 14 aufweist.

18. Pharmazeutische Zusammensetzung zur Verwendung in der Gentherapie, **gekennzeichnet durch** einen viralen Partikel nach Anspruch 17.

19. Split-Packaging-System zur Erzeugung retroviraler Partikel umfassend eine DNA, die eine retrovirale RNA kodiert, ein erstes Helferplasmid, das ein gag-pro-pol-Fusionsprotein kodiert und ein zweites Helferplasmid, das ein virales Hüllprotein kodiert, **dadurch gekennzeichnet, dass** die retrovirale RNA eine Nukleotidsequenz nach einem der Ansprüche 1 bis 14 aufweist.

20. System nach Anspruch 19, **dadurch gekennzeichnet, dass** die DNA, die die retrovirale RNA kodiert, das erste Helferplasmid und/oder das zweite Helferplasmid in eine eukaryotische Zelle transfiziert oder in das Genom einer eukaryotischen Zelle integriert sind.

21. System nach einem der Ansprüche 19 bis 20 **dadurch gekennzeichnet, dass** das erste Helferplasmid das gag-pro-pol-Fusionsprotein und/oder das zweite Helferplasmid das virale Hüllprotein mit Kodons kodiert, die an den Kodongebrauch der sie enthaltenden eukaryotischen Zelle angepasst sind.

22. System nach einem der Ansprüche 19 bis 21 **dadurch gekennzeichnet, dass** das erste Helferplasmid das gag-pro-pol-Fusionsprotein mit einer Nukleotidsequenz entsprechend Nukleotiden Nr. 1475 bis Nr. 6291 von Seq.-ID Nr. 14 kodiert.

23. Verfahren zur Produktion viraler Partikel durch Expression eines gag-pro-pol-Fusionsproteins und eines viralen Hüllproteins in einer Verpackungszelle, **dadurch gekennzeichnet, dass** die retrovirale RNA in der Verpackungszelle von einer DNA nach einem der Ansprüche 15 oder 16 transkribiert wird.

24. Viraler Partikel nach Anspruch 17 zur Verwendung in der Gentherapie.

25. Viraler Partikel nach Anspruch 24 zur Verwendung in der Gentherapie für die genetische Modifikation somatischer adulter Zellen, hämatopoetischer Stammzellen oder nicht-menschlicher embryonaler Stammzellen.

26. Transduktion von Zielzellen durch Kontaktieren der Zellen in vitro mit einer retroviralen RNA nach einem der Ansprüche 1 bis 14.

## Claims

1. Retroviral RNA having a 5'-region that has an R-region and a U5-region from avian sarcoma leukosis virus (ASLV), and arranged in 3' thereto a primer binding site (PBS), a packaging signal (Y), an expression cassette, a 3'-U3-region and an R-region, **characterized in that** the 3'-U3-region has a nucleotide sequence corresponding to SEQ ID NO: 1, from which at least nucleotides No. 28 to No. 186, inclusive, are deleted, whereby the promoter and enhancer activity of the 3'-U3-region is eliminated and that no nucleic acid sequences are contained that at least partially encode viral structural proteins or a splice donor site (SD).

2. Retroviral RNA according to claim 1, **characterized in that** in 3' to the deletion a polyadenylation sequence is arranged.

3. Retroviral RNA according to one of the preceding claims, **characterized in that** the 3'-U3-region has a nucleotide sequence corresponding to SEQ ID NO: 1, and in addition nucleotides No. 200 to No. 206 are mutated to a partial sequence which is no TATA box.

4. Retroviral RNA according to claim 3, **characterized in that** the nucleotides No. 200 to No. 206 are mutated to a sequence TGTCTAA.

5. Retroviral RNA according to one of the preceding claims, **characterized in that** in the 3'-U3-region in addition nucleotide No. 186 is mutated to C and nucleotide No. 189 is mutated to T.

6. Retroviral RNA according to one of the preceding claims, **characterized in that** the 3'-U3-region has a nucleotide sequence corresponding to SEQ ID NO: 1 from which in addition nucleotides No. 187 to No. 202 are deleted.

7. Retroviral RNA according to one of the preceding claims, **characterized in that** the 3'-U3-region comprises or consists of a sequence corresponding to SEQ ID NO: 2, SEQ ID NO: 3 or Seq ID No. 4.

8. Retroviral RNA according to one of the preceding claims, **characterized in that** the 3'-U3-region including the 3'-R-region comprises or consists of a sequence corresponding to nucleotide No. 3282 to No. 3372 of SEQ ID NO: 5, a sequence corresponding to nucleotides No. 3282 to No. 3356 of SEQ ID NO: 7, or a sequence corresponding to nucleotides No. 3282 to No. 3372 of SEQ ID NO: 9.

9. Retroviral RNA according to one of the preceding claims, **characterized in that** the deletion of the 3'-U3-region is replaced by an insertion with a nucleotide sequence which has no promoter sequence or enhancer sequence.

10. Retroviral RNA according to one of the preceding claims, characterized that the 3'-U3-region has an inserted nucleic acid sequence which encodes miRNA, shRNA, a polyadenylation enhancer, an insulator and/or a recognition sequence for a DNA recombinase.

11. Retroviral RNA according to one the preceding claims, **characterized in that** the expression cassette form 5' to 3' has a promoter, a nucleic acid sequence encoding a transgene and a post-transcriptional regulatory element (PRE).

12. Retroviral RNA according to one of the preceding claims, **characterized in that** the nucleic acid sequence that is arranged in 5' adjacent to the promoter from 5' to 3' encodes an R-element, immediately adjacent thereto a U5-element and a packaging signal.

13. Retroviral RNA according to one of claims 1 to 11, **characterized in that** the nucleic acid sequence which is arranged in 5' to the expression cassette comprises or consists of the sequence of nucleotides Nos. 922 to 1292 of SEQ ID NO: 5.

14. Retroviral RNA according to one of the preceding claims, **characterized in that** the 3'-U3-region has an integrase attachment site in nucleotides 1 - 25 and a polyA-signal.

15. DNA having a promoter element which is arranged functionally in 5' to a nucleic acid sequence that encodes a retroviral RNA according to one of the preceding claims.

16. DNA according to claim 15, characterized that the nucleotide sequence encoding the retroviral RNA comprises a 5'-R-region and a 5'-U5-region having a sequence corresponding to nucleotides Nos. 922 to 1022 of SEQ ID NO: 5, and in 3' thereto a 3'-LTR having a sequence corresponding to nucleotides Nos. 3282 to 3452 of SEQ ID NO: 5 or having a sequence corresponding to nucleotides Nos. 3282 to 3436 of SEQ ID NO: 7 or having a sequence corresponding to nucleotides Nos. 3282 to 3452 of SEQ ID NO: 9.

17. Viral particle having an envelope protein, characterized that the particle has a viral RNA according to one of claims 1 to 14.

18. Pharmaceutical composition for use in gene therapy, **characterized by** a viral particle according to claim 17.

19. Split-packaging system for the generation of retroviral particles comprising a DNA encoding a retroviral RNA, a first helper plasmid encoding a gag-pro-pol-fusion protein and a second helper plasmid encoding a viral envelope protein, characterized that the retroviral RNA has a nucleotide sequence according to one of claims 1 to 14.

20. System according to claim 19, **characterized in that** the DNA encoding the retroviral RNA, the first helper plasmid and/or the second helper plasmid are transfected into a eucaryotic cells or are integrated into the genome of a eucaryotic cell.

21. System according to one of claims 19 to 20, **characterized in that** the first helper plasmid encodes the gag-pro-pol-fusion protein and/or the second helper plasmid encodes the viral envelope protein by codons which are adapted to the codon usage of the eucaryotic cell containing them.

22. System according to one of claims 19 to 21, **characterized in that** the first helper plasmid encodes the gag-pro-pol-fusion protein by a nucleotide sequence corresponding to nucleotides No. 1475 to number 6291 of SEQ ID NO: 14.

23. Process for the production of viral particles by expression gag-pro-pol-fusion protein and of a viral envelope protein in a packaging cell, **characterized in that** the retroviral RNA is transcribed within the packaging cell from a DNA according to one of claims 15 or 16.

24. Viral particle according to claim 17 for use in gene therapy.

25. Viral particle according to claim 24 for use in gene therapy for the genetic modification of somatic adult cells, haematopoietic stem cells or non human embryonic stem cells.

26. Transduction of target cells by contacting of cells in vitro with a retroviral RNA according to one of claims 1 to 14.

## Revendications

1. ARN rétroviral comportant un domaine 5' lequel présente une région R et une région U5 provenant de virus de sarcomes et de leucoses aviaires (ASLV) et, de plus, localisé en 3', un site d'initiation de la transcription inverse (PBS), un signal d'emballage (ψ), une cassette d'expression, une région 3'-U3 et une région R, **caractérisé en ce que** la région 3'-U3 présente une séquence de nucléotides correspondant à SEQ ID No 1, à partir de laquelle au moins le nucléotide No 28 jusqu'au nucléotide No 186 inclus sont délétés, moyennant quoi l'activité de promoteur et d'activateur de la région 3'-U3 est éliminée, et **en ce qu'**il n'y a aucune séquences d'acides nucléiques qui codent, au moins proportionnellement, des protéines de structure virales ou un site donneur d'épissage (SD).

2. ARN rétroviral selon la revendication 1, **caractérisé en ce qu'**une séquence de polyadénylation est localisée en 3' en vue de la délétion.

3. ARN rétroviral selon l'une quelconque des revendications précédentes, **caractérisé en ce que** la région 3'-U3 présente une séquence de nucléotides correspondant à SEQ ID No 1, et, de plus, les nucléotides No 200 à 206 ont muté en une séquence partielle qui n'est pas une boîte TATA.

4. ARN rétroviral selon la revendication 3, **caractérisé en ce que** les nucléotides No 200 à No 206 ont muté en une séquence TGTCTAA.

5. ARN rétroviral selon l'une quelconque des revendications précédentes, **caractérisé en ce que**, de plus, dans la région 3'-U3, le nucléotide No 187 a muté en C et le nucléotide No 189 a muté en T.

6. ARN rétroviral selon l'une quelconque des revendications précédentes, **caractérisé en ce que** la région 3'-U3 présente une séquence de nucléotides correspondant à SEQ ID No 1, à partir de laquelle, de surcroît, les nucléotides No 187 à No 202 sont délétés.

7. ARN rétroviral selon l'une quelconque des revendications précédentes, **caractérisé en ce que** la région 3'-U3 présente une séquence correspondant à SEQ ID No 2, SEQ ID No 3 ou SEQ ID No 4 ou est constituée de cette séquence.

8. ARN rétroviral selon l'une quelconque des revendications précédentes, **caractérisé en ce que** la région 3'-U3, y compris la région 3'-R présente une séquence correspondant au nucléotide No 3282 à No 3372 de SEQ ID No 5, une séquence correspondant au nucléotide No 3282 à 3356 de SEQ ID No 7 ou une séquence correspondant au nucléotide No 3282 à No 3372 de SEQ ID No 9 ou est constituée d'une telle séquence.

9. ARN rétroviral selon l'une quelconque des revendications précédentes, **caractérisé en ce que** la délétion de la région 3'-U3 est remplacée par une insertion comportant une séquence de nucléotides ne présentant aucune séquence de promoteur ou d'activateur.

10. ARN rétroviral selon l'une quelconque des revendications précédentes, **caractérisé en ce que** la région 3'-U3 présente une séquence d'acides nucléiques insérée qui code le miARN, le shARN, un renforçateur de la polyadénylation, un isolateur et/ou une séquence de reconnaissance pour une ADN-recombinase.

11. ARN rétroviral selon l'une quelconque des revendications précédentes, **caractérisé en ce que** la cassette d'expression de 5' vers 3' présente un promoteur, une séquence d'acides nucléiques qui code un transgène et un élément de régulation post-transcriptionnelle (ERP).

12. ARN rétroviral selon l'une quelconque des revendications précédentes, **caractérisé en ce que** la séquence d'acides nucléiques, localisée en 5' adjacente au promoteur, code de 5' vers 3' un élément R, de façon directement adjacente à celui-ci un élément U5 et un signal d'emballage.

13. ARN rétroviral selon l'une quelconque des revendications 1 à 11, **caractérisé en ce que** la séquence d'acides nucléiques, localisée en 5' vers la cassette d'expression, présente la séquence de nucléotides No 922 à 1292 de SEQ ID No 5 ou est constituée de celle-ci.

14. ARN rétroviral selon l'une quelconque des revendications précédentes, **caractérisé en ce que** la région 3'-U3 présente un site d'attaque de l'intégrase dans les nucléotides 1 à 25, ainsi qu'un signal PolyA.

15. ADN comportant un élément promoteur localisé, au niveau fonctionnel, en 5' vers une séquence de nucléotides qui code un ARN rétroviral selon l'une quelconque des revendications précédentes.

16. ADN selon la revendication 15, **caractérisé en ce que** la séquence de nucléotides qui code l'ARN rétroviral, présente une région 5'-R et une région 5'-U5 comportant une séquence correspondant aux nucléotides No 922 à 1022 de SEQ ID No 5 et, de plus, en 3', une LTR 3' comprenant une séquence correspondant aux nucléotides No 3282 à 3452 de SEQ ID No 5 ou une séquence correspondant aux nucléotides No 3282 à 3436 de SEQ ID No 7 ou une séquence correspondant aux nucléotides No 3282 à 3452 de SEQ ID No 9.

17. Particule virale comportant une protéine d'enveloppe, **caractérisée en ce que** la particule présente un ARN rétroviral selon l'une quelconque des revendications 1 à 14.

18. Composé pharmaceutique destiné à être utilisé en génothérapie, **caractérisé par** une particule virale selon la revendication 17.

19. Système split-emballage destiné à la production de particules rétrovirales comprenant un ADN qui code un ARN rétroviral, un premier plasmide assistant qui code une protéine de fusion gag-pro-pol et un deuxième plasmide assistant qui code une protéine d'enveloppe virale, **caractérisé en ce que** l'ARN rétroviral présente une séquence de nucléotides selon l'une quelconque des revendications 1 à 14.

20. Système selon la revendication 19, **caractérisé en ce que** l'ADN qui code l'ARN rétroviral, le premier plasmide assistant et/ou le deuxième plasmide assistant sont transférés dans une cellule eucaryote ou intégrés dans le génome d'une cellule eucaryote.

21. Système selon l'une quelconque des revendications 19 à 20, **caractérisé en ce que** le premier plasmide assistant code la protéine de fusion gag-pro-pol et/ou le deuxième plasmide assistant la protéine d'enveloppe virale avec des codons adaptés à l'utilisation des codons de la cellule eucaryote qu'ils contiennent.

22. Système selon l'une quelconque des revendications 19 à 21, **caractérisé en ce que** le premier plasmide assistant code la protéine de fusion gag-pro-pol comportant une séquence de nucléotides correspondant aux nucléotides No 1475 à No 6291 de SEQ ID No 14.

23. Procédé pour la production de particules virales par expression d'une protéine de fusion gag-pro-pol et d'une protéine d'enveloppe virale dans une cellule d'empaquetage, **caractérisé en ce que** l'ARN rétroviral est transcris, dans la cellule d'empaquetage, par un ADN selon l'une quelconque des revendications 15 ou 16.

24. Particules virales selon la revendication 17 destinées à être utilisées en génothérapie.

25. Particules virales selon la revendication 24 destinées à être utilisées en génothérapie pour la modification génétique de cellules adultes somatiques, de cellules souches hématopoïétiques et de cellules souches embryonnaires non humaines.

26. Transduction de cellules-cibles par mise en contact des cellules in vitro avec un ARN rétroviral selon l'une quelconque des revendications 1 à 14.


### SEQUENCE LISTING

<110> Medizinische Hochschule Hannover
   Axel, Schambach, Dr.
   Christopher, Baum, Prof. Dr.
   Julia, Sürth
<120> ASLV Vektorsystem
<130> M1013PCT
<160> 16
<170> PatentIn version 3.5
<210> 1
   <211> 229
   <212> DNA
   <213> Avian sarcoma virus
<220>
   <221> misc_feature
   <222> (1)..(229)
   <223> Seq.-ID Nr. 1: 3'-U3-Region Wildtyp ASLV
<400> 1
   aatgtagtct tatgcaatac tcttgtagtc ttgcaacatg gtaacgatga gttagcaaca 60
   tgccttacaa ggagagaaaa agcaccgtgc atgccgattg gtggaagtaa ggtggtacga 120
   tcgtgcctta ttaggaaggc aacagacggg tctgacatgg attggacgaa ccactgaatt 180
   ccgcattgca gagatattgt atttaagtgc ctagctcgat acaataaac 229
<210> 2
   <211> 70
   <212> DNA
   <213> Artificial Sequence
<220>
   <223> /note="Seq.-ID Nr. 2: U3-Region mit Deletion"
<400> 2
   aatgtagtct tatgcaatac tcttgtacgt agagatattg tatttaagtg cctagctcga 60
   tacaataaac 70
<210> 3
   <211> 70
   <212> DNA
   <213> Artificial Sequence
<220>
   <223> /note="Seq.-ID Nr. 3: U3-Region mit Deletion"
<400> 3
   aatgtagtct tatgcaatac tcttgtacgt agagatattg tgtctaagtg cctagctcga 60
   tacaataaac 70
<210> 4
   <211> 54
   <212> DNA
   <213> Artificial Sequence
<220>
   <223> /note="Seq.-ID Nr. 4: U3-Region mit Deletion"
<400> 4
   aatgtagtct tatgcaatac tcttgtacgt agtgcctagc tcgatacaat aaac 54
<210> 5
   <211> 5704
   <212> DNA
   <213> Artificial Sequence
<220>
   <223> /note="Seq.-ID Nr. 5: viraler vektor mit deletierter U3-Region"
<220>
   <221> enhancer
   <222> (452)..(684)
   <223> SV40 enhancer
<220>
   <221> promoter
   <222> (693)..(921)
   <223> RSV Promotor
<220>
   <221> misc_feature
   <222> (922)..(942)
   <223> 5'- LTR: R-Region
<220>
   <221> misc_feature
   <222> (943)..(1022)
   <223> 5'- LTR: U5-Region
<220>
   <221> PBS
   <222> (1023)..(1041)
   <223> Primer Bindestelle
<220>
   <221> promoter
   <222> (1293)..(1684)
   <223> Interner Promotor der Expressionskassette: SFFV
<220>
   <221> CDS
   <222> (1764)..(2483)
   <223> EGFP: kodierende Sequenz für eGFP
<220>
   <221> misc_feature
   <222> (2522)..(3124)
   <223> PRE der Expressionskassette
<220>
   <221> misc_feature
   <222> (3282)..(3351)
   <223> 3'-U3: U3-Region mit Deletion
<220>
   <221> misc_feature
   <222> (3352)..(3372)
   <223> 3'- R-Region
<220>
   <221> misc_feature
   <222> (3373)..(3452)
   <223> 3'- U5-Region
<400> 5
   ctgcctcgcg cgtttcggtg atgacggtga aaacctctga cacatgcagc tcccggagac      60
   ggtcacagct tgtctgtaag cggatgccgg gagcagacaa gcccgtcagg gcgcgtcagc     120
   gggtgttggc gggtgtcggg gcgcagccat gacccagtca cgtagcgata gttactatgc     180
   ggcatcagag cagattgtac tgagagtgca ccatatgcgg tgtgaaatac cgcacagatg     240
   cgtaaggaga aaataccgca tcaggcgcca ttcgccattc aggctgcgca actgttggga     300
   agggcgatcg gtgcgggcct cttcgctatt acgccagctg gcgaaagggg gatgtgctgc     360
   aaggcgatta agttgggtaa cgccagggtt ttcccagtca cgacgttgta aaacgacggc     420
   cagtgaatta gtactctagc ttaagacgcg tggcctgaaa taacctctga aagaggaact     480
   tggttaggta ccttctgagg cggaaagaac cagctgtgga atgtgtgtca gttagggtgt     540
   ggaaagtccc caggctcccc agcaggcaga agtatgcaaa gcatgcatct caattagtca     600
   gcaaccaggt gtggaaagtc cccaggctcc ccagcaggca gaagtatgca aagcatgcat     660
   ctcaattagt cagcaaccat agtcccttaa gaatgtagtc ttatgcaata ctcttgtagt     720
   cttgcaacat ggtaacgatg agttagcaac atgccttaca aggagagaaa aagcaccgtg     780
   catgccgatt ggtggaagta aggtggtacg atcgtgcctt attaggaagg caacagacgg     840
   gtctgacatg gattggacga accactgaat tgccgcattg cagagatatt gtatttaagt     900
   gcctagctcg atacaataaa cgccatttga ccattcacca cattggtgtg cacctgggtt     960
   gatggccgga ccgttgattc cctgacgact acgagcacct gcatgaagca gaaggcttca    1020
   tttggtgacc ccgacgtgat agttagggaa tagtggtcgg ccacagacgg cgtggcgatc    1080
   ctgtctccat ccgtctcgtc tatcgggagg cgagttcgat gaccctggtg gagggggctg    1140
   cggcttaggg aggcagaagc tgagtaccgt cggagggagc tccagggccc ggagcgactg    1200
   acccctgccg agaactcaga gggtcgtcgg aagacggaga gtgagcccga cgaccacccc    1260
   aggcacgtct ttggtcggcc tgcggatcaa gcagcggccg ctagctgcag taacgccatt    1320
   ttgcaaggca tggaaaaata ccaaaccaag aatagagaag ttcagatcaa gggcgggtac    1380
   atgaaaatag ctaacgttgg gccaaacagg atatctgcgg tgagcagttt cggccccggc    1440
   ccggggccaa gaacagatgg tcaccgcagt ttcggccccg gcccgaggcc aagaacagat    1500
   ggtccccaga tatggcccaa ccctcagcag tttcttaaga cccatcagat gtttccaggc    1560
   tcccccaagg acctgaaatg accctgcgcc ttatttgaat taaccaatca gcctgcttct    1620
   cgcttctgtt cgcgcgcttc tgcttcccga gctctataaa agagctcaca acccctcact    1680
   cggcgcgcca gtcctccgac agactgagtc ggccggtcga atcaagctta tcgataccgt    1740
   cgacggatcc accggtcgcc acc atg gtg agc aag ggc gag gag ctg ttc acc    1793
                             Met Val Ser Lys Gly Glu Glu Leu Phe Thr
                             1               5                   10
   ggg gtg gtg ccc atc ctg gtc gag ctg gac ggc gac gta aac ggc cac       1841
   Gly Val Val Pro Ile Leu Val Glu Leu Asp Gly Asp Val Asn Gly His
                   15                  20                  25
   aag ttc agc gtg tcc ggc gag ggc gag ggc gat gcc acc tac ggc aag        1889
   Lys Phe Ser Val Ser Gly Glu Gly Glu Gly Asp Ala Thr Tyr Gly Lys
               30                  35                  40
   ctg acc ctg aag ttc atc tgc acc acc ggc aag ctg ccc gtg ccc tgg        1937
   Leu Thr Leu Lys Phe Ile Cys Thr Thr Gly Lys Leu Pro Val Pro Trp
           45                  50                  55
   ccc acc ctc gtg acc acc ctg acc tac ggc gtg cag tgc ttc agc cgc        1985
   Pro Thr Leu Val Thr Thr Leu Thr Tyr Gly Val Gln Cys Phe Ser Arg
       60                  65                  70
   tac ccc gac cac atg aag cag cac gac ttc ttc aag tcc gcc atg ccc        2033
   Tyr Pro Asp His Met Lys Gln His Asp Phe Phe Lys Ser Ala Met Pro
   75                  80                  85                  90
   gaa ggc tac gtc cag gag cgc acc atc ttc ttc aag gac gac ggc aac        2081
   Glu Gly Tyr Val Gln Glu Arg Thr Ile Phe Phe Lys Asp Asp Gly Asn
                   95                  100                 105
   tac aag acc cgc gcc gag gtg aag ttc gag ggc gac acc ctg gtg aac        2129
   Tyr Lys Thr Arg Ala Glu Val Lys Phe Glu Gly Asp Thr Leu Val Asn
               110                 115                 120
   cgc atc gag ctg aag ggc atc gac ttc aag gag gac ggc aac atc ctg        2177
   Arg Ile Glu Leu Lys Gly Ile Asp Phe Lys Glu Asp Gly Asn Ile Leu
           125                 130                 135
   ggg cac aag ctg gag tac aac tac aac agc cac aac gtc tat atc atg        2225
   Gly His Lys Leu Glu Tyr Asn Tyr Asn Ser His Asn Val Tyr Ile Met
       140                 145                 150
   gcc gac aag cag aag aac ggc atc aag gtg aac ttc aag atc cgc cac        2273
   Ala Asp Lys Gln Lys Asn Gly Ile Lys Val Asn Phe Lys Ile Arg His
   155                 160                 165                 170
   aac atc gag gac ggc agc gtg cag ctc gcc gac cac tac cag cag aac        2321
   Asn Ile Glu Asp Gly Ser Val Gln Leu Ala Asp His Tyr Gln Gln Asn
                   175                 180                 185
   acc ccc atc ggc gac ggc ccc gtg ctg ctg ccc gac aac cac tac ctg        2369
   Thr Pro Ile Gly Asp Gly Pro Val Leu Leu Pro Asp Asn His Tyr Leu
               190                 195                 200
   agc acc cag tcc gcc ctg agc aaa gac ccc aac gag aag cgc gat cac        2417
   Ser Thr Gln Ser Ala Leu Ser Lys Asp Pro Asn Glu Lys Arg Asp His
           205                 210                 215
   atg gtc ctg ctg gag ttc gtg acc gcc gcc ggg atc act ctc ggc atg        2465
   Met Val Leu Leu Glu Phe Val Thr Ala Ala Gly Ile Thr Leu Gly Met
       220                 225                 230
   gac gag ctg tac aag taa agcggccgcg tcgacggatc ccccgggctg               2513
   Asp Glu Leu Tyr Lys
   235
   caggaattcg agcatcttac cgccatttat acccatattt gttctgtttt tcttgatttg      2573
   ggtatacatt taaatgttaa taaaacaaaa tggtggggca atcatttaca tttttaggga      2633
   tatgtaatta ctagttcagg tgtattgcca caagacaaac atgttaagaa actttcccgt      2693
   tatttacgct ctgttcctgt taatcaacct ctggattaca aaatttgtga aagattgact      2753
   gatattctta actatgttgc tccttttacg ctgtgtggat atgctgcttt aatgcctctg      2813
   tatcatgcta ttgcttcccg tacggctttc gttttctcct ccttgtataa atcctggttg      2873
   ctgtctcttt atgaggagtt gtggcccgtt gtccgtcaac gtggcgtggt gtgctctgtg      2933
   tttgctgacg caacccccac tggctggggc attgccacca cctgtcaact cctttctggg 2993
   actttcgctt tccccctccc gatcgccacg gcagaactca tcgccgcctg ccttgcccgc 3053
   tgctggacag gggctaggtt gctgggcact gataattccg tggtgttgtc ggggaagctg 3113
   acgtcctttc gaattcgaaa gcttttaaat atcgatgcga tgtacgggcc agatatacgc 3173
   gtatctgagg ggactagggt gtgtttaggc gaaaagcggg gcttcggttg tacgcggtta 3233
   ggagtcccct taggatatag tagtttcgct tttgcatagg gagggggaaa tgtagtctta 3293
   tgcaatactc ttgtacgtag agatattgta tttaagtgcc tagctcgata caataaacgc 3353
   catttgacca ttcaccacat tggtgtgcac ctgggttgat ggccggaccg ttgattccct 3413
   gacgactacg agcacctgca tgaagcagaa ggcttcattc tcgagagctt tggcgtaatc 3473
   atggtcatag ctgtttcctg tgtgaaattg ttatccgctc acaattccac acaacatacg 3533
   agccggaagc ataaagtgta aagcctgggg tgcctaatga gtgagctaac tcacattaat 3593
   tgcgttgcgc tcactgcccg ctttccagtc gggaaacctg tcgtgccagc tgcattaatg 3653
   aatcggccaa cgcgcgggga gaggcggttt gcgtattggg cgctcttccg cttcctcgct 3713
   cactgactcg ctgcgctcgg tcgttcggct gcggcgagcg gtatcagctc actcaaaggc 3773
   ggtaatacgg ttatccacag aatcagggga taacgcagga aagaacatgt gagcaaaagg 3833
   ccagcaaaag gccaggaacc gtaaaaaggc cgcgttgctg gcgtttttcc ataggctccg 3893
   cccccctgac gagcatcaca aaaatcgacg ctcaagtcag aggtggcgaa acccgacagg 3953
   actataaaga taccaggcgt ttccccctgg aagctccctc gtgcgctctc ctgttccgac 4013
   cctgccgctt accggatacc tgtccgcctt tctcccttcg ggaagcgtgg cgctttctca 4073
   atgctcacgc tgtaggtatc tcagttcggt gtaggtcgtt cgctccaagc tgggctgtgt 4133
   gcacgaaccc cccgttcagc ccgaccgctg cgccttatcc ggtaactatc gtcttgagtc 4193
   caacccggta agacacgact tatcgccact ggcagcagcc actggtaaca ggattagcag 4253
   agcgaggtat gtaggcggtg ctacagagtt cttgaagtgg tggcctaact acggctacac 4313
   tagaaggaca gtatttggta tctgcgctct gctgaagcca gttaccttcg gaaaaagagt 4373
   tggtagctct tgatccggca aacaaaccac cgctggtagc ggtggttttt ttgtttgcaa 4433
   gcagcagatt acgcgcagaa aaaaaggatc tcaagaagat cctttgatct tttctacggg 4493
   gtctgacgct cagtggaacg aaaactcacg ttaagggatt ttggtcatga gattatcaaa 4553
   aaggatcttc acctagatcc ttttaaatta aaaatgaagt tttaaatcaa tctaaagtat 4613
   atatgagtaa acttggtctg acagttacca atgcttaatc agtgaggcac ctatctcagc 4673
   gatctgtcta tttcgttcat ccatagttgc ctgactcccc gtcgtgtaga taactacgat 4733
   acgggagggc ttaccatctg gccccagtgc tgcaatgata ccgcgagacc cacgctcacc 4793
   ggctccagat ttatcagcaa taaaccagcc agccggaagg gccgagcgca gaagtggtcc 4853
   tgcaacttta tccgcctcca tccagtctat taattgttgc cgggaagcta gagtaagtag 4913
   ttcgccagtt aatagtttgc gcaacgttgt tgccattgct gctggcatcg tggtgtcacg 4973
   ctcgtcgttt ggtatggctt cattcagctc cggttcccaa cgatcaaggc gagttacatg 5033
   atcccccatg ttgtgcaaaa aagcggttag ctccttcggt cctccgatcg ttgtcagaag 5093
   taagttggcc gcagtgttat cactcatggt tatggcagca ctgcataatt ctcttactgt 5153
   catgccatcc gtaagatgct tttctgtgac tggtgagtac tcaaccaagt cattctgaga 5213
   atagtgtatg cggcgaccga gttgctcttg cccggcgtca atacgggata ataccgcgcc 5273
   acatagcaga actttaaaag tgctcatcat tggaaaacgt tcttcggggc gaaaactctc 5333
   aaggatctta ccgctgttga gatccagttc gatgtaaccc actcgtgcac ccaactgatc 5393
   ttcagcatct tttactttca ccagcgtttc tgggtgagca aaaacaggaa ggcaaaatgc 5453
   cgcaaaaaag ggaataaggg cgacacggaa atgttgaata ctcatactct tcctttttca 5513
   atattattga agcatttatc agggttattg tctcatgagc ggatacatat ttgaatgtat 5573
   ttagaaaaat aaacaaatag gggttccgcg cacatttccc cgaaaagtgc cacctgacgt 5633
   ctaagaaacc attattatca tgacattaac ctataaaaat aggcgtatca cgaggccctt 5693
   tcgtcttcaa g 5704
<210> 6
   <211> 239
   <212> PRT
   <213> Artificial Sequence
<220>
   <223> Synthetic Construct
<400> 6
   Met Val Ser Lys Gly Glu Glu Leu Phe Thr Gly Val Val Pro Ile Leu
   1               5                   10                  15
   Val Glu Leu Asp Gly Asp Val Asn Gly His Lys Phe Ser Val Ser Gly
               20                  25                  30
   Glu Gly Glu Gly Asp Ala Thr Tyr Gly Lys Leu Thr Leu Lys Phe Ile
           35                  40                  45
   Cys Thr Thr Gly Lys Leu Pro Val Pro Trp Pro Thr Leu Val Thr Thr
       50                  55                  60
   Leu Thr Tyr Gly Val Gln Cys Phe Ser Arg Tyr Pro Asp His Met Lys
   65                  70                  75                  80
   Gln His Asp Phe Phe Lys Ser Ala Met Pro Glu Gly Tyr Val Gln Glu
                   85                  90                  95
   Arg Thr Ile Phe Phe Lys Asp Asp Gly Asn Tyr Lys Thr Arg Ala Glu
               100                 105                 110
   Val Lys Phe Glu Gly Asp Thr Leu Val Asn Arg Ile Glu Leu Lys Gly
           115                 120                 125
   Ile Asp Phe Lys Glu Asp Gly Asn Ile Leu Gly His Lys Leu Glu Tyr
       130                 135                 140
   Asn Tyr Asn Ser His Asn Val Tyr Ile Met Ala Asp Lys Gln Lys Asn
   145                 150                 155                 160
   Gly Ile Lys Val Asn Phe Lys Ile Arg His Asn Ile Glu Asp Gly Ser
                   165                 170                 175
   Val Gln Leu Ala Asp His Tyr Gln Gln Asn Thr Pro Ile Gly Asp Gly
               180                 185                 190
   Pro Val Leu Leu Pro Asp Asn His Tyr Leu Ser Thr Gln Ser Ala Leu
           195                 200                 205
   Ser Lys Asp Pro Asn Glu Lys Arg Asp His Met Val Leu Leu Glu Phe
       210                 215                 220
   Val Thr Ala Ala Gly Ile Thr Leu Gly Met Asp Glu Leu Tyr Lys
   225                 230                 235
<210> 7
   <211> 5688
   <212> DNA
   <213> Artificial Sequence
<220>
   <223> /note="Seq.-ID Nr. 7: viraler vektor mit deletierter U3-Region"
<220>
   <221> enhancer
   <222> (452)..(684)
   <223> SV40 Enhancer
<220>
   <221> promoter
   <222> (693)..(921)
   <223> RSV Promotor
<220>
   <221> misc_feature
   <222> (922)..(942)
   <223> 5'- R-Region
<220>
   <221> misc_feature
   <222> (943)..(1022)
   <223> 5'- U5-Region
<220>
   <221> PBS
   <222> (1023)..(1041)
   <223> Primer Bindestelle
<220>
   <221> promoter
   <222> (1293)..(1684)
   <223> Interner Promoter der Expressionskassette: SFFV
<220>
   <221> CDS
   <222> (1764)..(2483)
   <223> EGFP: kodierende Sequenz für eGFP
<220>
   <221> misc_feature
   <222> (2522)..(3124)
   <223> PRE der Expressionskassette
<220>
   <221> misc_feature
   <222> (3282)..(3335)
   <223> 3'- U3-Region mit Deletion
<220>
   <221> misc_feature
   <222> (3336)..(3356)
   <223> 3'-R-Region
<220>
   <221> misc_feature
   <222> (3357)..(3436)
   <223> 3'- U5-Region
<400> 7
   ctgcctcgcg cgtttcggtg atgacggtga aaacctctga cacatgcagc tcccggagac 60
   ggtcacagct tgtctgtaag cggatgccgg gagcagacaa gcccgtcagg gcgcgtcagc 120
   gggtgttggc gggtgtcggg gcgcagccat gacccagtca cgtagcgata gttactatgc 180
   ggcatcagag cagattgtac tgagagtgca ccatatgcgg tgtgaaatac cgcacagatg 240
   cgtaaggaga aaataccgca tcaggcgcca ttcgccattc aggctgcgca actgttggga 300
   agggcgatcg gtgcgggcct cttcgctatt acgccagctg gcgaaagggg gatgtgctgc 360
   aaggcgatta agttgggtaa cgccagggtt ttcccagtca cgacgttgta aaacgacggc 420
   cagtgaatta gtactctagc ttaagacgcg tggcctgaaa taacctctga aagaggaact 480
   tggttaggta ccttctgagg cggaaagaac cagctgtgga atgtgtgtca gttagggtgt 540
   ggaaagtccc caggctcccc agcaggcaga agtatgcaaa gcatgcatct caattagtca 600
   gcaaccaggt gtggaaagtc cccaggctcc ccagcaggca gaagtatgca aagcatgcat 660
   ctcaattagt cagcaaccat agtcccttaa gaatgtagtc ttatgcaata ctcttgtagt 720
   cttgcaacat ggtaacgatg agttagcaac atgccttaca aggagagaaa aagcaccgtg 780
   catgccgatt ggtggaagta aggtggtacg atcgtgcctt attaggaagg caacagacgg 840
   gtctgacatg gattggacga accactgaat tgccgcattg cagagatatt gtatttaagt 900
   gcctagctcg atacaataaa cgccatttga ccattcacca cattggtgtg cacctgggtt 960
   gatggccgga ccgttgattc cctgacgact acgagcacct gcatgaagca gaaggcttca 1020
   tttggtgacc ccgacgtgat agttagggaa tagtggtcgg ccacagacgg cgtggcgatc 1080
   ctgtctccat ccgtctcgtc tatcgggagg cgagttcgat gaccctggtg gagggggctg 1140
   cggcttaggg aggcagaagc tgagtaccgt cggagggagc tccagggccc ggagcgactg 1200
   acccctgccg agaactcaga gggtcgtcgg aagacggaga gtgagcccga cgaccacccc 1260
   aggcacgtct ttggtcggcc tgcggatcaa gcagcggccg ctagctgcag taacgccatt 1320
   ttgcaaggca tggaaaaata ccaaaccaag aatagagaag ttcagatcaa gggcgggtac 1380
   atgaaaatag ctaacgttgg gccaaacagg atatctgcgg tgagcagttt cggccccggc    1440
   ccggggccaa gaacagatgg tcaccgcagt ttcggccccg gcccgaggcc aagaacagat    1500
   ggtccccaga tatggcccaa ccctcagcag tttcttaaga cccatcagat gtttccaggc    1560
   tcccccaagg acctgaaatg accctgcgcc ttatttgaat taaccaatca gcctgcttct    1620
   cgcttctgtt cgcgcgcttc tgcttcccga gctctataaa agagctcaca acccctcact    1680
   cggcgcgcca gtcctccgac agactgagtc ggccggtcga atcaagctta tcgataccgt    1740
   cgacggatcc accggtcgcc acc atg gtg agc aag ggc gag gag ctg ttc acc    1793
                             Met Val Ser Lys Gly Glu Glu Leu Phe Thr
                             1               5                   10
   ggg gtg gtg ccc atc ctg gtc gag ctg gac ggc gac gta aac ggc cac      1841
   Gly Val Val Pro Ile Leu Val Glu Leu Asp Gly Asp Val Asn Gly His
                   15                  20                  25
   aag ttc agc gtg tcc ggc gag ggc gag ggc gat gcc acc tac ggc aag      1889
   Lys Phe Ser Val Ser Gly Glu Gly Glu Gly Asp Ala Thr Tyr Gly Lys
               30                  35                  40
   ctg acc ctg aag ttc atc tgc acc acc ggc aag ctg ccc gtg ccc tgg      1937
   Leu Thr Leu Lys Phe Ile Cys Thr Thr Gly Lys Leu Pro Val Pro Trp
           45                  50                  55
   ccc acc ctc gtg acc acc ctg acc tac ggc gtg cag tgc ttc agc cgc      1985
   Pro Thr Leu Val Thr Thr Leu Thr Tyr Gly Val Gln Cys Phe Ser Arg
       60                  65                  70
   tac ccc gac cac atg aag cag cac gac ttc ttc aag tcc gcc atg ccc      2033
   Tyr Pro Asp His Met Lys Gln His Asp Phe Phe Lys Ser Ala Met Pro
   75                  80                  85                  90
   gaa ggc tac gtc cag gag cgc acc atc ttc ttc aag gac gac ggc aac      2081
   Glu Gly Tyr Val Gln Glu Arg Thr Ile Phe Phe Lys Asp Asp Gly Asn
                   95                  100                 105
   tac aag acc cgc gcc gag gtg aag ttc gag ggc gac acc ctg gtg aac      2129
   Tyr Lys Thr Arg Ala Glu Val Lys Phe Glu Gly Asp Thr Leu Val Asn
               110                 115                 120
   cgc atc gag ctg aag ggc atc gac ttc aag gag gac ggc aac atc ctg      2177
   Arg Ile Glu Leu Lys Gly Ile Asp Phe Lys Glu Asp Gly Asn Ile Leu
           125                 130                 135
   ggg cac aag ctg gag tac aac tac aac agc cac aac gtc tat atc atg      2225
   Gly His Lys Leu Glu Tyr Asn Tyr Asn Ser His Asn Val Tyr Ile Met
       140                 145                 150
   gcc gac aag cag aag aac ggc atc aag gtg aac ttc aag atc cgc cac      2273
   Ala Asp Lys Gln Lys Asn Gly Ile Lys Val Asn Phe Lys Ile Arg His
   155                 160                 165                 170
   aac atc gag gac ggc agc gtg cag ctc gcc gac cac tac cag cag aac      2321
   Asn Ile Glu Asp Gly Ser Val Gln Leu Ala Asp His Tyr Gln Gln Asn
                   175                 180                 185
   acc ccc atc ggc gac ggc ccc gtg ctg ctg ccc gac aac cac tac ctg      2369
   Thr Pro Ile Gly Asp Gly Pro Val Leu Leu Pro Asp Asn His Tyr Leu
               190                 195                 200
   agc acc cag tcc gcc ctg agc aaa gac ccc aac gag aag cgc gat cac      2417
   Ser Thr Gln Ser Ala Leu Ser Lys Asp Pro Asn Glu Lys Arg Asp His
           205                 210                 215
   atg gtc ctg ctg gag ttc gtg acc gcc gcc ggg atc act ctc ggc atg       2465
   Met Val Leu Leu Glu Phe Val Thr Ala Ala Gly Ile Thr Leu Gly Met
       220                 225                 230
   gac gag ctg tac aag taa agcggccgcg tcgacggatc ccccgggctg              2513
   Asp Glu Leu Tyr Lys
   235
   caggaattcg agcatcttac cgccatttat acccatattt gttctgtttt tcttgatttg    2573
   ggtatacatt taaatgttaa taaaacaaaa tggtggggca atcatttaca tttttaggga    2633
   tatgtaatta ctagttcagg tgtattgcca caagacaaac atgttaagaa actttcccgt    2693
   tatttacgct ctgttcctgt taatcaacct ctggattaca aaatttgtga aagattgact    2753
   gatattctta actatgttgc tccttttacg ctgtgtggat atgctgcttt aatgcctctg    2813
   tatcatgcta ttgcttcccg tacggctttc gttttctcct ccttgtataa atcctggttg    2873
   ctgtctcttt atgaggagtt gtggcccgtt gtccgtcaac gtggcgtggt gtgctctgtg    2933
   tttgctgacg caacccccac tggctggggc attgccacca cctgtcaact cctttctggg    2993
   actttcgctt tccccctccc gatcgccacg gcagaactca tcgccgcctg ccttgcccgc    3053
   tgctggacag gggctaggtt gctgggcact gataattccg tggtgttgtc ggggaagctg    3113
   acgtcctttc gaattcgaaa gcttttaaat atcgatgcga tgtacgggcc agatatacgc    3173
   gtatctgagg ggactagggt gtgtttaggc gaaaagcggg gcttcggttg tacgcggtta    3233
   ggagtcccct taggatatag tagtttcgct tttgcatagg gagggggaaa tgtagtctta    3293
   tgcaatactc ttgtacgtag tgcctagctc gatacaataa acgccatttg accattcacc    3353
   acattggtgt gcacctgggt tgatggccgg accgttgatt ccctgacgac tacgagcacc    3413
   tgcatgaagc agaaggcttc attctcgaga gctttggcgt aatcatggtc atagctgttt    3473
   cctgtgtgaa attgttatcc gctcacaatt ccacacaaca tacgagccgg aagcataaag    3533
   tgtaaagcct ggggtgccta atgagtgagc taactcacat taattgcgtt gcgctcactg    3593
   cccgctttcc agtcgggaaa cctgtcgtgc cagctgcatt aatgaatcgg ccaacgcgcg    3653
   gggagaggcg gtttgcgtat tgggcgctct tccgcttcct cgctcactga ctcgctgcgc    3713
   tcggtcgttc ggctgcggcg agcggtatca gctcactcaa aggcggtaat acggttatcc    3773
   acagaatcag gggataacgc aggaaagaac atgtgagcaa aaggccagca aaaggccagg    3833
   aaccgtaaaa aggccgcgtt gctggcgttt ttccataggc tccgcccccc tgacgagcat    3893
   cacaaaaatc gacgctcaag tcagaggtgg cgaaacccga caggactata aagataccag    3953
   gcgtttcccc ctggaagctc cctcgtgcgc tctcctgttc cgaccctgcc gcttaccgga    4013
   tacctgtccg cctttctccc ttcgggaagc gtggcgcttt ctcaatgctc acgctgtagg    4073
   tatctcagtt cggtgtaggt cgttcgctcc aagctgggct gtgtgcacga accccccgtt    4133
   cagcccgacc gctgcgcctt atccggtaac tatcgtcttg agtccaaccc ggtaagacac    4193
   gacttatcgc cactggcagc agccactggt aacaggatta gcagagcgag gtatgtaggc    4253
   ggtgctacag agttcttgaa gtggtggcct aactacggct acactagaag gacagtattt    4313
   ggtatctgcg ctctgctgaa gccagttacc ttcggaaaaa gagttggtag ctcttgatcc    4373
   ggcaaacaaa ccaccgctgg tagcggtggt ttttttgttt gcaagcagca gattacgcgc 4433
   agaaaaaaag gatctcaaga agatcctttg atcttttcta cggggtctga cgctcagtgg 4493
   aacgaaaact cacgttaagg gattttggtc atgagattat caaaaaggat cttcacctag 4553
   atccttttaa attaaaaatg aagttttaaa tcaatctaaa gtatatatga gtaaacttgg 4613
   tctgacagtt accaatgctt aatcagtgag gcacctatct cagcgatctg tctatttcgt 4673
   tcatccatag ttgcctgact ccccgtcgtg tagataacta cgatacggga gggcttacca 4733
   tctggcccca gtgctgcaat gataccgcga gacccacgct caccggctcc agatttatca 4793
   gcaataaacc agccagccgg aagggccgag cgcagaagtg gtcctgcaac tttatccgcc 4853
   tccatccagt ctattaattg ttgccgggaa gctagagtaa gtagttcgcc agttaatagt 4913
   ttgcgcaacg ttgttgccat tgctgctggc atcgtggtgt cacgctcgtc gtttggtatg 4973
   gcttcattca gctccggttc ccaacgatca aggcgagtta catgatcccc catgttgtgc 5033
   aaaaaagcgg ttagctcctt cggtcctccg atcgttgtca gaagtaagtt ggccgcagtg 5093
   ttatcactca tggttatggc agcactgcat aattctctta ctgtcatgcc atccgtaaga 5153
   tgcttttctg tgactggtga gtactcaacc aagtcattct gagaatagtg tatgcggcga 5213
   ccgagttgct cttgcccggc gtcaatacgg gataataccg cgccacatag cagaacttta 5273
   aaagtgctca tcattggaaa acgttcttcg gggcgaaaac tctcaaggat cttaccgctg 5333
   ttgagatcca gttcgatgta acccactcgt gcacccaact gatcttcagc atcttttact 5393
   ttcaccagcg tttctgggtg agcaaaaaca ggaaggcaaa atgccgcaaa aaagggaata 5453
   agggcgacac ggaaatgttg aatactcata ctcttccttt ttcaatatta ttgaagcatt 5513
   tatcagggtt attgtctcat gagcggatac atatttgaat gtatttagaa aaataaacaa 5573
   ataggggttc cgcgcacatt tccccgaaaa gtgccacctg acgtctaaga aaccattatt 5633
   atcatgacat taacctataa aaataggcgt atcacgaggc cctttcgtct tcaag 5688
<210> 8
   <211> 239
   <212> PRT
   <213> Artificial Sequence
<220>
   <223> Synthetic Construct
<400> 8
   Met Val Ser Lys Gly Glu Glu Leu Phe Thr Gly Val Val Pro Ile Leu
   1               5                   10                  15
   Val Glu Leu Asp Gly Asp Val Asn Gly His Lys Phe Ser Val Ser Gly
               20                  25                  30
   Glu Gly Glu Gly Asp Ala Thr Tyr Gly Lys Leu Thr Leu Lys Phe Ile
           35                  40                  45
   Cys Thr Thr Gly Lys Leu Pro Val Pro Trp Pro Thr Leu Val Thr Thr
       50                  55                  60
   Leu Thr Tyr Gly Val Gln Cys Phe Ser Arg Tyr Pro Asp His Met Lys
   65                  70                  75                  80
   Gln His Asp Phe Phe Lys Ser Ala Met Pro Glu Gly Tyr Val Gln Glu
                   85                  90                  95
   Arg Thr Ile Phe Phe Lys Asp Asp Gly Asn Tyr Lys Thr Arg Ala Glu
               100                 105                 110
   Val Lys Phe Glu Gly Asp Thr Leu Val Asn Arg Ile Glu Leu Lys Gly
           115                 120                 125
   Ile Asp Phe Lys Glu Asp Gly Asn Ile Leu Gly His Lys Leu Glu Tyr
       130                 135                 140
   Asn Tyr Asn Ser His Asn Val Tyr Ile Met Ala Asp Lys Gln Lys Asn
   145                 150                 155                 160
   Gly Ile Lys Val Asn Phe Lys Ile Arg His Asn Ile Glu Asp Gly Ser
                   165                 170                 175
   Val Gln Leu Ala Asp His Tyr Gln Gln Asn Thr Pro Ile Gly Asp Gly
               180                 185                 190
   Pro Val Leu Leu Pro Asp Asn His Tyr Leu Ser Thr Gln Ser Ala Leu
           195                 200                 205
   Ser Lys Asp Pro Asn Glu Lys Arg Asp His Met Val Leu Leu Glu Phe
       210                 215                 220
   Val Thr Ala Ala Gly Ile Thr Leu Gly Met Asp Glu Leu Tyr Lys
   225                 230                 235
<210> 9
   <211> 5704
   <212> DNA
   <213> Artificial Sequence
<220>
   <223> /note="Seq.-ID Nr. 9: viraler Vektor mit Deletion und mutierter TATA-Box in 3'-U3-Region"
<220>
   <221> enhancer
   <222> (452)..(684)
   <223> SV40 enhancer
<220>
   <221> promoter
   <222> (693)..(921)
   <223> RSV Promotor
<220>
   <221> misc_feature
   <222> (922)..(942)
   <223> 5'- R-Region
<220>
   <221> misc_feature
   <222> (943)..(1022)
   <223> 5'- U5-Region
<220>
   <221> PBS
   <222> (1023)..(1041)
<220>
   <221> promoter
   <222> (1293)..(1684)
   <223> SFFV Promotor
<220>
   <221> CDS
   <222> (1764)..(2483)
   <223> EGFP: kodierende Sequenz für eGFP
<220>
   <221> misc_signal
   <222> (2522)..(3124)
   <223> PRE
<220>
   <221> misc_feature
   <222> (3282)..(3351)
   <223> deletierte 3'- U3
<220>
   <221> misc_feature
   <222> (3322)..(3328)
   <223> mutierte TATA Box in deletierter 3'- U3
<220>
   <221> misc_feature
   <222> (3352)..(3372)
   <223> 3'- R-Region
<220>
   <221> misc_feature
   <222> (3373)..(3452)
   <223> 3'- U5-Region
<400> 9
   ctgcctcgcg cgtttcggtg atgacggtga aaacctctga cacatgcagc tcccggagac      60
   ggtcacagct tgtctgtaag cggatgccgg gagcagacaa gcccgtcagg gcgcgtcagc     120
   gggtgttggc gggtgtcggg gcgcagccat gacccagtca cgtagcgata gttactatgc     180
   ggcatcagag cagattgtac tgagagtgca ccatatgcgg tgtgaaatac cgcacagatg     240
   cgtaaggaga aaataccgca tcaggcgcca ttcgccattc aggctgcgca actgttggga     300
   agggcgatcg gtgcgggcct cttcgctatt acgccagctg gcgaaagggg gatgtgctgc     360
   aaggcgatta agttgggtaa cgccagggtt ttcccagtca cgacgttgta aaacgacggc     420
   cagtgaatta gtactctagc ttaagacgcg tggcctgaaa taacctctga aagaggaact     480
   tggttaggta ccttctgagg cggaaagaac cagctgtgga atgtgtgtca gttagggtgt     540
   ggaaagtccc caggctcccc agcaggcaga agtatgcaaa gcatgcatct caattagtca     600
   gcaaccaggt gtggaaagtc cccaggctcc ccagcaggca gaagtatgca aagcatgcat     660
   ctcaattagt cagcaaccat agtcccttaa gaatgtagtc ttatgcaata ctcttgtagt      720
   cttgcaacat ggtaacgatg agttagcaac atgccttaca aggagagaaa aagcaccgtg      780
   catgccgatt ggtggaagta aggtggtacg atcgtgcctt attaggaagg caacagacgg      840
   gtctgacatg gattggacga accactgaat tgccgcattg cagagatatt gtatttaagt      900
   gcctagctcg atacaataaa cgccatttga ccattcacca cattggtgtg cacctgggtt      960
   gatggccgga ccgttgattc cctgacgact acgagcacct gcatgaagca gaaggcttca     1020
   tttggtgacc ccgacgtgat agttagggaa tagtggtcgg ccacagacgg cgtggcgatc     1080
   ctgtctccat ccgtctcgtc tatcgggagg cgagttcgat gaccctggtg gagggggctg     1140
   cggcttaggg aggcagaagc tgagtaccgt cggagggagc tccagggccc ggagcgactg     1200
   acccctgccg agaactcaga gggtcgtcgg aagacggaga gtgagcccga cgaccacccc     1260
   aggcacgtct ttggtcggcc tgcggatcaa gcagcggccg ctagctgcag taacgccatt     1320
   ttgcaaggca tggaaaaata ccaaaccaag aatagagaag ttcagatcaa gggcgggtac     1380
   atgaaaatag ctaacgttgg gccaaacagg atatctgcgg tgagcagttt cggccccggc     1440
   ccggggccaa gaacagatgg tcaccgcagt ttcggccccg gcccgaggcc aagaacagat     1500
   ggtccccaga tatggcccaa ccctcagcag tttcttaaga cccatcagat gtttccaggc     1560
   tcccccaagg acctgaaatg accctgcgcc ttatttgaat taaccaatca gcctgcttct     1620
   cgcttctgtt cgcgcgcttc tgcttcccga gctctataaa agagctcaca acccctcact     1680
   cggcgcgcca gtcctccgac agactgagtc ggccggtcga atcaagctta tcgataccgt     1740
   cgacggatcc accggtcgcc acc atg gtg agc aag ggc gag gag ctg ttc acc    1793
                             Met Val Ser Lys Gly Glu Glu Leu Phe Thr
                             1               5                   10
   ggg gtg gtg ccc atc ctg gtc gag ctg gac ggc gac gta aac ggc cac      1841
   Gly Val Val Pro Ile Leu Val Glu Leu Asp Gly Asp Val Asn Gly His
                   15                  20                  25
   aag ttc agc gtg tcc ggc gag ggc gag ggc gat gcc acc tac ggc aag      1889
   Lys Phe Ser Val Ser Gly Glu Gly Glu Gly Asp Ala Thr Tyr Gly Lys
               30                  35                  40
   ctg acc ctg aag ttc atc tgc acc acc ggc aag ctg ccc gtg ccc tgg      1937
   Leu Thr Leu Lys Phe Ile Cys Thr Thr Gly Lys Leu Pro Val Pro Trp
           45                  50                  55
   ccc acc ctc gtg acc acc ctg acc tac ggc gtg cag tgc ttc agc cgc      1985
   Pro Thr Leu Val Thr Thr Leu Thr Tyr Gly Val Gln Cys Phe Ser Arg
       60                  65                  70
   tac ccc gac cac atg aag cag cac gac ttc ttc aag tcc gcc atg ccc      2033
   Tyr Pro Asp His Met Lys Gln His Asp Phe Phe Lys Ser Ala Met Pro
   75                  80                  85                  90
   gaa ggc tac gtc cag gag cgc acc atc ttc ttc aag gac gac ggc aac      2081
   Glu Gly Tyr Val Gln Glu Arg Thr Ile Phe Phe Lys Asp Asp Gly Asn
                   95                  100                 105
   tac aag acc cgc gcc gag gtg aag ttc gag ggc gac acc ctg gtg aac      2129
   Tyr Lys Thr Arg Ala Glu Val Lys Phe Glu Gly Asp Thr Leu Val Asn
               110                 115                 120
   cgc atc gag ctg aag ggc atc gac ttc aag gag gac ggc aac atc ctg        2177
   Arg Ile Glu Leu Lys Gly Ile Asp Phe Lys Glu Asp Gly Asn Ile Leu
           125                 130                 135
   ggg cac aag ctg gag tac aac tac aac agc cac aac gtc tat atc atg        2225
   Gly His Lys Leu Glu Tyr Asn Tyr Asn Ser His Asn Val Tyr Ile Met
       140                 145                 150
   gcc gac aag cag aag aac ggc atc aag gtg aac ttc aag atc cgc cac        2273
   Ala Asp Lys Gln Lys Asn Gly Ile Lys Val Asn Phe Lys Ile Arg His
   155                 160                 165                 170
   aac atc gag gac ggc agc gtg cag ctc gcc gac cac tac cag cag aac        2321
   Asn Ile Glu Asp Gly Ser Val Gln Leu Ala Asp His Tyr Gln Gln Asn
                   175                 180                 185
   acc ccc atc ggc gac ggc ccc gtg ctg ctg ccc gac aac cac tac ctg        2369
   Thr Pro Ile Gly Asp Gly Pro Val Leu Leu Pro Asp Asn His Tyr Leu
               190                 195                 200
   agc acc cag tcc gcc ctg agc aaa gac ccc aac gag aag cgc gat cac        2417
   Ser Thr Gln Ser Ala Leu Ser Lys Asp Pro Asn Glu Lys Arg Asp His
           205                 210                 215
   atg gtc ctg ctg gag ttc gtg acc gcc gcc ggg atc act ctc ggc atg        2465
   Met Val Leu Leu Glu Phe Val Thr Ala Ala Gly Ile Thr Leu Gly Met
       220                 225                 230
   gac gag ctg tac aag taa agcggccgcg tcgacggatc ccccgggctg               2513
   Asp Glu Leu Tyr Lys
   235
   caggaattcg agcatcttac cgccatttat acccatattt gttctgtttt tcttgatttg      2573
   ggtatacatt taaatgttaa taaaacaaaa tggtggggca atcatttaca tttttaggga      2633
   tatgtaatta ctagttcagg tgtattgcca caagacaaac atgttaagaa actttcccgt      2693
   tatttacgct ctgttcctgt taatcaacct ctggattaca aaatttgtga aagattgact      2753
   gatattctta actatgttgc tccttttacg ctgtgtggat atgctgcttt aatgcctctg      2813
   tatcatgcta ttgcttcccg tacggctttc gttttctcct ccttgtataa atcctggttg      2873
   ctgtctcttt atgaggagtt gtggcccgtt gtccgtcaac gtggcgtggt gtgctctgtg      2933
   tttgctgacg caacccccac tggctggggc attgccacca cctgtcaact cctttctggg      2993
   actttcgctt tccccctccc gatcgccacg gcagaactca tcgccgcctg ccttgcccgc      3053
   tgctggacag gggctaggtt gctgggcact gataattccg tggtgttgtc ggggaagctg      3113
   acgtcctttc gaattcgaaa gcttttaaat atcgatgcga tgtacgggcc agatatacgc      3173
   gtatctgagg ggactagggt gtgtttaggc gaaaagcggg gcttcggttg tacgcggtta      3233
   ggagtcccct taggatatag tagtttcgct tttgcatagg gagggggaaa tgtagtctta      3293
   tgcaatactc ttgtacgtag agatattgtg tctaagtgcc tagctcgata caataaacgc      3353
   catttgacca ttcaccacat tggtgtgcac ctgggttgat ggccggaccg ttgattccct      3413
   gacgactacg agcacctgca tgaagcagaa ggcttcattc tcgagagctt tggcgtaatc      3473
   atggtcatag ctgtttcctg tgtgaaattg ttatccgctc acaattccac acaacatacg      3533
   agccggaagc ataaagtgta aagcctgggg tgcctaatga gtgagctaac tcacattaat      3593
   tgcgttgcgc tcactgcccg ctttccagtc gggaaacctg tcgtgccagc tgcattaatg      3653
   aatcggccaa cgcgcgggga gaggcggttt gcgtattggg cgctcttccg cttcctcgct 3713
   cactgactcg ctgcgctcgg tcgttcggct gcggcgagcg gtatcagctc actcaaaggc 3773
   ggtaatacgg ttatccacag aatcagggga taacgcagga aagaacatgt gagcaaaagg 3833
   ccagcaaaag gccaggaacc gtaaaaaggc cgcgttgctg gcgtttttcc ataggctccg 3893
   cccccctgac gagcatcaca aaaatcgacg ctcaagtcag aggtggcgaa acccgacagg 3953
   actataaaga taccaggcgt ttccccctgg aagctccctc gtgcgctctc ctgttccgac 4013
   cctgccgctt accggatacc tgtccgcctt tctcccttcg ggaagcgtgg cgctttctca 4073
   atgctcacgc tgtaggtatc tcagttcggt gtaggtcgtt cgctccaagc tgggctgtgt 4133
   gcacgaaccc cccgttcagc ccgaccgctg cgccttatcc ggtaactatc gtcttgagtc 4193
   caacccggta agacacgact tatcgccact ggcagcagcc actggtaaca ggattagcag 4253
   agcgaggtat gtaggcggtg ctacagagtt cttgaagtgg tggcctaact acggctacac 4313
   tagaaggaca gtatttggta tctgcgctct gctgaagcca gttaccttcg gaaaaagagt 4373
   tggtagctct tgatccggca aacaaaccac cgctggtagc ggtggttttt ttgtttgcaa 4433
   gcagcagatt acgcgcagaa aaaaaggatc tcaagaagat cctttgatct tttctacggg 4493
   gtctgacgct cagtggaacg aaaactcacg ttaagggatt ttggtcatga gattatcaaa 4553
   aaggatcttc acctagatcc ttttaaatta aaaatgaagt tttaaatcaa tctaaagtat 4613
   atatgagtaa acttggtctg acagttacca atgcttaatc agtgaggcac ctatctcagc 4673
   gatctgtcta tttcgttcat ccatagttgc ctgactcccc gtcgtgtaga taactacgat 4733
   acgggagggc ttaccatctg gccccagtgc tgcaatgata ccgcgagacc cacgctcacc 4793
   ggctccagat ttatcagcaa taaaccagcc agccggaagg gccgagcgca gaagtggtcc 4853
   tgcaacttta tccgcctcca tccagtctat taattgttgc cgggaagcta gagtaagtag 4913
   ttcgccagtt aatagtttgc gcaacgttgt tgccattgct gctggcatcg tggtgtcacg 4973
   ctcgtcgttt ggtatggctt cattcagctc cggttcccaa cgatcaaggc gagttacatg 5033
   atcccccatg ttgtgcaaaa aagcggttag ctccttcggt cctccgatcg ttgtcagaag 5093
   taagttggcc gcagtgttat cactcatggt tatggcagca ctgcataatt ctcttactgt 5153
   catgccatcc gtaagatgct tttctgtgac tggtgagtac tcaaccaagt cattctgaga 5213
   atagtgtatg cggcgaccga gttgctcttg cccggcgtca atacgggata ataccgcgcc 5273
   acatagcaga actttaaaag tgctcatcat tggaaaacgt tcttcggggc gaaaactctc 5333
   aaggatctta ccgctgttga gatccagttc gatgtaaccc actcgtgcac ccaactgatc 5393
   ttcagcatct tttactttca ccagcgtttc tgggtgagca aaaacaggaa ggcaaaatgc 5453
   cgcaaaaaag ggaataaggg cgacacggaa atgttgaata ctcatactct tcctttttca 5513
   atattattga agcatttatc agggttattg tctcatgagc ggatacatat ttgaatgtat 5573
   ttagaaaaat aaacaaatag gggttccgcg cacatttccc cgaaaagtgc cacctgacgt 5633
   ctaagaaacc attattatca tgacattaac ctataaaaat aggcgtatca cgaggccctt 5693
   tcgtcttcaa g 5704
<210> 10
   <211> 239
   <212> PRT
   <213> Artificial Sequence
<220>
   <223> synthetic Construct
<400> 10
   Met Val Ser Lys Gly Glu Glu Leu Phe Thr Gly Val Val Pro Ile Leu
   1               5                   10                  15
   Val Glu Leu Asp Gly Asp Val Asn Gly His Lys Phe Ser Val Ser Gly
               20                  25                  30
   Glu Gly Glu Gly Asp Ala Thr Tyr Gly Lys Leu Thr Leu Lys Phe Ile
           35                  40                  45
   Cys Thr Thr Gly Lys Leu Pro Val Pro Trp Pro Thr Leu Val Thr Thr
       50                  55                  60
   Leu Thr Tyr Gly Val Gln Cys Phe Ser Arg Tyr Pro Asp His Met Lys
   65                  70                  75                  80
   Gln His Asp Phe Phe Lys Ser Ala Met Pro Glu Gly Tyr Val Gln Glu
                   85                  90                  95
   Arg Thr Ile Phe Phe Lys Asp Asp Gly Asn Tyr Lys Thr Arg Ala Glu
               100                 105                 110
   Val Lys Phe Glu Gly Asp Thr Leu Val Asn Arg Ile Glu Leu Lys Gly
           115                 120                 125
   Ile Asp Phe Lys Glu Asp Gly Asn Ile Leu Gly His Lys Leu Glu Tyr
       130                 135                 140
   Asn Tyr Asn Ser His Asn Val Tyr Ile Met Ala Asp Lys Gln Lys Asn
   145                 150                 155                 160
   Gly Ile Lys Val Asn Phe Lys Ile Arg His Asn Ile Glu Asp Gly Ser
                   165                 170                 175
   Val Gln Leu Ala Asp His Tyr Gln Gln Asn Thr Pro Ile Gly Asp Gly
               180                 185                 190
   Pro Val Leu Leu Pro Asp Asn His Tyr Leu Ser Thr Gln Ser Ala Leu
           195                 200                 205
   Ser Lys Asp Pro Asn Glu Lys Arg Asp His Met Val Leu Leu Glu Phe
       210                 215                 220
   Val Thr Ala Ala Gly Ile Thr Leu Gly Met Asp Glu Leu Tyr Lys
   225                 230                 235
<210> 11
   <211> 10768
   <212> DNA
   <213> Artificial Sequence
<220>
   <223> /note="Seq.-ID Nr. 11: 1. Helferplasmid gag-pol (wildtyp)"
<220>
   <221> promoter
   <222> (232)..(819)
   <223> CMV Promotor
<220>
   <221> Intron
   <222> (907)..(1463)
   <223> beta Globin-Intron
<220>
   <221> CDS
   <222> (1475)..(3586)
   <223> gag: kodierende Sequenz für Gag, wildtyp
<220>
   <221> CDS
   <222> (3604)..(6291)
   <223> pol: kodierende Sequenz für Pol, wildtyp
<220>
   <221> polyA_signal
   <222> (6368)..(6588)
   <223> poly-Adenylierungselement von bGH
<400> 11
   gacggatcgg gagatctccc gatcccctat ggtgcactct cagtacaatc tgctctgatg      60
   ccgcatagtt aagccagtat ctgctccctg cttgtgtgtt ggaggtcgct gagtagtgcg     120
   cgagcaaaat ttaagctaca acaaggcaag gcttgaccga caattgcatg aagaatctgc     180
   ttagggttag gcgttttgcg ctgcttcgcg atgtacgggc cagatatacg cgttgacatt     240
   gattattgac tagttattaa tagtaatcaa ttacggggtc attagttcat agcccatata     300
   tggagttccg cgttacataa cttacggtaa atggcccgcc tggctgaccg cccaacgacc     360
   cccgcccatt gacgtcaata atgacgtatg ttcccatagt aacgccaata gggactttcc     420
   attgacgtca atgggtggac tatttacggt aaactgccca cttggcagta catcaagtgt     480
   atcatatgcc aagtacgccc cctattgacg tcaatgacgg taaatggccc gcctggcatt     540
   atgcccagta catgacctta tgggactttc ctacttggca gtacatctac gtattagtca     600
   tcgctattac catggtgatg cggttttggc agtacatcaa tgggcgtgga tagcggtttg     660
   actcacgggg atttccaagt ctccacccca ttgacgtcaa tgggagtttg ttttggcacc     720
   aaaatcaacg ggactttcca aaatgtcgta acaactccgc cccattgacg caaatgggcg     780
   gtaggcgtgt acggtgggag gtctatataa gcagagctct ctggctaact agagaaccca     840
   ctgcttactg gcttatcgaa attaatacga ctcactatag ggagacccaa gcttggtacc     900
   gagctcggat cctgagaact tcagggtgag tctatgggac ccttgatgtt ttctttcccc      960
   ttcttttcta tggttaagtt catgtcatag gaaggggaga agtaacaggg tacacatatt     1020
   gaccaaatca gggtaatttt gcatttgtaa ttttaaaaaa tgctttcttc ttttaatata     1080
   cttttttgtt tatcttattt ctaatacttt ccctaatctc tttctttcag ggcaataatg      1140
   atacaatgta tcatgcctct ttgcaccatt ctaaagaata acagtgataa tttctgggtt     1200
   aaggcaatag caatatttct gcatataaat atttctgcat ataaattgta actgatgtaa     1260
   gaggtttcat attgctaata gcagctacaa tccagctacc attctgcttt tattttatgg     1320
   ttgggataag gctggattat tctgagtcca agctaggccc ttttgctaat catgttcata     1380
   cctcttatct tcctcccaca gctcctgggc aacgtgctgg tctgtgtgct ggcccatcac     1440
   tttggcaaag cacgtgagat ctgaattcgc cacc atg gaa gcc gtc att aag gtg     1495
                                         Met Glu Ala Val Ile Lys Val
                                         1               5
   att tcg tcc gcg tgt aaa acc tat tgc ggg aaa atc tct cct tct aag       1543
   Ile Ser Ser Ala Cys Lys Thr Tyr Cys Gly Lys Ile Ser Pro Ser Lys
           10                  15                  20
   aag gaa ata ggg gcc atg ttg tcc ctg tta caa aag gaa ggg ttg ctt       1591
   Lys Glu Ile Gly Ala Met Leu Ser Leu Leu Gln Lys Glu Gly Leu Leu
       25                  30                  35
   atg tct ccc tca gat tta tat tct ccg ggg tcc tgg gat ccc atc act       1639
   Met Ser Pro Ser Asp Leu Tyr Ser Pro Gly Ser Trp Asp Pro Ile Thr
   40                  45                  50                  55
   gcg gcg ctc tcc cag cgg gca atg gta ctt gga aaa tcg gga gag tta       1687
   Ala Ala Leu Ser Gln Arg Ala Met Val Leu Gly Lys Ser Gly Glu Leu
                   60                  65                  70
   aaa acc tgg gga ttg gtt ttg ggg gca ttg aag gcg gct cga gag gaa       1735
   Lys Thr Trp Gly Leu Val Leu Gly Ala Leu Lys Ala Ala Arg Glu Glu
               75                  80                  85
   cag gtt aca tct gag caa gca aag ttt tgg ttg gga tta ggg gga ggg       1783
   Gln Val Thr Ser Glu Gln Ala Lys Phe Trp Leu Gly Leu Gly Gly Gly
           90                  95                  100
   agg gtc tct ccc cca ggt ccg gag tgc atc gag aaa cca gct acg gag       1831
   Arg Val Ser Pro Pro Gly Pro Glu Cys Ile Glu Lys Pro Ala Thr Glu
       105                 110                 115
   cgg cga atc gac aaa ggg gag gag gtg gga gaa aca act gtg cag cga       1879
   Arg Arg Ile Asp Lys Gly Glu Glu Val Gly Glu Thr Thr Val Gln Arg
   120                 125                 130                 135
   gat gcg aag atg gcg cca gag gaa gcg gcc aca cct aaa acc gtt ggc       1927
   Asp Ala Lys Met Ala Pro Glu Glu Ala Ala Thr Pro Lys Thr Val Gly
                   140                 145                 150
   aca tcc tgc tat cat tgc gga aca gct gtt ggc tgc aat tgc gcc acc       1975
   Thr Ser Cys Tyr His Cys Gly Thr Ala Val Gly Cys Asn Cys Ala Thr
               155                 160                 165
   gcc aca gcc tcg gcc cct cct ccc cct tat gtg ggg agt ggt ttg tat       2023
   Ala Thr Ala Ser Ala Pro Pro Pro Pro Tyr Val Gly Ser Gly Leu Tyr
           170                 175                 180
   cct tcc ctg gcg ggg gtg gga gag cag cag ggc cag gga gat aac acg       2071
   Pro Ser Leu Ala Gly Val Gly Glu Gln Gln Gly Gln Gly Asp Asn Thr
       185                 190                 195
   tct cgg ggg gcg gag cag cca agg gag gag cca ggg cac gcg ggt cag      2119
   Ser Arg Gly Ala Glu Gln Pro Arg Glu Glu Pro Gly His Ala Gly Gln
   200                 205                 210                 215
   gcc cct ggg ccg gcc ctg act gac tgg gca agg gta agg gag gag ctt      2167
   Ala Pro Gly Pro Ala Leu Thr Asp Trp Ala Arg Val Arg Glu Glu Leu
                   220                 225                 230
   gcg agt act ggt ccg ccc gtg gtg gcc atg cct gta gtg att aag aca      2215
   Ala Ser Thr Gly Pro Pro Val Val Ala Met Pro Val Val Ile Lys Thr
               235                 240                 245
   gag gga ccc gcc tgg acc cct ctg gag cca aaa ttg atc aca aga ctg      2263
   Glu Gly Pro Ala Trp Thr Pro Leu Glu Pro Lys Leu Ile Thr Arg Leu
           250                 255                 260
   gct gat acg gtc agg acc aag ggc tta cga tcc ccg atc act atg gca      2311
   Ala Asp Thr Val Arg Thr Lys Gly Leu Arg Ser Pro Ile Thr Met Ala
       265                 270                 275
   gaa gtg gaa gcg ctc atg tcc tcc ccg ttg ctg ccg cat gac gtc acg      2359
   Glu Val Glu Ala Leu Met Ser Ser Pro Leu Leu Pro His Asp Val Thr
   280                 285                 290                 295
   aat cta atg aga gtg att tta gga cct gcc cca tat gcc tta tgg atg      2407
   Asn Leu Met Arg Val Ile Leu Gly Pro Ala Pro Tyr Ala Leu Trp Met
                   300                 305                 310
   gac gct tgg gga gtc caa ctc cag acg gtt ata gcg gca gcc act cgc      2455
   Asp Ala Trp Gly Val Gln Leu Gln Thr Val Ile Ala Ala Ala Thr Arg
               315                 320                 325
   gac ccc cga cac cca gcg aac ggt caa ggg cgg ggg gaa cgg act aac      2503
   Asp Pro Arg His Pro Ala Asn Gly Gln Gly Arg Gly Glu Arg Thr Asn
           330                 335                 340
   ttg gat cga tta aag ggc tta gct gat ggg atg gtg ggc aac cca cag      2551
   Leu Asp Arg Leu Lys Gly Leu Ala Asp Gly Met Val Gly Asn Pro Gln
       345                 350                 355
   ggt cag gcc gca tta tta aga ccg ggg gaa ttg gtt gct att acg gcg      2599
   Gly Gln Ala Ala Leu Leu Arg Pro Gly Glu Leu Val Ala Ile Thr Ala
   360                 365                 370                 375
   tcg gct ctc cag gcg ttt aga gaa gtt gcc cgg ctg gcg gaa cct gca      2647
   Ser Ala Leu Gln Ala Phe Arg Glu Val Ala Arg Leu Ala Glu Pro Ala
                   380                 385                 390
   ggt cca tgg gcg gac atc acg cag gga cca tct gag tcc ttt gtt gat      2695
   Gly Pro Trp Ala Asp Ile Thr Gln Gly Pro Ser Glu Ser Phe Val Asp
               395                 400                 405
   ttt gcc aat cgg ctt ata aag gcg gtt gag ggg tca gat ctc ccg cct      2743
   Phe Ala Asn Arg Leu Ile Lys Ala Val Glu Gly Ser Asp Leu Pro Pro
           410                 415                 420
   tcc gcg cgg gct ccg gtg atc att gac tgc ttt agg cag aag tca cag      2791
   Ser Ala Arg Ala Pro Val Ile Ile Asp Cys Phe Arg Gln Lys Ser Gln
       425                 430                 435
   cca gat att cag cag ctt ata cgg gca gca ccc tcc acg ctg acc acc      2839
   Pro Asp Ile Gln Gln Leu Ile Arg Ala Ala Pro Ser Thr Leu Thr Thr
   440                 445                 450                 455
   cca gga gag ata atc aaa tat gtg cta gac agg cag aag att gcc cct      2887
   Pro Gly Glu Ile Ile Lys Tyr Val Leu Asp Arg Gln Lys Ile Ala Pro
                   460                 465                 470
   ctt acg gat caa ggc ata gcc gcg gcc atg tcg tct gct atc cag ccc      2935
   Leu Thr Asp Gln Gly Ile Ala Ala Ala Met Ser Ser Ala Ile Gln Pro
               475                 480                 485
   tta gtt atg gca gta gtc aat aga gag agg gat gga caa act ggg tcg      2983
   Leu Val Met Ala Val Val Asn Arg Glu Arg Asp Gly Gln Thr Gly Ser
           490                 495                 500
   ggt ggt cgt gcc cga ggg ctc tgc tac act tgt gga tcc ccg gga cat      3031
   Gly Gly Arg Ala Arg Gly Leu Cys Tyr Thr Cys Gly Ser Pro Gly His
       505                 510                 515
   tat cag gca cag tgc ccg aaa aaa cga aag tca gga aac agc cgt gag      3079
   Tyr Gln Ala Gln Cys Pro Lys Lys Arg Lys Ser Gly Asn Ser Arg Glu
   520                 525                 530                 535
   cga tgt cag ctg tgt gac ggg atg gga cac aac gct aaa cag tgt agg      3127
   Arg Cys Gln Leu Cys Asp Gly Met Gly His Asn Ala Lys Gln Cys Arg
                   540                 545                 550
   aag cgg gat ggc aac cag ggc caa cgc cca gga aga ggt ctc tct tcg      3175
   Lys Arg Asp Gly Asn Gln Gly Gln Arg Pro Gly Arg Gly Leu Ser Ser
               555                 560                 565
   ggg ccg tgg ccc ggc cct gag cag cct gcc gtc tcg tta gcg atg aca      3223
   Gly Pro Trp Pro Gly Pro Glu Gln Pro Ala Val Ser Leu Ala Met Thr
           570                 575                 580
   atg gaa cat aaa gat cgc ccc ttg gtt agg gtc att ctg act aac act      3271
   Met Glu His Lys Asp Arg Pro Leu Val Arg Val Ile Leu Thr Asn Thr
       585                 590                 595
   ggg agt cat cca gtc aaa caa cgt tcg gtg tat atc acc gcg ctg ttg      3319
   Gly Ser His Pro Val Lys Gln Arg Ser Val Tyr Ile Thr Ala Leu Leu
   600                 605                 610                 615
   gac tcc gga gcg gac atc act att att tcg gag gag gat tgg cct act      3367
   Asp Ser Gly Ala Asp Ile Thr Ile Ile Ser Glu Glu Asp Trp Pro Thr
                   620                 625                 630
   gat tgg ccg gtg gtg gac acc gcg aac cca cag atc cat ggc ata gga      3415
   Asp Trp Pro Val Val Asp Thr Ala Asn Pro Gln Ile His Gly Ile Gly
               635                 640                 645
   ggg gga att ccc atg cga aaa tcc cgg gat atg ata gag gtg ggg gtt      3463
   Gly Gly Ile Pro Met Arg Lys Ser Arg Asp Met Ile Glu Val Gly Val
           650                 655                 660
   att aac cga gac ggg tcg ttg gag cga ccc ctg ctc ctc ttc ccc gca      3511
   Ile Asn Arg Asp Gly Ser Leu Glu Arg Pro Leu Leu Leu Phe Pro Ala
       665                 670                 675
   gtc gct atg gtt aga ggg agt atc cta gga aga gat tgt ctg cag ggc      3559
   Val Ala Met Val Arg Gly Ser Ile Leu Gly Arg Asp Cys Leu Gln Gly
   680                 685                 690                 695
   cta ggg ctc cgc ttg aca aat tta tag ggagggccac tgttctt act gtt      3609
   Leu Gly Leu Arg Leu Thr Asn Leu                        Thr Val
                   700                                        705
   gcg cta cat ctg gct att ccg ctc aaa tgg aag tca gac cgc acg cct      3657
   Ala Leu His Leu Ala Ile Pro Leu Lys Trp Lys Ser Asp Arg Thr Pro
                   710                 715                 720
   gtg tgg att gac cag tgg ccc ctt cct gaa ggt aaa ctt gta gcg cta      3705
   Val Trp Ile Asp Gln Trp Pro Leu Pro Glu Gly Lys Leu Val Ala Leu
               725                 730                 735
   acg caa tta gtg gaa aaa gaa tta cag tta gga cat ata gag ccc tca      3753
   Thr Gln Leu Val Glu Lys Glu Leu Gln Leu Gly His Ile Glu Pro Ser
           740                 745                 750
   ctt agt tgt tgg aac aca cct gtt ttt gtg atc cgg aag gct tcc ggg      3801
   Leu Ser Cys Trp Asn Thr Pro Val Phe Val Ile Arg Lys Ala Ser Gly
       755                 760                 765
   tct tat cgc tta ttg cat gat ttg cgc gct gtt aac gcc aag ctt gtc      3849
   Ser Tyr Arg Leu Leu His Asp Leu Arg Ala Val Asn Ala Lys Leu Val
   770                 775                 780                 785
   cct ttt ggg gcc gtc caa cag ggg gcg cca gtt ctc tcc gcg ctc ccg      3897
   Pro Phe Gly Ala Val Gln Gln Gly Ala Pro Val Leu Ser Ala Leu Pro
                   790                 795                 800
   cgt ggc tgg ccc ctg atg gtc cta gac ctc aag gat tgc ttc ttt tct      3945
   Arg Gly Trp Pro Leu Met Val Leu Asp Leu Lys Asp Cys Phe Phe Ser
               805                 810                 815
   atc cct ctt gcg gaa caa gat cgc gaa gct ttt gca ttt acg ctc ccc      3993
   Ile Pro Leu Ala Glu Gln Asp Arg Glu Ala Phe Ala Phe Thr Leu Pro
           820                 825                 830
   tct gtg aat aac cag gcc ccc gct cga aga ttc caa tgg aag gtc ttg      4041
   Ser Val Asn Asn Gln Ala Pro Ala Arg Arg Phe Gln Trp Lys Val Leu
       835                 840                 845
   ccc caa ggg atg acc tgt tct ccc act atc tgt cag ttg gta gtg ggt      4089
   Pro Gln Gly Met Thr Cys Ser Pro Thr Ile Cys Gln Leu Val Val Gly
   850                 855                 860                 865
   cag gtg ctc gag ccc ttg cga ctc aag cac cca gct ctg cgc atg ttg      4137
   Gln Val Leu Glu Pro Leu Arg Leu Lys His Pro Ala Leu Arg Met Leu
                   870                 875                 880
   cat tat atg gac gat ctt ttg cta gcc gcc tca agt cat gat ggg ttg      4185
   His Tyr Met Asp Asp Leu Leu Leu Ala Ala Ser Ser His Asp Gly Leu
               885                 890                 895
   gaa gcg gca ggg aag gag gtt atc ggt aca ttg gaa aga gcc ggg ttc      4233
   Glu Ala Ala Gly Lys Glu Val Ile Gly Thr Leu Glu Arg Ala Gly Phe
           900                 905                 910
   act att tcg ccg gat aag atc cag agg gag ccc gga gta caa tat ctt      4281
   Thr Ile Ser Pro Asp Lys Ile Gln Arg Glu Pro Gly Val Gln Tyr Leu
       915                 920                 925
   ggg tac aag tta ggc agt acg tat gta gca ccc gta ggc ttg gta gca      4329
   Gly Tyr Lys Leu Gly Ser Thr Tyr Val Ala Pro Val Gly Leu Val Ala
   930                 935                 940                 945
   gaa ccc agg ata gcc acc ttg tgg gat gtt caa aag ctg gtg ggg tca      4377
   Glu Pro Arg Ile Ala Thr Leu Trp Asp Val Gln Lys Leu Val Gly Ser
                   950                 955                 960
   ctt cag tgg ctt cgc cca gcg tta ggg atc ccg cca cga ctg atg ggt      4425
   Leu Gln Trp Leu Arg Pro Ala Leu Gly Ile Pro Pro Arg Leu Met Gly
               965                 970                 975
   ccc ttt tat gag cag tta cga ggg tca gat cct aac gag gcg agg gaa      4473
   Pro Phe Tyr Glu Gln Leu Arg Gly Ser Asp Pro Asn Glu Ala Arg Glu
           980                 985                 990
   tgg aat cta gac atg aaa atg  gcc tgg aga gag atc  gta cag ctt agc    4521
   Trp Asn Leu Asp Met Lys Met  Ala Trp Arg Glu Ile  Val Gln Leu Ser
       995                 1000                 1005
   act  act gct gcc ttg gaa  cga tgg gac cct gcc  cag cct ctg gaa       4566
   Thr  Thr Ala Ala Leu Glu  Arg Trp Asp Pro Ala  Gln Pro Leu Glu
   1010                1015                1020
   gga gcg gtc gct aga tgt gaa cag ggg gca ata ggg gtc ctg gga      4611
   Gly Ala Val Ala Arg Cys Glu Gln Gly Ala Ile Gly Val Leu Gly
   1025                1030                1035
   cag gga ctg tcc aca cac cca agg cca tgt ttg tgg tta ttc tcc      4656
   Gln Gly Leu Ser Thr His Pro Arg Pro Cys Leu Trp Leu Phe Ser
   1040                1045                1050
   acc caa ccc acc aag gcg ttt act gct tgg tta gaa gtg ctc acc      4701
   Thr Gln Pro Thr Lys Ala Phe Thr Ala Trp Leu Glu Val Leu Thr
   1055                1060                1065
   ctt ttg att act aag cta cgc gct tcg gca gtg cga acc ttt ggc      4746
   Leu Leu Ile Thr Lys Leu Arg Ala Ser Ala Val Arg Thr Phe Gly
   1070                1075                1080
   aag gag gtt gat atc ctc ctg ttg cct gca tgc ttc cgg gag gac      4791
   Lys Glu Val Asp Ile Leu Leu Leu Pro Ala Cys Phe Arg Glu Asp
   1085                1090                1095
   ctt ccg ctc ccg gag ggg atc ctg tta gca ctt agg ggg ttt gca      4836
   Leu Pro Leu Pro Glu Gly Ile Leu Leu Ala Leu Arg Gly Phe Ala
   1100                1105                1110
   gga aaa atc agg agt agt gac acg cca tct att ttt gac att gcg      4881
   Gly Lys Ile Arg Ser Ser Asp Thr Pro Ser Ile Phe Asp Ile Ala
   1115                1120                1125
   cgt cca ctg cat gtt tct ctg aaa gtg agg gtt acc gac cac cct      4926
   Arg Pro Leu His Val Ser Leu Lys Val Arg Val Thr Asp His Pro
   1130                1135                1140
   gtg ccg gga ccc act gtc ttt acc gac gcc tcc tca agc acc cat      4971
   Val Pro Gly Pro Thr Val Phe Thr Asp Ala Ser Ser Ser Thr His
   1145                1150                1155
   aaa ggg gtg gta gtc tgg agg gag ggc cca agg tgg gag ata aaa      5016
   Lys Gly Val Val Val Trp Arg Glu Gly Pro Arg Trp Glu Ile Lys
   1160                1165                1170
   gaa ata gtt gat ttg ggg gca agt gta caa caa ctg gag gca cgc      5061
   Glu Ile Val Asp Leu Gly Ala Ser Val Gln Gln Leu Glu Ala Arg
   1175                1180                1185
   gct gtg gcc atg gca ctt ctg ctg tgg ccg aca acg ccc act aat      5106
   Ala Val Ala Met Ala Leu Leu Leu Trp Pro Thr Thr Pro Thr Asn
   1190                1195                1200
   gta gtg act gac tct gcg ttt gtt gcg aaa atg tta ctc aag atg      5151
   Val Val Thr Asp Ser Ala Phe Val Ala Lys Met Leu Leu Lys Met
   1205                1210                1215
   gga cag gag gga gtc ccg tct aca gcg gca gct ttt att tta gag      5196
   Gly Gln Glu Gly Val Pro Ser Thr Ala Ala Ala Phe Ile Leu Glu
   1220                1225                1230
   gat gcg tta agc caa agg tca gcc atg gcc gcc gtt ctc cac gtg      5241
   Asp Ala Leu Ser Gln Arg Ser Ala Met Ala Ala Val Leu His Val
   1235                1240                1245
   cgg agt cat tct gaa gtg cca ggg ttt ttc aca gaa gga aat gac      5286
   Arg Ser His Ser Glu Val Pro Gly Phe Phe Thr Glu Gly Asn Asp
   1250                1255                1260
   gtg gca gat agc caa gcc acc ttt caa gcg tat ccc ttg aga gag      5331
   Val Ala Asp Ser Gln Ala Thr Phe Gln Ala Tyr Pro Leu Arg Glu
   1265                1270                1275
   gct  aaa gat ctt cat acc  gct ctc cat att gga  ccc cgc gcg cta     5376
   Ala  Lys Asp Leu His Thr  Ala Leu His Ile Gly  Pro Arg Ala Leu
   1280                 1285                 1290
   tcc  aaa gcg tgt aat ata  tct atg cag cag gct  agg gag gtt gtt     5421
   Ser  Lys Ala Cys Asn Ile  Ser Met Gln Gln Ala  Arg Glu Val Val
   1295                 1300                 1305
   cag  acc tgc ccg cat tgt  aat tca gcc cct gcg  ttg gag gcc ggg     5466
   Gln  Thr Cys Pro His Cys  Asn Ser Ala Pro Ala  Leu Glu Ala Gly
   1310                 1315                 1320
   gta  aac cct agg ggt ttg  gga ccc cta cag ata  tgg cag aca gac     5511
   Val  Asn Pro Arg Gly Leu  Gly Pro Leu Gln Ile  Trp Gln Thr Asp
   1325                 1330                 1335
   ttt  acg ctt gag cct aga  atg gct ccc cgt tcc  tgg ctc gct gtt     5556
   Phe  Thr Leu Glu Pro Arg  Met Ala Pro Arg Ser  Trp Leu Ala Val
   1340                 1345                 1350
   act  gtg gac acc gcc tca  tca gcg ata gtc gta  act cag cat ggc     5601
   Thr  Val Asp Thr Ala Ser  Ser Ala Ile Val Val  Thr Gln His Gly
   1355                 1360                 1365
   cgt  gtt aca tcg gtt gct  gca caa cat cat tgg  gcc acg gct atc     5646
   Arg  Val Thr Ser Val Ala  Ala Gln His His Trp  Ala Thr Ala Ile
   1370                 1375                 1380
   gcc  gtt ttg gga aga cca  aag gcc ata aaa aca  gat aac ggg tcc     5691
   Ala  Val Leu Gly Arg Pro  Lys Ala Ile Lys Thr  Asp Asn Gly Ser
   1385                 1390                 1395
   tgc  ttc acg tct aga tcc  acg cgg gag tgg ctc  gcg aga tgg ggg     5736
   Cys  Phe Thr Ser Arg Ser  Thr Arg Glu Trp Leu  Ala Arg Trp Gly
   1400                 1405                 1410
   ata  gca cac acc acc ggg  att ccg gga aat tcc  cag ggt caa gct     5781
   Ile  Ala His Thr Thr Gly  Ile Pro Gly Asn Ser  Gln Gly Gln Ala
   1415                 1420                 1425
   atg  gta gag cgg gcc aac  cgg ctc ctg aaa gat  aag atc cgt gtg     5826
   Met  Val Glu Arg Ala Asn  Arg Leu Leu Lys Asp  Lys Ile Arg Val
   1430                 1435                 1440
   ctt  gcg gag ggg gac ggc  ttt atg aaa aga atc  ccc gcc agc aaa     5871
   Leu  Ala Glu Gly Asp Gly  Phe Met Lys Arg Ile  Pro Ala Ser Lys
   1445                 1450                 1455
   cag  ggg gaa cta cta gcc  aag gca atg tat gcc  ctc aat cac ttt     5916
   Gln  Gly Glu Leu Leu Ala  Lys Ala Met Tyr Ala  Leu Asn His Phe
   1460                 1465                 1470
   gag  cgt ggt gaa aac aca  aaa aca ccg gta caa  aaa cac tgg aga     5961
   Glu  Arg Gly Glu Asn Thr  Lys Thr Pro Val Gln  Lys His Trp Arg
   1475                 1480                 1485
   cct  acc gtt ctt aca gaa  gga ccc ccg gtt aaa  ata cga ata gag     6006
   Pro  Thr Val Leu Thr Glu  Gly Pro Pro Val Lys  Ile Arg Ile Glu
   1490                 1495                 1500
   aca  ggg gag tgg gaa aaa  gga tgg aac gtg ctg  gtc tgg gga cga     6051
   Thr  Gly Glu Trp Glu Lys  Gly Trp Asn Val Leu  Val Trp Gly Arg
   1505                 1510                 1515
   ggt  tat gcc gct gtg aaa  aac agg gac act gat  aag gtt att tgg     6096
   Gly  Tyr Ala Ala Val Lys  Asn Arg Asp Thr Asp  Lys Val Ile Trp
   1520                 1525                 1530
   gta  ccc tct cgg aaa gtt  aaa ccg gat gtc acc  caa aag gat gag     6141
   Val  Pro Ser Arg Lys Val  Lys Pro Asp Val Thr  Gln Lys Asp Glu
   1535                 1540                 1545
   gtg  act aag aaa gat gag  gcg agc cct ctt ttt  gca ggc att tct     6186
   Val  Thr Lys Lys Asp Glu  Ala Ser Pro Leu Phe  Ala Gly Ile Ser
   1550                 1555                 1560
   gac  tgg ata ccc tgg gaa  gac gag caa gaa gga  ctc caa gga gaa     6231
   Asp  Trp Ile Pro Trp Glu  Asp Glu Gln Glu Gly  Leu Gln Gly Glu
   1565                 1570                 1575
   acc  gct agc aac aag caa  gaa aga ccc gga gaa  gac acc ctt gct     6276
   Thr  Ala Ser Asn Lys Gln  Glu Arg Pro Gly Glu  Asp Thr Leu Ala
   1580                 1585                 1590
   gcc  aac gag agt taa gaattctgca gatatccagc acagtggcgg ccgctcgagt   6331
   Ala  Asn Glu Ser
   1595
   ctagagggcc cgtttaaacc cgctgatcag cctcgactgt gccttctagt tgccagccat   6391
   ctgttgtttg cccctccccc gtgccttcct tgaccctgga aggtgccact cccactgtcc   6451
   tttcctaata aaatgaggaa attgcatcgc attgtctgag taggtgtcat tctattctgg   6511
   ggggtggggt ggggcaggac agcaaggggg aggattggga agacaatagc aggcatgctg   6571
   gggatgcggt gggctctatg gcttctgagg cggaaagaac cagctggggc tctagggggt   6631
   atccccacgc gccctgtagc ggcgcattaa gcgcggcggg tgtggtggtt acgcgcagcg   6691
   tgaccgctac acttgccagc gccctagcgc ccgctccttt cgctttcttc ccttcctttc   6751
   tcgccacgtt cgccggcttt ccccgtcaag ctctaaatcg ggggctccct ttagggttcc   6811
   gatttagtgc tttacggcac ctcgacccca aaaaacttga ttagggtgat ggttcacgta   6871
   gtgggccatc gccctgatag acggtttttc gccctttgac gttggagtcc acgttcttta   6931
   atagtggact cttgttccaa actggaacaa cactcaaccc tatctcggtc tattcttttg   6991
   atttataagg gattttgccg atttcggcct attggttaaa aaatgagctg atttaacaaa   7051
   aatttaacgc gaattaattc tgtggaatgt gtgtcagtta gggtgtggaa agtccccagg   7111
   ctccccagca ggcagaagta tgcaaagcat gcatctcaat tagtcagcaa ccaggtgtgg   7171
   aaagtcccca ggctccccag caggcagaag tatgcaaagc atgcatctca attagtcagc   7231
   aaccatagtc ccgcccctaa ctccgcccat cccgccccta actccgccca gttccgccca   7291
   ttctccgccc catggctgac taattttttt tatttatgca gaggccgagg ccgcctctgc   7351
   ctctgagcta ttccagaagt agtgaggagg cttttttgga ggcctaggct tttgcaaaaa   7411
   gctcccggga gcttgtatat ccattttcgg atctgatcaa gagacaggat gaggatcgtt   7471
   tcgcatgatt gaacaagatg gattgcacgc aggttctccg gccgcttggg tggagaggct   7531
   attcggctat gactgggcac aacagacaat cggctgctct gatgccgccg tgttccggct   7591
   gtcagcgcag gggcgcccgg ttctttttgt caagaccgac ctgtccggtg ccctgaatga   7651
   actgcaggac gaggcagcgc ggctatcgtg gctggccacg acgggcgttc cttgcgcagc   7711
   tgtgctcgac gttgtcactg aagcgggaag ggactggctg ctattgggcg aagtgccggg   7771
   gcaggatctc ctgtcatctc accttgctcc tgccgagaaa gtatccatca tggctgatgc   7831
   aatgcggcgg ctgcatacgc ttgatccggc tacctgccca ttcgaccacc aagcgaaaca 7891
   tcgcatcgag cgagcacgta ctcggatgga agccggtctt gtcgatcagg atgatctgga 7951
   cgaagagcat caggggctcg cgccagccga actgttcgcc aggctcaagg cgcgcatgcc 8011
   cgacggcgag gatctcgtcg tgacccatgg cgatgcctgc ttgccgaata tcatggtgga 8071
   aaatggccgc ttttctggat tcatcgactg tggccggctg ggtgtggcgg accgctatca 8131
   ggacatagcg ttggctaccc gtgatattgc tgaagagctt ggcggcgaat gggctgaccg 8191
   cttcctcgtg ctttacggta tcgccgctcc cgattcgcag cgcatcgcct tctatcgcct 8251
   tcttgacgag ttcttctgag cgggactctg gggttcgaaa tgaccgacca agcgacgccc 8311
   aacctgccat cacgagattt cgattccacc gccgccttct atgaaaggtt gggcttcgga 8371
   atcgttttcc gggacgccgg ctggatgatc ctccagcgcg gggatctcat gctggagttc 8431
   ttcgcccacc ccaacttgtt tattgcagct tataatggtt acaaataaag caatagcatc 8491
   acaaatttca caaataaagc atttttttca ctgcattcta gttgtggttt gtccaaactc 8551
   atcaatgtat cttatcatgt ctgtataccg tcgacctcta gctagagctt ggcgtaatca 8611
   tggtcatagc tgtttcctgt gtgaaattgt tatccgctca caattccaca caacatacga 8671
   gccggaagca taaagtgtaa agcctggggt gcctaatgag tgagctaact cacattaatt 8731
   gcgttgcgct cactgcccgc tttccagtcg ggaaacctgt cgtgccagct gcattaatga 8791
   atcggccaac gcgcggggag aggcggtttg cgtattgggc gctcttccgc ttcctcgctc 8851
   actgactcgc tgcgctcggt cgttcggctg cggcgagcgg tatcagctca ctcaaaggcg 8911
   gtaatacggt tatccacaga atcaggggat aacgcaggaa agaacatgtg agcaaaaggc 8971
   cagcaaaagg ccaggaaccg taaaaaggcc gcgttgctgg cgtttttcca taggctccgc 9031
   ccccctgacg agcatcacaa aaatcgacgc tcaagtcaga ggtggcgaaa cccgacagga 9091
   ctataaagat accaggcgtt tccccctgga agctccctcg tgcgctctcc tgttccgacc 9151
   ctgccgctta ccggatacct gtccgccttt ctcccttcgg gaagcgtggc gctttctcat 9211
   agctcacgct gtaggtatct cagttcggtg taggtcgttc gctccaagct gggctgtgtg 9271
   cacgaacccc ccgttcagcc cgaccgctgc gccttatccg gtaactatcg tcttgagtcc 9331
   aacccggtaa gacacgactt atcgccactg gcagcagcca ctggtaacag gattagcaga 9391
   gcgaggtatg taggcggtgc tacagagttc ttgaagtggt ggcctaacta cggctacact 9451
   agaagaacag tatttggtat ctgcgctctg ctgaagccag ttaccttcgg aaaaagagtt 9511
   ggtagctctt gatccggcaa acaaaccacc gctggtagcg gtttttttgt ttgcaagcag 9571
   cagattacgc gcagaaaaaa aggatctcaa gaagatcctt tgatcttttc tacggggtct 9631
   gacgctcagt ggaacgaaaa ctcacgttaa gggattttgg tcatgagatt atcaaaaagg 9691
   atcttcacct agatcctttt aaattaaaaa tgaagtttta aatcaatcta aagtatatat 9751
   gagtaaactt ggtctgacag ttaccaatgc ttaatcagtg aggcacctat ctcagcgatc 9811
   tgtctatttc gttcatccat agttgcctga ctccccgtcg tgtagataac tacgatacgg 9871
   gagggcttac catctggccc cagtgctgca atgataccgc gagacccacg ctcaccggct      9931
   ccagatttat cagcaataaa ccagccagcc ggaagggccg agcgcagaag tggtcctgca      9991
   actttatccg cctccatcca gtctattaat tgttgccggg aagctagagt aagtagttcg     10051
   ccagttaata gtttgcgcaa cgttgttgcc attgctacag gcatcgtggt gtcacgctcg     10111
   tcgtttggta tggcttcatt cagctccggt tcccaacgat caaggcgagt tacatgatcc     10171
   cccatgttgt gcaaaaaagc ggttagctcc ttcggtcctc cgatcgttgt cagaagtaag     10231
   ttggccgcag tgttatcact catggttatg gcagcactgc ataattctct tactgtcatg     10291
   ccatccgtaa gatgcttttc tgtgactggt gagtactcaa ccaagtcatt ctgagaatag     10351
   tgtatgcggc gaccgagttg ctcttgcccg gcgtcaatac gggataatac cgcgccacat     10411
   agcagaactt taaaagtgct catcattgga aaacgttctt cggggcgaaa actctcaagg     10471
   atcttaccgc tgttgagatc cagttcgatg taacccactc gtgcacccaa ctgatcttca     10531
   gcatctttta ctttcaccag cgtttctggg tgagcaaaaa caggaaggca aaatgccgca     10591
   aaaaagggaa taagggcgac acggaaatgt tgaatactca tactcttcct ttttcaatat     10651
   tattgaagca tttatcaggg ttattgtctc atgagcggat acatatttga atgtatttag     10711
   aaaaataaac aaataggggt tccgcgcaca tttccccgaa aagtgccacc tgacgtc        10768
<210> 12
   <211> 703
   <212> PRT
   <213> Artificial Sequence
<220>
   <223> Synthetic Construct
<400> 12
   Met Glu Ala Val Ile Lys Val Ile Ser Ser Ala Cys Lys Thr Tyr Cys
   1               5                   10                  15
   Gly Lys Ile Ser Pro Ser Lys Lys Glu Ile Gly Ala Met Leu Ser Leu
               20                  25                  30
   Leu Gln Lys Glu Gly Leu Leu Met Ser Pro Ser Asp Leu Tyr Ser Pro
           35                  40                  45
   Gly Ser Trp Asp Pro Ile Thr Ala Ala Leu Ser Gln Arg Ala Met Val
       50                  55                  60
   Leu Gly Lys Ser Gly Glu Leu Lys Thr Trp Gly Leu Val Leu Gly Ala
   65                  70                  75                  80
   Leu Lys Ala Ala Arg Glu Glu Gln Val Thr Ser Glu Gln Ala Lys Phe
                   85                  90                  95
   Trp Leu Gly Leu Gly Gly Gly Arg Val Ser Pro Pro Gly Pro Glu Cys
               100                 105                 110
   Ile Glu Lys Pro Ala Thr Glu Arg Arg Ile Asp Lys Gly Glu Glu Val
   115 120 125
   Gly Glu Thr Thr Val Gln Arg Asp Ala Lys Met Ala Pro Glu Glu Ala
   130 135 140
   Ala Thr Pro Lys Thr Val Gly Thr Ser Cys Tyr His Cys Gly Thr Ala
   145 150 155 160
   Val Gly Cys Asn Cys Ala Thr Ala Thr Ala Ser Ala Pro Pro Pro Pro
   165 170 175
   Tyr Val Gly Ser Gly Leu Tyr Pro Ser Leu Ala Gly Val Gly Glu Gln
   180 185 190
   Gln Gly Gln Gly Asp Asn Thr Ser Arg Gly Ala Glu Gln Pro Arg Glu
   195 200 205
   Glu Pro Gly His Ala Gly Gln Ala Pro Gly Pro Ala Leu Thr Asp Trp
   210 215 220
   Ala Arg Val Arg Glu Glu Leu Ala Ser Thr Gly Pro Pro Val Val Ala
   225 230 235 240
   Met Pro Val Val Ile Lys Thr Glu Gly Pro Ala Trp Thr Pro Leu Glu
   245 250 255
   Pro Lys Leu Ile Thr Arg Leu Ala Asp Thr Val Arg Thr Lys Gly Leu
   260 265 270
   Arg Ser Pro Ile Thr Met Ala Glu Val Glu Ala Leu Met Ser Ser Pro
   275 280 285
   Leu Leu Pro His Asp Val Thr Asn Leu Met Arg Val Ile Leu Gly Pro
   290 295 300
   Ala Pro Tyr Ala Leu Trp Met Asp Ala Trp Gly Val Gln Leu Gln Thr
   305 310 315 320
   Val Ile Ala Ala Ala Thr Arg Asp Pro Arg His Pro Ala Asn Gly Gln
   325 330 335
   Gly Arg Gly Glu Arg Thr Asn Leu Asp Arg Leu Lys Gly Leu Ala Asp
   340 345 350
   Gly Met Val Gly Asn Pro Gln Gly Gln Ala Ala Leu Leu Arg Pro Gly
   355 360 365
   Glu Leu Val Ala Ile Thr Ala Ser Ala Leu Gln Ala Phe Arg Glu Val
   370 375 380
   Ala Arg Leu Ala Glu Pro Ala Gly Pro Trp Ala Asp Ile Thr Gln Gly
   385                 390                 395                 400
   Pro Ser Glu Ser Phe Val Asp Phe Ala Asn Arg Leu Ile Lys Ala Val
                   405                 410                 415
   Glu Gly Ser Asp Leu Pro Pro Ser Ala Arg Ala Pro Val Ile Ile Asp
               420                 425                 430
   Cys Phe Arg Gln Lys Ser Gln Pro Asp Ile Gln Gln Leu Ile Arg Ala
           435                 440                 445
   Ala Pro Ser Thr Leu Thr Thr Pro Gly Glu Ile Ile Lys Tyr Val Leu
       450                 455                 460
   Asp Arg Gln Lys Ile Ala Pro Leu Thr Asp Gln Gly Ile Ala Ala Ala
   465                 470                 475                 480
   Met Ser Ser Ala Ile Gln Pro Leu Val Met Ala Val Val Asn Arg Glu
                   485                 490                 495
   Arg Asp Gly Gln Thr Gly Ser Gly Gly Arg Ala Arg Gly Leu Cys Tyr
               500                 505                 510
   Thr Cys Gly Ser Pro Gly His Tyr Gln Ala Gln Cys Pro Lys Lys Arg
           515                 520                 525
   Lys Ser Gly Asn Ser Arg Glu Arg Cys Gln Leu Cys Asp Gly Met Gly
       530                 535                 540
   His Asn Ala Lys Gln Cys Arg Lys Arg Asp Gly Asn Gln Gly Gln Arg
   545                 550                 555                 560
   Pro Gly Arg Gly Leu Ser Ser Gly Pro Trp Pro Gly Pro Glu Gln Pro
                   565                 570                 575
   Ala Val Ser Leu Ala Met Thr Met Glu His Lys Asp Arg Pro Leu Val
               580                 585                 590
   Arg Val Ile Leu Thr Asn Thr Gly Ser His Pro Val Lys Gln Arg Ser
           595                 600                 605
   Val Tyr Ile Thr Ala Leu Leu Asp Ser Gly Ala Asp Ile Thr Ile Ile
       610                 615                 620
   Ser Glu Glu Asp Trp Pro Thr Asp Trp Pro Val Val Asp Thr Ala Asn
   625                 630                 635                 640
   Pro Gln Ile His Gly Ile Gly Gly Gly Ile Pro Met Arg Lys Ser Arg
                   645                 650                 655
   Asp Met Ile Glu Val Gly Val Ile Asn Arg Asp Gly Ser Leu Glu Arg
               660                 665                 670
   Pro Leu Leu Leu Phe Pro Ala Val Ala Met Val Arg Gly Ser Ile Leu
   675 680 685
   Gly Arg Asp Cys Leu Gln Gly Leu Gly Leu Arg Leu Thr Asn Leu
   690 695 700
<210> 13
   <211> 895
   <212> PRT
   <213> Artificial Sequence
<220>
   <223> Synthetic Construct
<400> 13
   Thr Val Ala Leu His Leu Ala Ile Pro Leu Lys Trp Lys Ser Asp Arg
   1               5                   10                  15
   Thr Pro Val Trp Ile Asp Gln Trp Pro Leu Pro Glu Gly Lys Leu Val
               20                  25                  30
   Ala Leu Thr Gln Leu Val Glu Lys Glu Leu Gln Leu Gly His Ile Glu
           35                  40                  45
   Pro Ser Leu Ser Cys Trp Asn Thr Pro Val Phe Val Ile Arg Lys Ala
       50                  55                  60
   Ser Gly Ser Tyr Arg Leu Leu His Asp Leu Arg Ala Val Asn Ala Lys
   65                  70                  75                  80
   Leu Val Pro Phe Gly Ala Val Gln Gln Gly Ala Pro Val Leu Ser Ala
                   85                  90                  95
   Leu Pro Arg Gly Trp Pro Leu Met Val Leu Asp Leu Lys Asp Cys Phe
               100                 105                 110
   Phe Ser Ile Pro Leu Ala Glu Gln Asp Arg Glu Ala Phe Ala Phe Thr
           115                 120                 125
   Leu Pro Ser Val Asn Asn Gln Ala Pro Ala Arg Arg Phe Gln Trp Lys
       130                 135                 140
   Val Leu Pro Gln Gly Met Thr Cys Ser Pro Thr Ile Cys Gln Leu Val
   145                 150                 155                 160
   Val Gly Gln Val Leu Glu Pro Leu Arg Leu Lys His Pro Ala Leu Arg
                   165                 170                 175
   Met Leu His Tyr Met Asp Asp Leu Leu Leu Ala Ala Ser Ser His Asp
               180                 185                 190
   Gly Leu Glu Ala Ala Gly Lys Glu Val Ile Gly Thr Leu Glu Arg Ala
               195                 200                 205
   Gly Phe Thr Ile Ser Pro Asp Lys Ile Gln Arg Glu Pro Gly Val Gln
       210                 215                 220
   Tyr Leu Gly Tyr Lys Leu Gly Ser Thr Tyr Val Ala Pro Val Gly Leu
   225                 230                 235                 240
   Val Ala Glu Pro Arg Ile Ala Thr Leu Trp Asp Val Gln Lys Leu Val
                   245                 250                 255
   Gly Ser Leu Gln Trp Leu Arg Pro Ala Leu Gly Ile Pro Pro Arg Leu
               260                 265                 270
   Met Gly Pro Phe Tyr Glu Gln Leu Arg Gly Ser Asp Pro Asn Glu Ala
           275                 280                 285
   Arg Glu Trp Asn Leu Asp Met Lys Met Ala Trp Arg Glu Ile Val Gln
       290                 295                 300
   Leu Ser Thr Thr Ala Ala Leu Glu Arg Trp Asp Pro Ala Gln Pro Leu
   305                 310                 315                 320
   Glu Gly Ala Val Ala Arg Cys Glu Gln Gly Ala Ile Gly Val Leu Gly
                   325                 330                 335
   Gln Gly Leu Ser Thr His Pro Arg Pro Cys Leu Trp Leu Phe Ser Thr
               340                 345                 350
   Gln Pro Thr Lys Ala Phe Thr Ala Trp Leu Glu Val Leu Thr Leu Leu
           355                 360                 365
   Ile Thr Lys Leu Arg Ala Ser Ala Val Arg Thr Phe Gly Lys Glu Val
       370                 375                 380
   Asp Ile Leu Leu Leu Pro Ala Cys Phe Arg Glu Asp Leu Pro Leu Pro
   385                 390                 395                 400
   Glu Gly Ile Leu Leu Ala Leu Arg Gly Phe Ala Gly Lys Ile Arg Ser
                   405                 410                 415
   Ser Asp Thr Pro Ser Ile Phe Asp Ile Ala Arg Pro Leu His Val Ser
               420                 425                 430
   Leu Lys Val Arg Val Thr Asp His Pro Val Pro Gly Pro Thr Val Phe
           435                 440                 445
   Thr Asp Ala Ser Ser Ser Thr His Lys Gly Val Val Val Trp Arg Glu
       450                 455                 460
   Gly Pro Arg Trp Glu Ile Lys Glu Ile Val Asp Leu Gly Ala Ser Val
   465                 470                 475                 480
   Gln Gln Leu Glu Ala Arg Ala Val Ala Met Ala Leu Leu Leu Trp Pro
   485 490 495
   Thr Thr Pro Thr Asn Val Val Thr Asp Ser Ala Phe Val Ala Lys Met
   500 505 510
   Leu Leu Lys Met Gly Gln Glu Gly Val Pro Ser Thr Ala Ala Ala Phe
   515 520 525
   Ile Leu Glu Asp Ala Leu Ser Gln Arg Ser Ala Met Ala Ala Val Leu
   530 535 540
   His Val Arg Ser His Ser Glu Val Pro Gly Phe Phe Thr Glu Gly Asn
   545 550 555 560
   Asp Val Ala Asp Ser Gln Ala Thr Phe Gln Ala Tyr Pro Leu Arg Glu
   565 570 575
   Ala Lys Asp Leu His Thr Ala Leu His Ile Gly Pro Arg Ala Leu Ser
   580 585 590
   Lys Ala Cys Asn Ile Ser Met Gln Gln Ala Arg Glu Val Val Gln Thr
   595 600 605
   Cys Pro His Cys Asn Ser Ala Pro Ala Leu Glu Ala Gly Val Asn Pro
   610 615 620
   Arg Gly Leu Gly Pro Leu Gln Ile Trp Gln Thr Asp Phe Thr Leu Glu
   625 630 635 640
   Pro Arg Met Ala Pro Arg Ser Trp Leu Ala Val Thr Val Asp Thr Ala
   645 650 655
   Ser Ser Ala Ile Val Val Thr Gln His Gly Arg Val Thr Ser Val Ala
   660 665 670
   Ala Gln His His Trp Ala Thr Ala Ile Ala Val Leu Gly Arg Pro Lys
   675 680 685
   Ala Ile Lys Thr Asp Asn Gly Ser Cys Phe Thr Ser Arg Ser Thr Arg
   690 695 700
   Glu Trp Leu Ala Arg Trp Gly Ile Ala His Thr Thr Gly Ile Pro Gly
   705 710 715 720
   Asn Ser Gln Gly Gln Ala Met Val Glu Arg Ala Asn Arg Leu Leu Lys
   725 730 735
   Asp Lys Ile Arg Val Leu Ala Glu Gly Asp Gly Phe Met Lys Arg Ile
   740 745 750
   Pro Ala Ser Lys Gln Gly Glu Leu Leu Ala Lys Ala Met Tyr Ala Leu
           755                 760                 765
   Asn His Phe Glu Arg Gly Glu Asn Thr Lys Thr Pro Val Gln Lys His
       770                 775                 780
   Trp Arg Pro Thr Val Leu Thr Glu Gly Pro Pro Val Lys Ile Arg Ile
   785                 790                 795                 800
   Glu Thr Gly Glu Trp Glu Lys Gly Trp Asn Val Leu Val Trp Gly Arg
                   805                 810                 815
   Gly Tyr Ala Ala Val Lys Asn Arg Asp Thr Asp Lys Val Ile Trp Val
               820                 825                 830
   Pro Ser Arg Lys Val Lys Pro Asp Val Thr Gln Lys Asp Glu Val Thr
           835                 840                 845
   Lys Lys Asp Glu Ala Ser Pro Leu Phe Ala Gly Ile Ser Asp Trp Ile
       850                 855                 860
   Pro Trp Glu Asp Glu Gln Glu Gly Leu Gln Gly Glu Thr Ala Ser Asn
   865                 870                 875                 880
   Lys Gln Glu Arg Pro Gly Glu Asp Thr Leu Ala Ala Asn Glu Ser
                   885                 890                 895
<210> 14
   <211> 10768
   <212> DNA
   <213> Artificial Sequence
<220>
   <223> /note="Seq.-ID Nr. 14: 1. Helferplasmid für kodonoptimiertes gag-pol "
<220>
   <221> promoter
   <222> (232)..(819)
   <223> Promoter von CMV
<220>
   <221> Intron
   <222> (907)..(1463)
   <223> Intron von beta Globin
<220>
   <221> CDS
   <222> (1475)..(3586)
   <223> gal: Kodierende Sequenz für Gal, kodonoptimiert
<220>
   <221> misc_feature
   <222> (3437)..(3737)
   <223> Übergangsbereich zwischen für gag und pol kodierenden Bereichen (wildtyp)
<220>
   <221> misc_feature
   <222> (3574)..(3723)
   <223> Signal für Änderung des Leserahmens (frameshift signal)
<220>
   <221> CDS
   <222> (3604)..(6291)
   <223> pol: Kodierende Sequenz für Pol, kodonoptimiert
<220>
   <221> polyA_signal
   <222> (6368)..(6588)
   <223> poly Adenylierungssignal aus bGH
<400> 14
   gacggatcgg gagatctccc gatcccctat ggtgcactct cagtacaatc tgctctgatg      60
   ccgcatagtt aagccagtat ctgctccctg cttgtgtgtt ggaggtcgct gagtagtgcg     120
   cgagcaaaat ttaagctaca acaaggcaag gcttgaccga caattgcatg aagaatctgc     180
   ttagggttag gcgttttgcg ctgcttcgcg atgtacgggc cagatatacg cgttgacatt     240
   gattattgac tagttattaa tagtaatcaa ttacggggtc attagttcat agcccatata     300
   tggagttccg cgttacataa cttacggtaa atggcccgcc tggctgaccg cccaacgacc     360
   cccgcccatt gacgtcaata atgacgtatg ttcccatagt aacgccaata gggactttcc     420
   attgacgtca atgggtggac tatttacggt aaactgccca cttggcagta catcaagtgt     480
   atcatatgcc aagtacgccc cctattgacg tcaatgacgg taaatggccc gcctggcatt     540
   atgcccagta catgacctta tgggactttc ctacttggca gtacatctac gtattagtca     600
   tcgctattac catggtgatg cggttttggc agtacatcaa tgggcgtgga tagcggtttg     660
   actcacgggg atttccaagt ctccacccca ttgacgtcaa tgggagtttg ttttggcacc     720
   aaaatcaacg ggactttcca aaatgtcgta acaactccgc cccattgacg caaatgggcg     780
   gtaggcgtgt acggtgggag gtctatataa gcagagctct ctggctaact agagaaccca     840
   ctgcttactg gcttatcgaa attaatacga ctcactatag ggagacccaa gcttggtacc     900
   gagctcggat cctgagaact tcagggtgag tctatgggac ccttgatgtt ttctttcccc     960
   ttcttttcta tggttaagtt catgtcatag gaaggggaga agtaacaggg tacacatatt    1020
   gaccaaatca gggtaatttt gcatttgtaa ttttaaaaaa tgctttcttc ttttaatata    1080
   cttttttgtt tatcttattt ctaatacttt ccctaatctc tttctttcag ggcaataatg   1140
   atacaatgta tcatgcctct ttgcaccatt ctaaagaata acagtgataa tttctgggtt    1200
   aaggcaatag caatatttct gcatataaat atttctgcat ataaattgta actgatgtaa    1260
   gaggtttcat attgctaata gcagctacaa tccagctacc attctgcttt tattttatgg    1320
   ttgggataag gctggattat tctgagtcca agctaggccc ttttgctaat catgttcata    1380
   cctcttatct tcctcccaca gctcctgggc aacgtgctgg tctgtgtgct ggcccatcac    1440
   tttggcaaag cacgtgagat ctgaattcgc cacc atg gaa gcc gtg atc aaa gtg    1495
                                         Met Glu Ala Val Ile Lys Val
                                         1               5
   atc agc agc gcc tgc aag acc tac tgc ggc aag atc agc ccc agc aag      1543
   Ile Ser Ser Ala Cys Lys Thr Tyr Cys Gly Lys Ile Ser Pro Ser Lys
   10 15 20
   aaa gaa atc ggc gct atg ctg tct ctc ctg caa aag gaa ggc ctg ctg      1591
   Lys Glu Ile Gly Ala Met Leu Ser Leu Leu Gln Lys Glu Gly Leu Leu
       25                  30                  35
   atg agc ccc agc gac ctg tac agc ccc ggc agc tgg gat cct atc aca      1639
   Met Ser Pro Ser Asp Leu Tyr Ser Pro Gly Ser Trp Asp Pro Ile Thr
   40                  45                  50                  55
   gcc gcc ctg agc cag agg gcc atg gtg ctg ggc aag agc ggc gag ctg      1687
   Ala Ala Leu Ser Gln Arg Ala Met Val Leu Gly Lys Ser Gly Glu Leu
                   60                  65                  70
   aaa acc tgg ggg ctg gtg ctg gga gcc ctg aag gcc gcc aga gaa gaa      1735
   Lys Thr Trp Gly Leu Val Leu Gly Ala Leu Lys Ala Ala Arg Glu Glu
               75                  80                  85
   cag gtg acc agc gag cag gcc aag ttt tgg ctg ggc ctg ggc gga gga      1783
   Gln Val Thr Ser Glu Gln Ala Lys Phe Trp Leu Gly Leu Gly Gly Gly
           90                  95                  100
   aga gtg tct ccc cct ggc ccc gag tgt atc gag aag ccc gcc acc gag      1831
   Arg Val Ser Pro Pro Gly Pro Glu Cys Ile Glu Lys Pro Ala Thr Glu
       105                 110                 115
   cgg aga atc gac aag ggc gag gaa gtg ggc gag aca acc gtg cag cgg      1879
   Arg Arg Ile Asp Lys Gly Glu Glu Val Gly Glu Thr Thr Val Gln Arg
   120                 125                 130                 135
   gac gcc aag atg gcc cct gag gaa gcc gcc acc cct aag acc gtg ggc      1927
   Asp Ala Lys Met Ala Pro Glu Glu Ala Ala Thr Pro Lys Thr Val Gly
                   140                 145                 150
   acc agc tgc tac cac tgt ggc acc gcc gtg ggc tgt aat tgt gcc acc      1975
   Thr Ser Cys Tyr His Cys Gly Thr Ala Val Gly Cys Asn Cys Ala Thr
               155                 160                 165
   gcc acc gcc tct gcc cct cct cct cct tac gtg ggc agc ggc ctg tat      2023
   Ala Thr Ala Ser Ala Pro Pro Pro Pro Tyr Val Gly Ser Gly Leu Tyr
           170                 175                 180
   cct tct ctg gcc ggc gtg ggc gag cag cag gga cag ggc gac aat acc      2071
   Pro Ser Leu Ala Gly Val Gly Glu Gln Gln Gly Gln Gly Asp Asn Thr
       185                 190                 195
   agc aga ggc gcc gag cag cct aga gag gaa cct gga cac gct ggc cag      2119
   Ser Arg Gly Ala Glu Gln Pro Arg Glu Glu Pro Gly His Ala Gly Gln
   200                 205                 210                 215
   gcc cca gga cct gcc ctg aca gat tgg gcc aga gtg cgg gag gaa ctg      2167
   Ala Pro Gly Pro Ala Leu Thr Asp Trp Ala Arg Val Arg Glu Glu Leu
                   220                 225                 230
   gcc tct acc gga ccc cct gtg gtg gcc atg ccc gtg gtg atc aag aca      2215
   Ala Ser Thr Gly Pro Pro Val Val Ala Met Pro Val Val Ile Lys Thr
               235                 240                 245
   gag ggc cct gcc tgg acc cct ctg gaa ccc aag ctg atc acc cgg ctg      2263
   Glu Gly Pro Ala Trp Thr Pro Leu Glu Pro Lys Leu Ile Thr Arg Leu
           250                 255                 260
   gcc gat aca gtg cgg acc aag ggc ctg aga agc ccc atc acc atg gcc      2311
   Ala Asp Thr Val Arg Thr Lys Gly Leu Arg Ser Pro Ile Thr Met Ala
       265                 270                 275
   gag gtg gag gcc ctg atg agc agc ccc ctg ctg ccc cac gac gtg acc      2359
   Glu Val Glu Ala Leu Met Ser Ser Pro Leu Leu Pro His Asp Val Thr
   280                 285                 290                 295
   aac ctg atg aga gtg atc ctg ggc cct gct ccc tac gcc ctg tgg atg      2407
   Asn Leu Met Arg Val Ile Leu Gly Pro Ala Pro Tyr Ala Leu Trp Met
                   300                 305                 310
   gat gcc tgg ggc gtg cag ctg cag aca gtg atc gcc gct gcc acc aga      2455
   Asp Ala Trp Gly Val Gln Leu Gln Thr Val Ile Ala Ala Ala Thr Arg
               315                 320                 325
   gat ccc aga cac ccc gcc aat ggc cag ggc aga ggc gag cgg acc aac      2503
   Asp Pro Arg His Pro Ala Asn Gly Gln Gly Arg Gly Glu Arg Thr Asn
           330                 335                 340
   ctg gac aga ctg aag ggc ctg gcc gac ggc atg gtg ggc aat cct cag      2551
   Leu Asp Arg Leu Lys Gly Leu Ala Asp Gly Met Val Gly Asn Pro Gln
       345                 350                 355
   gga cag gcc gct ctg ctg agg cct ggc gaa ctg gtg gcc atc aca gcc      2599
   Gly Gln Ala Ala Leu Leu Arg Pro Gly Glu Leu Val Ala Ile Thr Ala
   360                 365                 370                 375
   agc gcc ctg cag gcc ttc aga gag gtg gcc aga ctg gcc gag cct gcc      2647
   Ser Ala Leu Gln Ala Phe Arg Glu Val Ala Arg Leu Ala Glu Pro Ala
                   380                 385                 390
   ggc cct tgg gcc gat atc acc cag ggc ccc agc gag agc ttc gtg gac      2695
   Gly Pro Trp Ala Asp Ile Thr Gln Gly Pro Ser Glu Ser Phe Val Asp
               395                 400                 405
   ttc gcc aac cgg ctg atc aag gcc gtg gag ggc agc gat ctg cct cct      2743
   Phe Ala Asn Arg Leu Ile Lys Ala Val Glu Gly Ser Asp Leu Pro Pro
           410                 415                 420
   agc gcc aga gcc ccc gtg atc atc gac tgc ttc cgg cag aag tcc cag      2791
   Ser Ala Arg Ala Pro Val Ile Ile Asp Cys Phe Arg Gln Lys Ser Gln
       425                 430                 435
   ccc gac atc cag cag ctg atc aga gcc gcc cct agc acc ctg acc acc      2839
   Pro Asp Ile Gln Gln Leu Ile Arg Ala Ala Pro Ser Thr Leu Thr Thr
   440                 445                 450                 455
   cct ggc gag atc atc aaa tac gtg ctg gac cgg cag aag atc gcc cct      2887
   Pro Gly Glu Ile Ile Lys Tyr Val Leu Asp Arg Gln Lys Ile Ala Pro
                   460                 465                 470
   ctg acc gat cag ggc att gcc gcc gct atg agc agc gcc atc cag cct      2935
   Leu Thr Asp Gln Gly Ile Ala Ala Ala Met Ser Ser Ala Ile Gln Pro
               475                 480                 485
   ctg gtg atg gcc gtg gtg aac aga gag agg gac ggc cag aca gga tct      2983
   Leu Val Met Ala Val Val Asn Arg Glu Arg Asp Gly Gln Thr Gly Ser
           490                 495                 500
   ggc ggc aga gcc aga ggc ctg tgc tac acc tgt ggc agc cct ggc cac      3031
   Gly Gly Arg Ala Arg Gly Leu Cys Tyr Thr Cys Gly Ser Pro Gly His
       505                 510                 515
   tac cag gcc cag tgc ccc aag aag cgg aag tcc ggc aac agc cgg gag      3079
   Tyr Gln Ala Gln Cys Pro Lys Lys Arg Lys Ser Gly Asn Ser Arg Glu
   520                 525                 530                 535
   aga tgc cag ctg tgt gac ggc atg ggc cac aac gcc aag cag tgc cgg      3127
   Arg Cys Gln Leu Cys Asp Gly Met Gly His Asn Ala Lys Gln Cys Arg
                   540                 545                 550
   aag cgg gat ggc aat cag ggc cag agg ccc ggc aga gga ctg tct agc      3175
   Lys Arg Asp Gly Asn Gln Gly Gln Arg Pro Gly Arg Gly Leu Ser Ser
               555                 560                 565
   ggc cct tgg cct gga cct gag cag cct gcc gtg agc ctg gcc atg acc        3223
   Gly Pro Trp Pro Gly Pro Glu Gln Pro Ala Val Ser Leu Ala Met Thr
           570                 575                 580
   atg gaa cac aag gac cgg ccc ctg gtg cgc gtg atc ctg acc aac acc        3271
   Met Glu His Lys Asp Arg Pro Leu Val Arg Val Ile Leu Thr Asn Thr
       585                 590                 595
   ggc agc cac ccc gtg aag cag cgg agc gtg tac atc acc gcc ctg ctg        3319
   Gly Ser His Pro Val Lys Gln Arg Ser Val Tyr Ile Thr Ala Leu Leu
   600                 605                 610                 615
   gac agc gga gcc gac atc acc atc atc agc gaa gag gac tgg ccc acc        3367
   Asp Ser Gly Ala Asp Ile Thr Ile Ile Ser Glu Glu Asp Trp Pro Thr
                   620                 625                 630
   gac tgg cct gtg gtg gac acc gcc aac ccc cag atc cac ggc atc ggc        3415
   Asp Trp Pro Val Val Asp Thr Ala Asn Pro Gln Ile His Gly Ile Gly
               635                 640                 645
   gga gga atc ccc atg cgg aag tcc cgg gat atg ata gag gtg ggg gtt        3463
   Gly Gly Ile Pro Met Arg Lys Ser Arg Asp Met Ile Glu Val Gly Val
           650                 655                 660
   att aac cga gac ggg tcg ttg gag cga ccc ctg ctc ctc ttc ccc gca        3511
   Ile Asn Arg Asp Gly Ser Leu Glu Arg Pro Leu Leu Leu Phe Pro Ala
       665                 670                 675
   gtc gct atg gtt aga ggg agt atc cta gga aga gat tgt ctg cag ggc        3559
   Val Ala Met Val Arg Gly Ser Ile Leu Gly Arg Asp Cys Leu Gln Gly
   680                 685                 690                 695
   cta ggg ctc cgc ttg aca aat tta tag ggagggccac tgttctt act gtt         3609
   Leu Gly Leu Arg Leu Thr Asn Leu                            Thr Val
                   700                                            705
   gcg cta cat ctg gct att ccg ctc aaa tgg aag tca gac cgc acg cct        3657
   Ala Leu His Leu Ala Ile Pro Leu Lys Trp Lys Ser Asp Arg Thr Pro
                   710                 715                 720
   gtg tgg att gac cag tgg ccc ctt cct gaa ggt aaa ctt gta gcg cta        3705
   Val Trp Ile Asp Gln Trp Pro Leu Pro Glu Gly Lys Leu Val Ala Leu
               725                 730                 735
   acg caa tta gtg gaa aaa gaa tta cag tta ggc cac atc gag ccc agc        3753
   Thr Gln Leu Val Glu Lys Glu Leu Gln Leu Gly His Ile Glu Pro Ser
           740                 745                 750
   ctg agc tgc tgg aac acc ccc gtg ttc gtg atc aga aag gcc agc ggc        3801
   Leu Ser Cys Trp Asn Thr Pro Val Phe Val Ile Arg Lys Ala Ser Gly
       755                 760                 765
   agc tac aga ctg ctg cac gac ctg cgg gcc gtg aat gcc aag ctg gtg        3849
   Ser Tyr Arg Leu Leu His Asp Leu Arg Ala Val Asn Ala Lys Leu Val
   770                 775                 780                 785
   ccc ttt ggc gcc gtg cag cag ggc gct cct gtg ctg tct gcc ctg cca        3897
   Pro Phe Gly Ala Val Gln Gln Gly Ala Pro Val Leu Ser Ala Leu Pro
                   790                 795                 800
   aga gga tgg ccc ctg atg gtg ctg gac ctg aag gac tgc ttc ttc agc        3945
   Arg Gly Trp Pro Leu Met Val Leu Asp Leu Lys Asp Cys Phe Phe Ser
               805                 810                 815
   atc cct ctg gcc gag cag gac aga gag gcc ttc gcc ttc acc ctg cct        3993
   Ile Pro Leu Ala Glu Gln Asp Arg Glu Ala Phe Ala Phe Thr Leu Pro
           820                 825                 830
   agc gtg aac aac cag gcc cca gcc aga cgg ttc cag tgg aag gtg ctg        4041
   Ser Val Asn Asn Gln Ala Pro Ala Arg Arg Phe Gln Trp Lys Val Leu
       835                 840                 845
   ccc cag ggc atg acc tgc agc ccc acc atc tgt cag ctg gtc gtg gga      4089
   Pro Gln Gly Met Thr Cys Ser Pro Thr Ile Cys Gln Leu Val Val Gly
   850                 855                 860                 865
   cag gtg ctg gaa ccc ctg aga ctg aag cac ccc gcc ctg cgg atg ctg      4137
   Gln Val Leu Glu Pro Leu Arg Leu Lys His Pro Ala Leu Arg Met Leu
                   870                 875                 880
   cac tac atg gac gac ctg ctc ctc gcc gct tct agc cac gac ggc ctg      4185
   His Tyr Met Asp Asp Leu Leu Leu Ala Ala Ser Ser His Asp Gly Leu
               885                 890                 895
   gaa gcc gcc gga aaa gaa gtg atc ggc acc ctg gaa aga gcc ggc ttc      4233
   Glu Ala Ala Gly Lys Glu Val Ile Gly Thr Leu Glu Arg Ala Gly Phe
           900                 905                 910
   acc atc agc ccc gac aag atc cag agg gaa ccc ggc gtg cag tac ctg      4281
   Thr Ile Ser Pro Asp Lys Ile Gln Arg Glu Pro Gly Val Gln Tyr Leu
       915                 920                 925
   ggc tac aag ctg ggc agc acc tat gtg gcc cct gtg ggc ctg gtg gcc      4329
   Gly Tyr Lys Leu Gly Ser Thr Tyr Val Ala Pro Val Gly Leu Val Ala
   930                 935                 940                 945
   gag cca aga atc gcc acc ctg tgg gac gtg cag aaa ctg gtg ggc agc      4377
   Glu Pro Arg Ile Ala Thr Leu Trp Asp Val Gln Lys Leu Val Gly Ser
                   950                 955                 960
   ctg cag tgg ctg agg cca gcc ctg ggc atc cct ccc aga ctg atg ggc      4425
   Leu Gln Trp Leu Arg Pro Ala Leu Gly Ile Pro Pro Arg Leu Met Gly
               965                 970                 975
   ccc ttc tac gag cag ctg cgg ggc tcc gac cct aat gag gcc cgg gag      4473
   Pro Phe Tyr Glu Gln Leu Arg Gly Ser Asp Pro Asn Glu Ala Arg Glu
           980                 985                 990
   tgg aac ctg gat atg aag atg  gcc tgg cgg gag atc  gtg cag ctg tcc      4521
   Trp Asn Leu Asp Met Lys Met  Ala Trp Arg Glu Ile  Val Gln Leu Ser
       995                 1000                1005
   acc  act gcc gcc ctg gaa  cgc tgg gat cca gca  cag cca ctc gaa      4566
   Thr  Thr Ala Ala Leu Glu  Arg Trp Asp Pro Ala  Gln Pro Leu Glu
   1010                 1015                 1020
   ggt  gcc gtg gcc aga tgt  gaa cag ggc gcc atc  ggc gtg ctg ggc      4611
   Gly  Ala Val Ala Arg Cys  Glu Gln Gly Ala Ile  Gly Val Leu Gly
   1025                 1030                 1035
   cag  gga ctg agc acc cac  ccc aga ccc tgc ctg  tgg ctg ttc agc      4656
   Gln  Gly Leu Ser Thr His  Pro Arg Pro Cys Leu  Trp Leu Phe Ser
   1040                 1045                 1050
   acc  cag ccc acc aag gcc  ttc acc gcc tgg ctg  gaa gtg ctg acc      4701
   Thr  Gln Pro Thr Lys Ala  Phe Thr Ala Trp Leu  Glu Val Leu Thr
   1055                 1060                 1065
   ctg  ctg atc acc aag ctg  cgg gcc agc gcc gtg  aga acc ttc ggc      4746
   Leu  Leu Ile Thr Lys Leu  Arg Ala Ser Ala Val  Arg Thr Phe Gly
   1070                 1075                 1080
   aaa  gag gtg gac atc ctg  ctg ctg cca gcc tgc  ttt aga gag gac      4791
   Lys  Glu Val Asp Ile Leu  Leu Leu Pro Ala Cys  Phe Arg Glu Asp
   1085                 1090                 1095
   ctg  ccc ctg cct gag gga  atc ctg ctg gcc ctg  aga ggc ttt gcc      4836
   Leu  Pro Leu Pro Glu Gly  Ile Leu Leu Ala Leu  Arg Gly Phe Ala
   1100 1105 1110
   ggc aag atc cgg tcc agc gac acc ccc agc atc ttc gat atc gcc 4881
   Gly Lys Ile Arg Ser Ser Asp Thr Pro Ser Ile Phe Asp Ile Ala
   1115 1120 1125
   aga ccc ctg cac gtg tcc ctg aaa gtg cgc gtg acc gat cac cct 4926
   Arg Pro Leu His Val Ser Leu Lys Val Arg Val Thr Asp His Pro
   1130 1135 1140
   gtg cct ggc ccc acc gtg ttt acc gac gcc agc agc agc acc cac 4971
   Val Pro Gly Pro Thr Val Phe Thr Asp Ala Ser Ser Ser Thr His
   1145 1150 1155
   aag ggc gtg gtg gtg tgg aga gaa ggc ccc aga tgg gag atc aaa 5016
   Lys Gly Val Val Val Trp Arg Glu Gly Pro Arg Trp Glu Ile Lys
   1160 1165 1170
   gaa atc gtg gac ctg ggc gcc tct gtg cag cag ctg gaa gcc aga 5061
   Glu Ile Val Asp Leu Gly Ala Ser Val Gln Gln Leu Glu Ala Arg
   1175 1180 1185
   gcc gtg gcc atg gct ctg ctg ctg tgg ccc acc acc ccc acc aac 5106
   Ala Val Ala Met Ala Leu Leu Leu Trp Pro Thr Thr Pro Thr Asn
   1190 1195 1200
   gtg gtg acc gac agc gcc ttc gtg gcc aag atg ctg ctg aag atg 5151
   Val Val Thr Asp Ser Ala Phe Val Ala Lys Met Leu Leu Lys Met
   1205 1210 1215
   ggc cag gaa ggc gtc cct agc acc gcc gct gcc ttc atc ctg gaa 5196
   Gly Gln Glu Gly Val Pro Ser Thr Ala Ala Ala Phe Ile Leu Glu
   1220 1225 1230
   gat gcc ctg tcc cag cgg agc gct atg gct gct gtg ctg cac gtg 5241
   Asp Ala Leu Ser Gln Arg Ser Ala Met Ala Ala Val Leu His Val
   1235 1240 1245
   cgg agc cac agc gag gtg ccc ggc ttc ttc acc gag ggc aac gac 5286
   Arg Ser His Ser Glu Val Pro Gly Phe Phe Thr Glu Gly Asn Asp
   1250 1255 1260
   gtg gcc gac agc cag gcc acc ttc cag gcc tac ccc ctg aga gag 5331
   Val Ala Asp Ser Gln Ala Thr Phe Gln Ala Tyr Pro Leu Arg Glu
   1265 1270 1275
   gcc aag gac ctg cac aca gcc ctg cac atc ggc ccc aga gcc ctg 5376
   Ala Lys Asp Leu His Thr Ala Leu His Ile Gly Pro Arg Ala Leu
   1280 1285 1290
   agc aag gcc tgc aac atc agc atg cag cag gcc aga gaa gtg gtg 5421
   Ser Lys Ala Cys Asn Ile Ser Met Gln Gln Ala Arg Glu Val Val
   1295 1300 1305
   cag acc tgt ccc cac tgt aat agc gcc cct gct ctg gaa gcc ggc 5466
   Gln Thr Cys Pro His Cys Asn Ser Ala Pro Ala Leu Glu Ala Gly
   1310 1315 1320
   gtg aac cct aga ggc ctc ggc cct ctg cag atc tgg cag acc gac 5511
   Val Asn Pro Arg Gly Leu Gly Pro Leu Gln Ile Trp Gln Thr Asp
   1325 1330 1335
   ttc acc ctg gaa ccc cgg atg gcc cct aga agc tgg ctg gcc gtg 5556
   Phe Thr Leu Glu Pro Arg Met Ala Pro Arg Ser Trp Leu Ala Val
   1340 1345 1350
   acc gtg gat acc gcc agc tcc gcc atc gtg gtg acc cag cac ggc 5601
   Thr Val Asp Thr Ala Ser Ser Ala Ile Val Val Thr Gln His Gly
   1355 1360 1365
   aga  gtg aca tct gtg gcc  gcc cag cac cac tgg  gcc aca gcc att      5646
   Arg  Val Thr Ser Val Ala  Ala Gln His His Trp  Ala Thr Ala Ile
   1370                 1375                 1380
   gcc  gtg ctg ggc aga ccc  aag gcc atc aag acc  gac aac ggc agc      5691
   Ala  Val Leu Gly Arg Pro  Lys Ala Ile Lys Thr  Asp Asn Gly Ser
   1385                 1390                 1395
   tgc  ttc acc agc cgg tcc  acc aga gaa tgg ctg  gcc aga tgg ggc      5736
   Cys  Phe Thr Ser Arg Ser  Thr Arg Glu Trp Leu  Ala Arg Trp Gly
   1400                 1405                 1410
   att  gct cac acc acc ggc  atc cca ggc aat agt  cag ggc cag gcc      5781
   Ile  Ala His Thr Thr Gly  Ile Pro Gly Asn Ser  Gln Gly Gln Ala
   1415                 1420                 1425
   atg  gtg gag aga gcc aac  agg ctg ctg aag gac  aag atc cgg gtg      5826
   Met  Val Glu Arg Ala Asn  Arg Leu Leu Lys Asp  Lys Ile Arg Val
   1430                 1435                 1440
   ctg  gcc gag ggc gac ggc  ttc atg aag cgg atc  ccc gcc agc aaa      5871
   Leu  Ala Glu Gly Asp Gly  Phe Met Lys Arg Ile  Pro Ala Ser Lys
   1445                 1450                 1455
   cag  ggc gag ctg ctg gcc  aag gcc atg tac gcc  ctg aac cac ttc      5916
   Gln  Gly Glu Leu Leu Ala  Lys Ala Met Tyr Ala  Leu Asn His Phe
   1460                 1465                 1470
   gag  cgg ggc gag aac acc  aag acc ccc gtg cag  aag cac tgg cgg      5961
   Glu  Arg Gly Glu Asn Thr  Lys Thr Pro Val Gln  Lys His Trp Arg
   1475                 1480                 1485
   cct  acc gtg ctg aca gag  gga ccc ccc gtg aag  atc cgg atc gag      6006
   Pro  Thr Val Leu Thr Glu  Gly Pro Pro Val Lys  Ile Arg Ile Glu
   1490                 1495                 1500
   aca  ggc gag tgg gag aag  ggg tgg aac gtg ctg  gtg tgg ggc aga      6051
   Thr  Gly Glu Trp Glu Lys  Gly Trp Asn Val Leu  Val Trp Gly Arg
   1505                 1510                 1515
   ggc  tac gcc gct gtc aag  aac cgg gac acc gac  aaa gtg atc tgg      6096
   Gly  Tyr Ala Ala Val Lys  Asn Arg Asp Thr Asp  Lys Val Ile Trp
   1520                 1525                 1530
   gtg  ccc agc cgg aaa gtg  aag ccc gac gtg acc  cag aaa gac gaa      6141
   Val  Pro Ser Arg Lys Val  Lys Pro Asp Val Thr  Gln Lys Asp Glu
   1535                 1540                 1545
   gtg  acc aag aag gac gag  gcc tcc ccc ctc ttt  gcc ggc atc agc      6186
   Val  Thr Lys Lys Asp Glu  Ala Ser Pro Leu Phe  Ala Gly Ile Ser
   1550                 1555                 1560
   gac  tgg atc cct tgg gag  gac gag cag gaa ggc  ctg cag ggc gag      6231
   Asp  Trp Ile Pro Trp Glu  Asp Glu Gln Glu Gly  Leu Gln Gly Glu
   1565                 1570                 1575
   aca  gcc agc aac aag cag  gaa aga ccc ggc gag  gac acc ctg gcc      6276
   Thr  Ala Ser Asn Lys Gln  Glu Arg Pro Gly Glu  Asp Thr Leu Ala
   1580                 1585                 1590
   gcc  aat gag agc tga gaattctgca gatatccagc acagtggcgg ccgctcgagt    6331
   Ala  Asn Glu Ser
   1595
   ctagagggcc cgtttaaacc cgctgatcag cctcgactgt gccttctagt tgccagccat   6391
   ctgttgtttg cccctccccc gtgccttcct tgaccctgga aggtgccact cccactgtcc   6451
   tttcctaata aaatgaggaa attgcatcgc attgtctgag taggtgtcat tctattctgg 6511
   ggggtggggt ggggcaggac agcaaggggg aggattggga agacaatagc aggcatgctg 6571
   gggatgcggt gggctctatg gcttctgagg cggaaagaac cagctggggc tctagggggt 6631
   atccccacgc gccctgtagc ggcgcattaa gcgcggcggg tgtggtggtt acgcgcagcg 6691
   tgaccgctac acttgccagc gccctagcgc ccgctccttt cgctttcttc ccttcctttc 6751
   tcgccacgtt cgccggcttt ccccgtcaag ctctaaatcg ggggctccct ttagggttcc 6811
   gatttagtgc tttacggcac ctcgacccca aaaaacttga ttagggtgat ggttcacgta 6871
   gtgggccatc gccctgatag acggtttttc gccctttgac gttggagtcc acgttcttta 6931
   atagtggact cttgttccaa actggaacaa cactcaaccc tatctcggtc tattcttttg 6991
   atttataagg gattttgccg atttcggcct attggttaaa aaatgagctg atttaacaaa 7051
   aatttaacgc gaattaattc tgtggaatgt gtgtcagtta gggtgtggaa agtccccagg 7111
   ctccccagca ggcagaagta tgcaaagcat gcatctcaat tagtcagcaa ccaggtgtgg 7171
   aaagtcccca ggctccccag caggcagaag tatgcaaagc atgcatctca attagtcagc 7231
   aaccatagtc ccgcccctaa ctccgcccat cccgccccta actccgccca gttccgccca 7291
   ttctccgccc catggctgac taattttttt tatttatgca gaggccgagg ccgcctctgc 7351
   ctctgagcta ttccagaagt agtgaggagg cttttttgga ggcctaggct tttgcaaaaa 7411
   gctcccggga gcttgtatat ccattttcgg atctgatcaa gagacaggat gaggatcgtt 7471
   tcgcatgatt gaacaagatg gattgcacgc aggttctccg gccgcttggg tggagaggct 7531
   attcggctat gactgggcac aacagacaat cggctgctct gatgccgccg tgttccggct 7591
   gtcagcgcag gggcgcccgg ttctttttgt caagaccgac ctgtccggtg ccctgaatga 7651
   actgcaggac gaggcagcgc ggctatcgtg gctggccacg acgggcgttc cttgcgcagc 7711
   tgtgctcgac gttgtcactg aagcgggaag ggactggctg ctattgggcg aagtgccggg 7771
   gcaggatctc ctgtcatctc accttgctcc tgccgagaaa gtatccatca tggctgatgc 7831
   aatgcggcgg ctgcatacgc ttgatccggc tacctgccca ttcgaccacc aagcgaaaca 7891
   tcgcatcgag cgagcacgta ctcggatgga agccggtctt gtcgatcagg atgatctgga 7951
   cgaagagcat caggggctcg cgccagccga actgttcgcc aggctcaagg cgcgcatgcc 8011
   cgacggcgag gatctcgtcg tgacccatgg cgatgcctgc ttgccgaata tcatggtgga 8071
   aaatggccgc ttttctggat tcatcgactg tggccggctg ggtgtggcgg accgctatca 8131
   ggacatagcg ttggctaccc gtgatattgc tgaagagctt ggcggcgaat gggctgaccg 8191
   cttcctcgtg ctttacggta tcgccgctcc cgattcgcag cgcatcgcct tctatcgcct 8251
   tcttgacgag ttcttctgag cgggactctg gggttcgaaa tgaccgacca agcgacgccc 8311
   aacctgccat cacgagattt cgattccacc gccgccttct atgaaaggtt gggcttcgga 8371
   atcgttttcc gggacgccgg ctggatgatc ctccagcgcg gggatctcat gctggagttc 8431
   ttcgcccacc ccaacttgtt tattgcagct tataatggtt acaaataaag caatagcatc 8491
   acaaatttca caaataaagc atttttttca ctgcattcta gttgtggttt gtccaaactc 8551
   atcaatgtat cttatcatgt ctgtataccg tcgacctcta gctagagctt ggcgtaatca 8611
   tggtcatagc tgtttcctgt gtgaaattgt tatccgctca caattccaca caacatacga 8671
   gccggaagca taaagtgtaa agcctggggt gcctaatgag tgagctaact cacattaatt 8731
   gcgttgcgct cactgcccgc tttccagtcg ggaaacctgt cgtgccagct gcattaatga 8791
   atcggccaac gcgcggggag aggcggtttg cgtattgggc gctcttccgc ttcctcgctc 8851
   actgactcgc tgcgctcggt cgttcggctg cggcgagcgg tatcagctca ctcaaaggcg 8911
   gtaatacggt tatccacaga atcaggggat aacgcaggaa agaacatgtg agcaaaaggc 8971
   cagcaaaagg ccaggaaccg taaaaaggcc gcgttgctgg cgtttttcca taggctccgc 9031
   ccccctgacg agcatcacaa aaatcgacgc tcaagtcaga ggtggcgaaa cccgacagga 9091
   ctataaagat accaggcgtt tccccctgga agctccctcg tgcgctctcc tgttccgacc 9151
   ctgccgctta ccggatacct gtccgccttt ctcccttcgg gaagcgtggc gctttctcat 9211
   agctcacgct gtaggtatct cagttcggtg taggtcgttc gctccaagct gggctgtgtg 9271
   cacgaacccc ccgttcagcc cgaccgctgc gccttatccg gtaactatcg tcttgagtcc 9331
   aacccggtaa gacacgactt atcgccactg gcagcagcca ctggtaacag gattagcaga 9391
   gcgaggtatg taggcggtgc tacagagttc ttgaagtggt ggcctaacta cggctacact 9451
   agaagaacag tatttggtat ctgcgctctg ctgaagccag ttaccttcgg aaaaagagtt 9511
   ggtagctctt gatccggcaa acaaaccacc gctggtagcg gtttttttgt ttgcaagcag 9571
   cagattacgc gcagaaaaaa aggatctcaa gaagatcctt tgatcttttc tacggggtct 9631
   gacgctcagt ggaacgaaaa ctcacgttaa gggattttgg tcatgagatt atcaaaaagg 9691
   atcttcacct agatcctttt aaattaaaaa tgaagtttta aatcaatcta aagtatatat 9751
   gagtaaactt ggtctgacag ttaccaatgc ttaatcagtg aggcacctat ctcagcgatc 9811
   tgtctatttc gttcatccat agttgcctga ctccccgtcg tgtagataac tacgatacgg 9871
   gagggcttac catctggccc cagtgctgca atgataccgc gagacccacg ctcaccggct 9931
   ccagatttat cagcaataaa ccagccagcc ggaagggccg agcgcagaag tggtcctgca 9991
   actttatccg cctccatcca gtctattaat tgttgccggg aagctagagt aagtagttcg 10051
   ccagttaata gtttgcgcaa cgttgttgcc attgctacag gcatcgtggt gtcacgctcg 10111
   tcgtttggta tggcttcatt cagctccggt tcccaacgat caaggcgagt tacatgatcc 10171
   cccatgttgt gcaaaaaagc ggttagctcc ttcggtcctc cgatcgttgt cagaagtaag 10231
   ttggccgcag tgttatcact catggttatg gcagcactgc ataattctct tactgtcatg 10291
   ccatccgtaa gatgcttttc tgtgactggt gagtactcaa ccaagtcatt ctgagaatag 10351
   tgtatgcggc gaccgagttg ctcttgcccg gcgtcaatac gggataatac cgcgccacat 10411
   agcagaactt taaaagtgct catcattgga aaacgttctt cggggcgaaa actctcaagg 10471
   atcttaccgc tgttgagatc cagttcgatg taacccactc gtgcacccaa ctgatcttca 10531
   gcatctttta ctttcaccag cgtttctggg tgagcaaaaa caggaaggca aaatgccgca 10591
   aaaaagggaa taagggcgac acggaaatgt tgaatactca tactcttcct ttttcaatat 10651
   tattgaagca tttatcaggg ttattgtctc atgagcggat acatatttga atgtatttag 10711
   aaaaataaac aaataggggt tccgcgcaca tttccccgaa aagtgccacc tgacgtc 10768
<210> 15
   <211> 703
   <212> PRT
   <213> Artificial Sequence
<220>
   <223> Synthetic Construct
<400> 15
   Met Glu Ala Val Ile Lys Val Ile Ser Ser Ala Cys Lys Thr Tyr Cys
   1               5                   10                  15
   Gly Lys Ile Ser Pro Ser Lys Lys Glu Ile Gly Ala Met Leu Ser Leu
               20                  25                  30
   Leu Gln Lys Glu Gly Leu Leu Met Ser Pro Ser Asp Leu Tyr Ser Pro
           35                  40                  45
   Gly Ser Trp Asp Pro Ile Thr Ala Ala Leu Ser Gln Arg Ala Met Val
       50                  55                  60
   Leu Gly Lys Ser Gly Glu Leu Lys Thr Trp Gly Leu Val Leu Gly Ala
   65                  70                  75                  80
   Leu Lys Ala Ala Arg Glu Glu Gln Val Thr Ser Glu Gln Ala Lys Phe
                   85                  90                  95
   Trp Leu Gly Leu Gly Gly Gly Arg Val Ser Pro Pro Gly Pro Glu Cys
               100                 105                 110
   Ile Glu Lys Pro Ala Thr Glu Arg Arg Ile Asp Lys Gly Glu Glu Val
           115                 120                 125
   Gly Glu Thr Thr Val Gln Arg Asp Ala Lys Met Ala Pro Glu Glu Ala
       130                 135                 140
   Ala Thr Pro Lys Thr Val Gly Thr Ser Cys Tyr His Cys Gly Thr Ala
   145                 150                 155                 160
   Val Gly Cys Asn Cys Ala Thr Ala Thr Ala Ser Ala Pro Pro Pro Pro
                   165                 170                 175
   Tyr Val Gly Ser Gly Leu Tyr Pro Ser Leu Ala Gly Val Gly Glu Gln
               180                 185                 190
   Gln Gly Gln Gly Asp Asn Thr Ser Arg Gly Ala Glu Gln Pro Arg Glu
           195                 200                 205
   Glu Pro Gly His Ala Gly Gln Ala Pro Gly Pro Ala Leu Thr Asp Trp
       210                 215                 220
   Ala Arg Val Arg Glu Glu Leu Ala Ser Thr Gly Pro Pro Val Val Ala
   225                 230                 235                 240
   Met Pro Val Val Ile Lys Thr Glu Gly Pro Ala Trp Thr Pro Leu Glu
                   245                 250                 255
   Pro Lys Leu Ile Thr Arg Leu Ala Asp Thr Val Arg Thr Lys Gly Leu
               260                 265                 270
   Arg Ser Pro Ile Thr Met Ala Glu Val Glu Ala Leu Met Ser Ser Pro
           275                 280                 285
   Leu Leu Pro His Asp Val Thr Asn Leu Met Arg Val Ile Leu Gly Pro
       290                 295                 300
   Ala Pro Tyr Ala Leu Trp Met Asp Ala Trp Gly Val Gln Leu Gln Thr
   305                 310                 315                 320
   Val Ile Ala Ala Ala Thr Arg Asp Pro Arg His Pro Ala Asn Gly Gln
                   325                 330                 335
   Gly Arg Gly Glu Arg Thr Asn Leu Asp Arg Leu Lys Gly Leu Ala Asp
               340                 345                 350
   Gly Met Val Gly Asn Pro Gln Gly Gln Ala Ala Leu Leu Arg Pro Gly
           355                 360                 365
   Glu Leu Val Ala Ile Thr Ala Ser Ala Leu Gln Ala Phe Arg Glu Val
       370                 375                 380
   Ala Arg Leu Ala Glu Pro Ala Gly Pro Trp Ala Asp Ile Thr Gln Gly
   385                 390                 395                 400
   Pro Ser Glu Ser Phe Val Asp Phe Ala Asn Arg Leu Ile Lys Ala Val
                   405                 410                 415
   Glu Gly Ser Asp Leu Pro Pro Ser Ala Arg Ala Pro Val Ile Ile Asp
               420                 425                 430
   Cys Phe Arg Gln Lys Ser Gln Pro Asp Ile Gln Gln Leu Ile Arg Ala
           435                 440                 445
   Ala Pro Ser Thr Leu Thr Thr Pro Gly Glu Ile Ile Lys Tyr Val Leu
       450                 455                 460
   Asp Arg Gln Lys Ile Ala Pro Leu Thr Asp Gln Gly Ile Ala Ala Ala
   465                 470                 475                 480
   Met Ser Ser Ala Ile Gln Pro Leu Val Met Ala Val Val Asn Arg Glu
   485 490 495
   Arg Asp Gly Gln Thr Gly Ser Gly Gly Arg Ala Arg Gly Leu Cys Tyr
   500 505 510
   Thr Cys Gly Ser Pro Gly His Tyr Gln Ala Gln Cys Pro Lys Lys Arg
   515 520 525
   Lys Ser Gly Asn Ser Arg Glu Arg Cys Gln Leu Cys Asp Gly Met Gly
   530 535 540
   His Asn Ala Lys Gln Cys Arg Lys Arg Asp Gly Asn Gln Gly Gln Arg
   545 550 555 560
   Pro Gly Arg Gly Leu Ser Ser Gly Pro Trp Pro Gly Pro Glu Gln Pro
   565 570 575
   Ala Val Ser Leu Ala Met Thr Met Glu His Lys Asp Arg Pro Leu Val
   580 585 590
   Arg Val Ile Leu Thr Asn Thr Gly Ser His Pro Val Lys Gln Arg Ser
   595 600 605
   Val Tyr Ile Thr Ala Leu Leu Asp Ser Gly Ala Asp Ile Thr Ile Ile
   610 615 620
   Ser Glu Glu Asp Trp Pro Thr Asp Trp Pro Val Val Asp Thr Ala Asn
   625 630 635 640
   Pro Gln Ile His Gly Ile Gly Gly Gly Ile Pro Met Arg Lys Ser Arg
   645 650 655
   Asp Met Ile Glu Val Gly Val Ile Asn Arg Asp Gly Ser Leu Glu Arg
   660 665 670
   Pro Leu Leu Leu Phe Pro Ala Val Ala Met Val Arg Gly Ser Ile Leu
   675 680 685
   Gly Arg Asp Cys Leu Gln Gly Leu Gly Leu Arg Leu Thr Asn Leu
   690 695 700
<210> 16
   <211> 895
   <212> PRT
   <213> Artificial Sequence
<220>
   <223> Synthetic Construct
<400> 16
   Thr Val Ala Leu His Leu Ala Ile Pro Leu Lys Trp Lys Ser Asp Arg
   1               5                   10                  15
   Thr Pro Val Trp Ile Asp Gln Trp Pro Leu Pro Glu Gly Lys Leu Val
   20 25 30
   Ala Leu Thr Gln Leu Val Glu Lys Glu Leu Gln Leu Gly His Ile Glu
   35 40 45
   Pro Ser Leu Ser Cys Trp Asn Thr Pro Val Phe Val Ile Arg Lys Ala
   50 55 60
   Ser Gly Ser Tyr Arg Leu Leu His Asp Leu Arg Ala Val Asn Ala Lys
   65 70 75 80
   Leu Val Pro Phe Gly Ala Val Gln Gln Gly Ala Pro Val Leu Ser Ala
   85 90 95
   Leu Pro Arg Gly Trp Pro Leu Met Val Leu Asp Leu Lys Asp Cys Phe
   100 105 110
   Phe Ser Ile Pro Leu Ala Glu Gln Asp Arg Glu Ala Phe Ala Phe Thr
   115 120 125
   Leu Pro Ser Val Asn Asn Gln Ala Pro Ala Arg Arg Phe Gln Trp Lys
   130 135 140
   Val Leu Pro Gln Gly Met Thr Cys Ser Pro Thr Ile Cys Gln Leu Val
   145 150 155 160
   Val Gly Gln Val Leu Glu Pro Leu Arg Leu Lys His Pro Ala Leu Arg
   165 170 175
   Met Leu His Tyr Met Asp Asp Leu Leu Leu Ala Ala Ser Ser His Asp
   180 185 190
   Gly Leu Glu Ala Ala Gly Lys Glu Val Ile Gly Thr Leu Glu Arg Ala
   195 200 205
   Gly Phe Thr Ile Ser Pro Asp Lys Ile Gln Arg Glu Pro Gly Val Gln
   210 215 220
   Tyr Leu Gly Tyr Lys Leu Gly Ser Thr Tyr Val Ala Pro Val Gly Leu
   225 230 235 240
   Val Ala Glu Pro Arg Ile Ala Thr Leu Trp Asp Val Gln Lys Leu Val
   245 250 255
   Gly Ser Leu Gln Trp Leu Arg Pro Ala Leu Gly Ile Pro Pro Arg Leu
   260 265 270
   Met Gly Pro Phe Tyr Glu Gln Leu Arg Gly Ser Asp Pro Asn Glu Ala
   275 280 285
   Arg Glu Trp Asn Leu Asp Met Lys Met Ala Trp Arg Glu Ile Val Gln
           290                 295                 300
   Leu Ser Thr Thr Ala Ala Leu Glu Arg Trp Asp Pro Ala Gln Pro Leu
   305                 310                 315                 320
   Glu Gly Ala Val Ala Arg Cys Glu Gln Gly Ala Ile Gly Val Leu Gly
                   325                 330                 335
   Gln Gly Leu Ser Thr His Pro Arg Pro Cys Leu Trp Leu Phe Ser Thr
               340                 345                 350
   Gln Pro Thr Lys Ala Phe Thr Ala Trp Leu Glu Val Leu Thr Leu Leu
           355                 360                 365
   Ile Thr Lys Leu Arg Ala Ser Ala Val Arg Thr Phe Gly Lys Glu Val
       370                 375                 380
   Asp Ile Leu Leu Leu Pro Ala Cys Phe Arg Glu Asp Leu Pro Leu Pro
   385                 390                 395                 400
   Glu Gly Ile Leu Leu Ala Leu Arg Gly Phe Ala Gly Lys Ile Arg Ser
                   405                 410                 415
   Ser Asp Thr Pro Ser Ile Phe Asp Ile Ala Arg Pro Leu His Val Ser
               420                 425                 430
   Leu Lys Val Arg Val Thr Asp His Pro Val Pro Gly Pro Thr Val Phe
           435                 440                 445
   Thr Asp Ala Ser Ser Ser Thr His Lys Gly Val Val Val Trp Arg Glu
       450                 455                 460
   Gly Pro Arg Trp Glu Ile Lys Glu Ile Val Asp Leu Gly Ala Ser Val
   465                 470                 475                 480
   Gln Gln Leu Glu Ala Arg Ala Val Ala Met Ala Leu Leu Leu Trp Pro
                   485                 490                 495
   Thr Thr Pro Thr Asn Val Val Thr Asp Ser Ala Phe Val Ala Lys Met
               500                 505                 510
   Leu Leu Lys Met Gly Gln Glu Gly Val Pro Ser Thr Ala Ala Ala Phe
           515                 520                 525
   Ile Leu Glu Asp Ala Leu Ser Gln Arg Ser Ala Met Ala Ala Val Leu
       530                 535                 540
   His Val Arg Ser His Ser Glu Val Pro Gly Phe Phe Thr Glu Gly Asn
   545                 550                 555                 560
   Asp Val Ala Asp Ser Gln Ala Thr Phe Gln Ala Tyr Pro Leu Arg Glu
                   565                 570                 575
   Ala Lys Asp Leu His Thr Ala Leu His Ile Gly Pro Arg Ala Leu Ser
   580 585 590
   Lys Ala Cys Asn Ile Ser Met Gln Gln Ala Arg Glu Val Val Gln Thr
   595 600 605
   Cys Pro His Cys Asn Ser Ala Pro Ala Leu Glu Ala Gly Val Asn Pro
   610 615 620
   Arg Gly Leu Gly Pro Leu Gln Ile Trp Gln Thr Asp Phe Thr Leu Glu
   625 630 635 640
   Pro Arg Met Ala Pro Arg Ser Trp Leu Ala Val Thr Val Asp Thr Ala
   645 650 655
   Ser Ser Ala Ile Val Val Thr Gln His Gly Arg Val Thr Ser Val Ala
   660 665 670
   Ala Gln His His Trp Ala Thr Ala Ile Ala Val Leu Gly Arg Pro Lys
   675 680 685
   Ala Ile Lys Thr Asp Asn Gly Ser Cys Phe Thr Ser Arg Ser Thr Arg
   690 695 700
   Glu Trp Leu Ala Arg Trp Gly Ile Ala His Thr Thr Gly Ile Pro Gly
   705 710 715 720
   Asn Ser Gln Gly Gln Ala Met Val Glu Arg Ala Asn Arg Leu Leu Lys
   725 730 735
   Asp Lys Ile Arg Val Leu Ala Glu Gly Asp Gly Phe Met Lys Arg Ile
   740 745 750
   Pro Ala Ser Lys Gln Gly Glu Leu Leu Ala Lys Ala Met Tyr Ala Leu
   755 760 765
   Asn His Phe Glu Arg Gly Glu Asn Thr Lys Thr Pro Val Gln Lys His
   770 775 780
   Trp Arg Pro Thr Val Leu Thr Glu Gly Pro Pro Val Lys Ile Arg Ile
   785 790 795 800
   Glu Thr Gly Glu Trp Glu Lys Gly Trp Asn Val Leu Val Trp Gly Arg
   805 810 815
   Gly Tyr Ala Ala Val Lys Asn Arg Asp Thr Asp Lys Val Ile Trp Val
   820 825 830
   Pro Ser Arg Lys Val Lys Pro Asp Val Thr Gln Lys Asp Glu Val Thr
   835 840 845
   Lys Lys Asp Glu Ala Ser Pro Leu Phe Ala Gly Ile Ser Asp Trp Ile
       850                 855                 860
   Pro Trp Glu Asp Glu Gln Glu Gly Leu Gln Gly Glu Thr Ala Ser Asn
   865                 870                 875                 880
   Lys Gln Glu Arg Pro Gly Glu Asp Thr Leu Ala Ala Asn Glu Ser
                   885                 890                 895